(19) Europäisches Patentamt
European Patent Office
Office européen des brevets

(11) EP 0 625 986 B1

(12) **FASCICULE DE BREVET EUROPEEN**

(45) Date de publication et mention de la délivrance du brevet:
**15.01.1997 Bulletin 1997/03**

(21) Numéro de dépôt: **93904155.4**

(22) Date de dépôt: **04.02.1993**

(51) Int Cl.[6]: **C07H 21/02**, C07H 21/04, C07H 21/00, C12Q 1/68, A61K 31/70

(86) Numéro de dépôt international:
**PCT/FR93/00115**

**WO 93/16095 (19.08.1993 Gazette 1993/20)**

# (54) OLIGOTHIONUCLEOTIDES

OLIGOTHIONUKLEOTIDE

OLIGOTHIONUCLEOTIDES

(84) Etats contractants désignés:
**AT BE CH DE DK ES FR GB GR IE IT LI LU MC NL PT SE**

(30) Priorité: **05.02.1992 FR 9201275**
**17.09.1992 FR 9211103**

(43) Date de publication de la demande:
**30.11.1994 Bulletin 1994/48**

(73) Titulaire: **CENTRE NATIONAL DE LA RECHERCHE SCIENTIFIQUE (CNRS)**
**75794 Paris Cédex 16 (FR)**

(72) Inventeurs:
- **BARASCUT, Jean-Louis**
**F-34980 Combaillaux (FR)**
- **IMBACH, Jean-Louis Impasse des Luques**
**F-34000 Montpellier (FR)**

(74) Mandataire: **Warcoin, Jacques et al**
**Cabinet Régimbeau,**
**26, avenue Kléber**
**75116 Paris (FR)**

(56) Documents cités:
**EP-A- 0 117 777**
**EP-A- 0 169 787**
**WO-A-83/01451**

- **BIOCHEMISTRY vol. 12, no. 11, 1973, EASTON, PA, US, pages 2041-2045, A.K.M. ANISUZZAMAN et al.**
- **BIOCHEMISTRY vol. 9, no. 11, 1970, EASTON, PA, US, pages 2367-2372, D. J. HOFFMAN et al.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 625 986 B1

## Description

La présente invention concerne des composés chimiques nouveaux, ainsi que leurs applications. Les composés chimiques selon la présente invention sont des composés oligonucléotides constitués au moins en partie par un oligo 4'-thio-ribonucléotide ou un oligo 4'-thio-2'désoxyribonucléotide comportant un enchaînement de 4'thionucléotides.

Les composés selon l'invention peuvent présenter la configuration anomérique naturelle bêta ou la configuration anomérique non naturelle alpha.

Ainsi sont représentés par les formules (a) et (b) respectivement des 4'-thionucléotides alpha(a) et bêta(b)

(a)

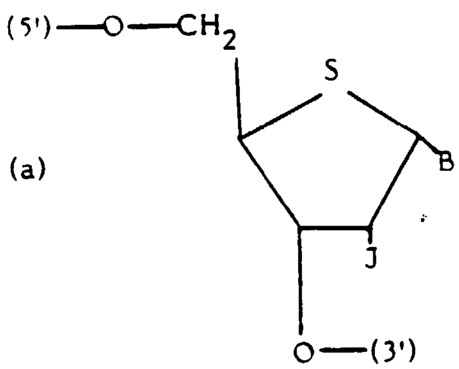

(b)

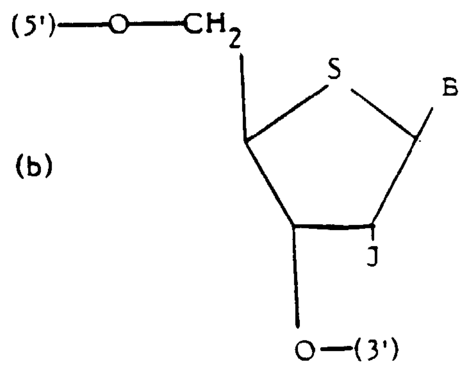

(a) et (b) représentent des 4'-thionucléosides, il faut rajouter un phosphate en 5' pour obtenir des nucléotides.

Dans ces conditions, de multiples utilisations à finalités biologiques et même pharmacologiques déjà connues pour les oligonucléotides peuvent être envisagées et ceci avec plus d'efficacité.

Plus précisément, la présente invention a pour objet des composés chimiques constitués par un oligo4'-thioribonucléotide ou un oligo-4'-thio-2'désoxyribonucléotide, caractérisés en ce qu'ils comportent un enchaînement de 4'-thioribonucléotides ou 4'-thio-2'désoxyribonucléotides respectivement, enchaînement pouvant être facultativement lié à un agent effecteur, notamment un radical correspondant à un agent d'intercalation ou un radical chimique ou photoactivable, tel qu'un radical porteur d'une fonction réagissant directement ou indirectement avec les chaînes de nucléotides ou un radical dont la présence permet une détection facile et sensible.

En particulier, l'invention a pour objet les nouveaux dérivés oligo-4'-thionucléotides, leur préparation et leur emploi notamment comme sondes permettant la détection d'une séquence définie d'acides nucléiques, comme nucléases artificielles spécifiques de séquences d'ADN ou d'ARN ou bien comme agents de blocage sélectif de l'expression d'un gène qu'il soit endogène (par exemple, gène oncogène) ou exogène (par exemple ADN ou ARN de virus, de parasites ou de bactéries).

Dans les demandes de brevet français FR 8301223 (2 540 122) et FR 8411795 (2 568 254) ont été décrits des composés chimiques constitués par un oligonucléotide ou un oligodésoxynucléotide comportant un enchaînement de nucléotides naturels ou modifiés, c'est-à-dire des bêta-nucléotides, sur lesquel se trouve fixé par une liaison covalente au moins un groupe intercalant, qui possèdent la propriété de bloquer sélectivement l'expression d'un gène et qui, de ce fait, sont particulièrement utiles en thérapeutique comme substances antivirales, antibiotiques, antiparasitaires ou antitumorales.

Dans la demande internationale PCT WO 83/01451, a été décrite une méthode pour bloquer la traduction de l'ARN messager (ARNm) en protéine par hybridation de l'ARNm avec un oligonucléotide ayant la séquence complémentaire de l'ARNm, l'oligonucléotide étant stabilisé sous forme phosphotriester.

Dans la demande internationale WO 88/04301 ont été décrits des oligonucléotides de configuration anomérique alpha présentant des appariements parallèles avec des séquences complémentaires.

La Chimiothérapie par les oligonucléotides antisens concerne des cibles à ARN ou à ADN de tous les organismes vivants (cellules, bactéries, parasites, virus ou oncogènes).

L'utilisation d'oligonucléotides antisens synthétiques ayant la même structure que les acides nucléiques naturels se heurte principalement en milieu biologique à des problèmes de sensibilité aux nucléases et de pénétration cellulaire.

Pour pallier ces limites, des analogues oligonucléotidiques susceptibles d'être plus résistants vis à vis des nucléases et de mieux pénétrer dans les cellules à travers la membrane cytoplasmique ont été synthétisés.

Il a en effet été décrit dans la technique antérieure des dérivés de composés oligonucléotides résistant mieux aux dégradations enzymatiques dont la partie phosphate était modifiée en thiophosphate ou méthylphosphonate notamment. Toutefois ces dérivés présentent une chiralité au niveau du phosphate susceptible de générer des diastéréoisomères insolubles.

La présente invention fournit une nouvelle classe d'oligonucléotides chimères les 4'-thio-oligonucléotides dans lesquels l'oxygène intracyclique du cycle furannose a été remplacé par un atome de soufre.

Il a en effet maintenant été trouvé, et c'est ce qui fait l'objet de la présente invention, que la substitution de l'oxygène par un soufre sur la partie sucre des nucléosides plutôt que sur le groupement phosphate d'une part, assure la solubilité des oligomères et, d'autre part, pourrait accroître leur pénétration dans la cellule cible, la substitution de l'oxygène par l'élément soufre rendant la molécule plus lipophile.

Les dérivés de 4'-thionucléotides forment des complexes d'hybridation avec les séquences complémentaires d'ARN beaucoup plus stables que ceux formés avec l'ADN et que, vis-à-vis des ARNs, les dérivés de 4'-thionucléotides forment des complexes d'hybridation plus stables que les dérivés de bêta-nucléotides naturels.

Parmi les composés, selon la présente invention, on peut citer plus particulièrement les composés oligomères bêta de formule

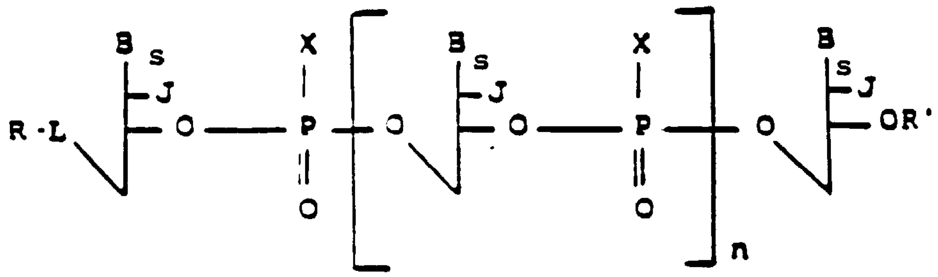 (I)

dans laquelle :

les radicaux B peuvent être identiques ou différents et représentent chacun une base d'un acide nucléique éventuellement modifiée, activable et/ou comportant un groupement intercalant ;

les radicaux X peuvant être identiques ou différents et représentent chacun un oxoanion $O^-$, un thioanion $S^-$, un groupe alkyle, un groupe alcoxy, aryloxy, un groupe aminoalkyle, un groupe aminoalcoxy, un groupe thioalkyle, un radical alkyle ou alcoxy substitué par un hétérocycle azoté ou un groupement -Y-Z ;

R et R', qui peuvent être identiques ou différents, représentent chacun un atome d'hydrogène ou un groupement -Y-Z ou Y'-Z' ;

Y et Y' identiques ou différents représentent chacun un radical alcoylène droit ou ramifié -alk- ou un radical choisi parmi

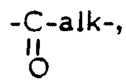 , 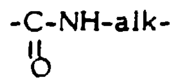 , 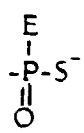 , 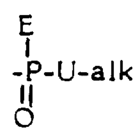 , 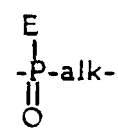 ,

alk-O-alk

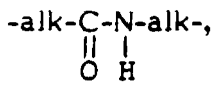 , 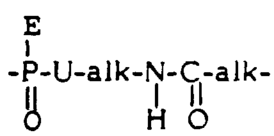 et 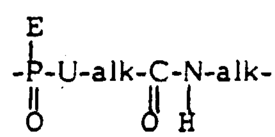

avec U = O, S ou N

En particulier, Y et Y' représentent un radical choisi parmi

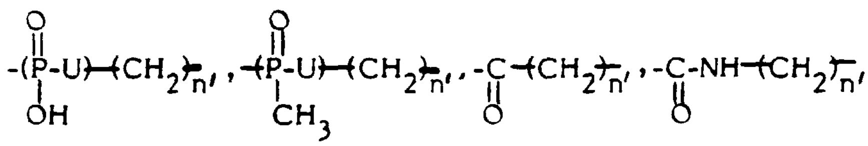

avec U = O, S ou N

E peut avoir les mêmes significations que X excepté Y-Z ou Y'-Z' ;
L représente O, S ou -NH-;
n et n' représentent un nombre entier y compris 0 ;
J représente un atome d'hydrogène ou un groupe hydroxy ;
Z et Z', identiques ou différents, représentent chacun OH ou un radical correspondant à un agent effecteur, notamment un radical correspondant à un agent d'intercalation ou un radical porteur d'une fonction qui réagit directement ou indirectement avec les chaînes de nucléotides ou un radical dont la présence permet une détection facile et sensible.

Dans cette formule I, on utilise la représentation condensée des nucléotides suivante :

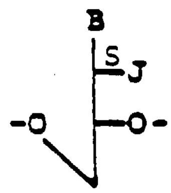

qui correspond à la formule développée :

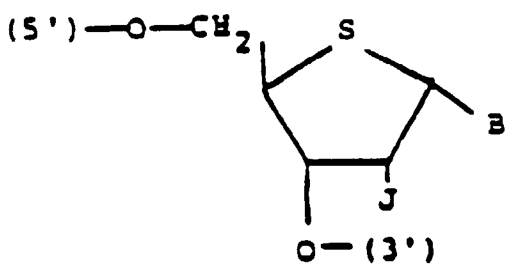

sur laquelle ont été mentionnées les extrémités (3') et (5').

Il convient de remarquer que la formule I représente un enchaînement de 4'-thionucléotides qui peuvent être identiques ou différents, de configuration D ou L, n indiquant simplement le nombre de nucléotides compris dans la molécule ; n est de préférence un nombre compris entre 1 et 50, et de préférence encore entre 1 et 25.

On cite en particulier les produits pour lesquels L représente l'oxygène ; R et R' représentent l'hydrogène et B une base nucléique naturelle, soit adénine, thymine, cytosine ou guanine quand J = H et adénine, uracile, cytosine et guanine quand J = OH.

Les agents d'intercalation sont des produits connus dans les techniques relatives aux acides nucléiques ; ce sont des composés capables de "s'intercaler" dans la structure des ADN ou des ARN, c'est-à-dire capables de s'insérer entre les plateaux de base des acides nucléiques.

Les agents d'intercalation peuvent être choisis parmi les composés polycycliques ayant une configuration plane tels que l'acridine et ses dérivés, la furocoumarine et ses dérivés, la daunomycine et les autres dérivés de l'anthracycline, la 1,10-phénanthroline, la phénanthridine et ses dérivés, la proflavine, les porphyrines, les dérivés du dipyrido [1,2-a : 3',2'-d] imidazole, l'ellipticine ou l'ellipticinium et leurs dérivés et le diazapyrène et ses dérivés.

Les radicaux chimiques réactifs peuvent être des radicaux pouvant réagir directement ou indirectement avec une chaîne de nucléotides pour former une liaison covalente ou pour la modifier chimiquement, ou pour la couper. De préférence, ces radicaux chimiques réactifs sont activables, par exemple, par voie chimique, biochimique ou photochimique.

Les radicaux réactifs activables peuvent être choisis parmi l'acide éthylène-diamine-tétraacétique, l'acide diéthylène-triamine-pentaacétique, les porphyrines, la 1,10-phénanthroline, l'azido-4 acétophénone, l'éthylène-imine, la bêta-chloroéthylamine, le psoralène et leurs dérivés, et les composés aromatiques absorbant les radiations du proche ultra-violet ou du visible ou pouvant réagir chimiquement avec les constituants nucléiques.

Plus particulièrement, les radicaux chimiquement activables en présence d'ions métalliques, d'oxygène et d'un agent réducteur (acide éthylène-diamine-tétraacétique, acide diéthylène-tri-amine-pentaacétique, porphyrine, phénanthroline) induisent la coupure dans des séquences d'acides nucléiques situées dans leur voisinage.

Par irradiation dans le domaine du visible ou du proche ultra-violet, il est possible d'activer les dérivés qui absorbent ces radiations et de réaliser des réactions de pontage ou des réactions photo-induites (coupure et modification des bases nucléiques) des acides nucléiques sur lesquels est fixé l'oligothionucléotide porteur du groupement activable.

Parmi les radicaux Z et Z' peuvent être plus particulièrement cités :

- les radicaux dérivés de l'acide éthylène-diamine-tétra-acétique de formule :

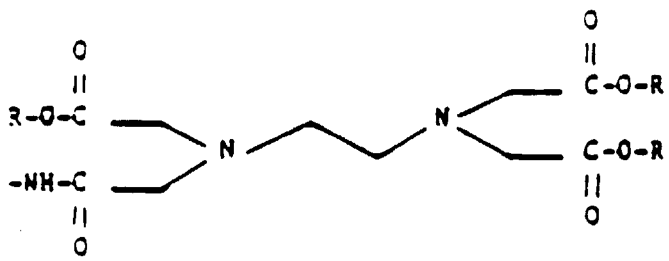

- les radicaux dérivés de l'acide diéthylène-triamine-pentaacétique,
- les radicaux dérivés de la méthylpyrroporphyrine de formule :

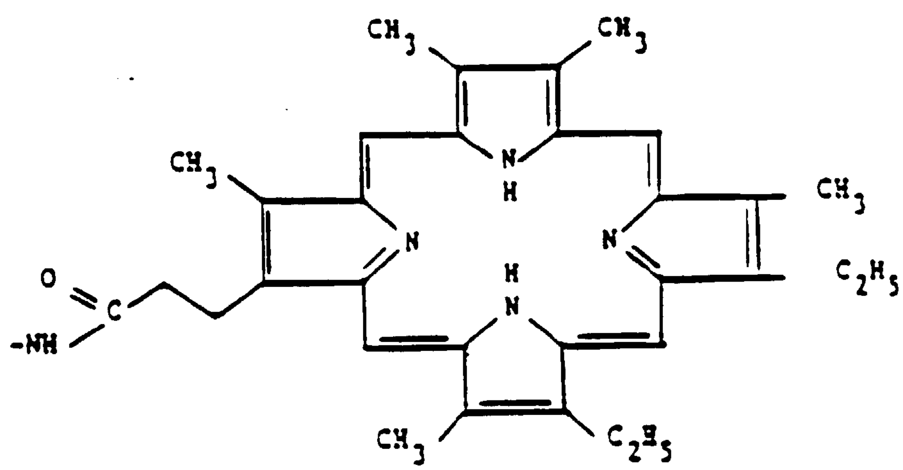

- les radicaux dérivés de la phénanthroline de formule :

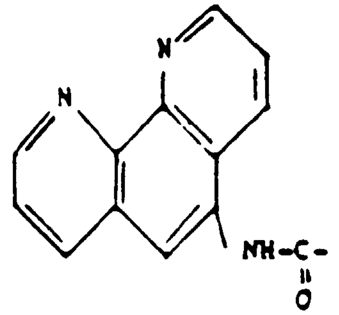

- les radicaux dérivés de l'acridine :

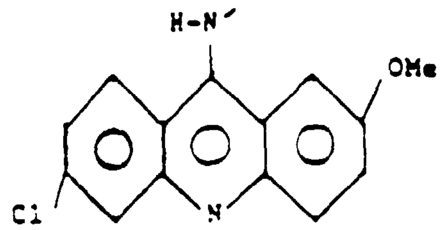

- les radicaux dérivés de la proflavine

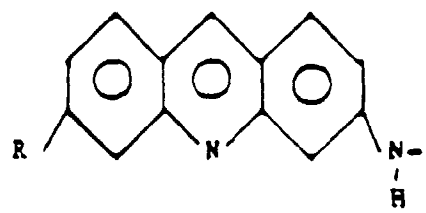

R représentant un groupement amino ($NH_2$) ou azido ($N_3$)

- les radicaux dérivés de la biotine

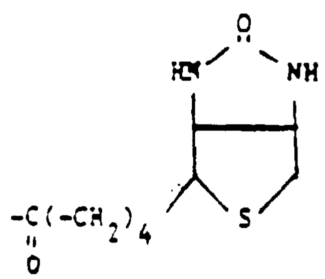

- les radicaux dérivés de l'azido-4 acétophénone de formule :

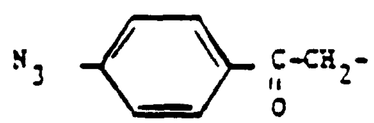

Le radical B peut être, de préférence, choisi parmi les bases nucléiques naturelles (thymine, adénine, cytosine, guanine, uracile) mais il est possible d'utiliser des bases nucléiques modifiées. Peuvent être cités l'amino-2 adénine et ses dérivés substitués, par exemple, sur l'atome d'azote $N^6$ par un radical aminoalkylène ou par un radical azidophényl-alkylène, la guanine substituée sur l'atome d'oxygène $O^6$, par exemple, par un groupement (w-alkylène)-9-acridine, la (w-aminoalkyl)-amino-8-adénine et ses dérivés substitués sur le radical amino en w par un groupe acridine, ou les dérivés halogénés ou azidés tels que le bromo-5 uracile, l'azido-8 adénine, la 7-déazaadénine, la 7-déazaguanine. Il est possible également d'utiliser des dérivés des bases nucléiques comportant un groupement intercalant ou un groupement chimiquement ou photochimiquement activable.

Ainsi, la fonctionnalisation de C ou T par un groupement aziridine en position 4 conduit à la formation de pontages covalents $CH_2$-$CH_2$ entre les deux brins complémentaires sur respectivement G et A. Donc, plus particulièrement, le radical B est alors choisi parmi la 4-azido-cytosine ou la 4-azido-thymine.

De préférence, le radical X représente un oxoanion. Cependant, le radical X peut représenter un radical alkyle contenant 1 à 7 atomes de carbone (méthyle, éthyle, propyle), un radical alcoxy dont la partie alkyle contient 1 à 7

atomes de carbone (méthoxy, éthoxy, diméthyl-2,2 propyloxy), un radical aminoalkyle ou aminoalcoxy de formule générale $R_1R_2N$-alk-A- dans laquelle A représente une liaison ou un atome d'oxygène, -alk- représente un radical alkylène contenant 1 à 10 atomes de carbone et $R_1$ et $R_2$, identiques ou différents, représentent un atome d'hydrogène ou un radical alkyle contenant 1 à 7 atomes de carbone ou forment ensemble avec l'atome d'azote auquel ils sont liés un hétérocycle azoté saturé à 5 ou 6 chaînons, étant entendu que le groupement $R_1R_2N$- peut être quaternarisé, ou un radical alcoylthio dont la partie alcoyle contient 1 à 7 atomes de carbone.

Dans la formule (I), le radical -alk- est de préférence un radical alcoylène droit ou ramifié ayant de 1 à 10 atomes de carbone.

En particulier, à partir des fonctions alcool terminales 3' ou 5' de l'oligothionucléotide, l'effecteur Z peut donc être introduit via une chaîne $(CH_2)_n$ liée à une fonctionnalisation Z', de natures diverses à la partie osidique de l'oligomère.

A partir de la formule suivante :

3'
ou $-O-Z'_1-(CH_2)_n$-effecteur (Z)
5'

on obtiendra, selon ce que représente $Z'_1$, par exemple

- un phosphate ou méthyl phosphonate de formule

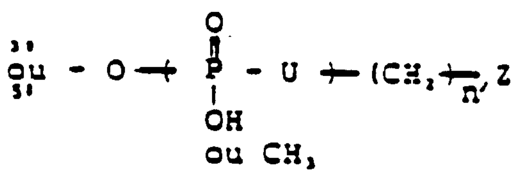

U = O, N ou S
- un éther de formule générale

3' ou 5' $-O-(CH_2)-(CH_2)_{n'-1}-Z$

- un ester de formule générale :

$-O-(CO)-(CH_2)_{n'}-Z$

ou encore
- un carbamate de formule générale :

$-O-(CO\ NH)-(CH_2)_{n'}-Z$

En général, n' est compris entre 1 et 10.

La présente invention concerne également les composés précédents sous forme de sel avec des bases ou des acides, et les composés sous forme racémique, ou sous forme d'isomètres R ou S optiques purifiés ou en mélange, de formule (I).

La présente invention concerne de préférence les composés précédents de formule (I) dans la série D.

On cite en particulier les produits composés D-oligothionucléotide de formule générale :

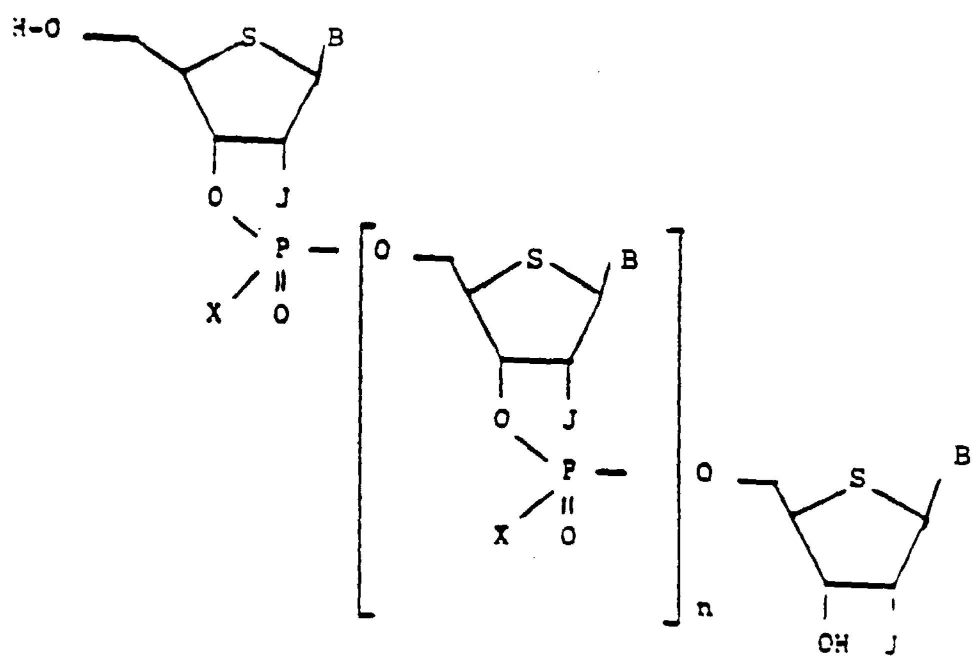

dans laquelle J et X sont définis comme précédemment pour les composés de formule (I) et B représente une base nucléique naturelle.

Les oligonucléotides selon la présente invention peuvent se présenter sous forme de séquences homogènes, comme dans les composés de formule (I) ou sous forme d'oligomères mixtes, c'est-à-dire sous forme de séquences particulières incluses dans d'autres types d'oligonucléotides de type ADN ou ARN, modifiés ou non au niveau de l'enchaînement phosphate, ces derniers corresondant à des composés de formule (I), dans lesquels l'oxygène intracyclique du cycle furannose est rétabli.

La présente invention a donc également pour objet des composés oligomères mixtes caractérisés en ce qu'ils sont constitués par des composés oligothionucléotides selon l'invention liés à des oligonucléotides de type ADN ou ARN. On entend ici par "oligonucléotides de type ADN ou ARN" une séquence d'acides nucléiques de type ADN ou ARN modifiés ou non au niveau de l'enchaînement phosphate et dans lesquels l'hétéroatome intracyclique du cycle furannose des nucléotides est l'oxygène.

La présente invention a notamment pour objet des composés oligomères mixtes constitués par une séquence oligothionucléotidique selon l'invention, comprenant une séquence oligonucléotidique de type ADN ou ARN en son sein ou à une de ses extrémités.

Dans un mode de réalisation particulier, donc, la séquence oligonucléotidique, de type ADN ou ARN, est encadrée par deux séquences d'oligothioribonucléotides.

Les nouveaux produits de formule générale (I) ou oligomères mixtes en comportant peuvent être préparés par voie chimique par des procédés connus et, en particulier, ceux qui sont décrits dans les demandes de procédés connus et, en particulier, ceux qui sont décrits dans les demandes de brevets français FR 8301223 (2 540 122) et FR 8411795 (2 568 254), WO 88/04301 et FR 88 122648.

Les composés oligothionucléotides notamment de formule (I) peuvent être préparés par voie chimique par des procédés dans lesquels on applique des synthèses au phosphodiester, au phosphotriester, au phosphoramidite ou à l'hydrogénophosphonate classiques pour des oligonucléotides.

Dans ces procédés, on prépare tout d'abord la chaîne de 4'-thionucléotides, les différents groupements non mis en oeuvre étant protégés, puis on élimine enfin les groupements protecteurs pour obtenir les produits recherchés.

On cite en particulier un procédé de synthèse de composés oligothionucléotides selon l'invention sans agent effecteur, caractérisé en ce que on réalise une synthèse supportée selon la méthode au phosphoroamidite comportant

- une protection en 3' et 5' des 4'-thionucléotides ou oligo4'-thionucléotides de départ avec par exemple du diméthoxytrityl en 5' et du diisopropylaminophosphoramidite de méthyle en 3',
- la fonctionnalisation d'un support solide incorporant un dérivé 4'-thionucléosidique par un lien par exemple succinyle entre le groupement hydroxyle-3' du dérivé 4'-thionucléosidique et un groupement amino du support solide,
- l'élongation de la chaîne oligothionucléotidique dans un réacteur synthétiseur,
- enfin le décrochage, la déprotection et la purification de l'oligothionucléotide prolongé.

On cite également un procédé de synthèse de composés oligothionucléotides incorporant un agent effecteur selon l'invention caractérisé en ce que l'on part d'un oligothionucléotide sans agent effecteur, ou d'un oligomère mixte en comprenant, protégé en 5', dont on fait réagir la terminaison OH 3' avec une fonction non protégée d'un bras difonctionnel dont la deuxième fonction est protégée et après déprotection du produit résultant, on lie ledit produit à l'agent effecteur par la deuxième fonction libre du bras difonctionnel.

La terminaison OH 3' de l'oligomère protégé en 5' peut être estérifiée avec un acide aminohexanoïque, dont la fonction amine est protégée.

Un procédé de préparation en phase solide de séquences oligothionucléotides selon la méthode au phosphoramidite, objet dela présente invention, comporte les étapes essentielles suivantes :

- On immobilise un dérivé 4'-thionucléoside protégé par l'intermédiaire d'une de ses fonctions hydroxyle en 3' ou, le cas échéant, 2' sur un support solide.
- On assemble sur ce support dans un réacteur synthétiseur manuel ou automatique la chaîne d'oligothionucléotide protégée par condensation de monomères constitués de 4'-thionucleosides protégés substitués par des groupes phosphoramidites en 3', le cas échéant, la fonction hydroxyle en 2' des riboses étant protégée par les groupes Ctmp ou TBDMS.
- Les oligothionucléotides sont obtenus après décrochage de l'oligomère obtenu du support et élimination des groupes protecteurs.

De façon appropriée, l'assemblage de l'oligothionucléotide se fait par condensation, en présence d'un agent activateur, desdits monomères entre leur fonction en 3' et la fonction 5' du composé 4'-thionucléoside immobilisé pour le premier monomère ou d'un composé intermédiaire polythionucléotide protégé fixé sur ledit composé thionucléoside immobilisé pour les monomères suivants.

Notamment, l'agent activateur pour la condensation desdits monomères peut être choisi parmi le tétrazole et ses dérivés, tels que le para-nitrophényl tétrazole.

Avantageusement, le groupe phosphoramidite en 3' desdits monomères est un groupe N,N dialkylaminophosphoramidite de formule

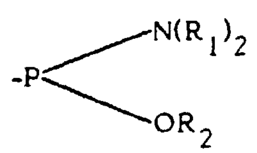

avec $R_1$ qui représente un alkyle en $C_1$ à $C_7$ éventuellement substitué identique ou différent, tel que $CH_3$, $(CH_3)_2CH$.

$R_2$ qui représente un alkyle en $C_1$ à $C_7$ éventuellement substitué, tel que $CH_3$, $CH_2CH_2CN$.

En particulier, le groupe phosphoramidite en 3' desdits monomères est le diisopropylaminophosphoramidite de méthyl ($R_1 = (CH_3)_2CH$ et $R_2 = CH_3$) ou le cyano-2 éthyl diisopropylaminophosphite ($R_1 = (CH_3)_2CH$ et $R_2 = CH_2CH_2CN$).

De façon appropriée, les groupes hydroxyles desdits monomères et dudit composé 4'-thionucléoside immobilisé peuvent être protégés en 5' par le groupe DmTr.

Le composé 4'thionucléoside immobilisé peut être fixé sur le support solide via un groupe divalent succinyle entre un de ses groupements OH en 2' ou 3' qui se trouve ainsi estérifié, et un groupement amino du support.

Le support solide peut être constitué par une longue chaîne alkylamine fixée sur un polymère, un gel de silice ou des billes de verre poreuses.

On peut citer comme groupe fonctionnel permettant la fixation ou étant lié sur support solide doté d'un groupe amino, le groupe de formule

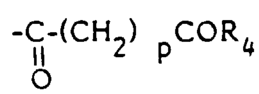

avec p = 1 à 5,

dans lequel $R_4$ représente H un groupe d'activation tel que $C_6Cl_5$ ou un radical NH lié à un support solide tel que le radical NH d'une chaîne alkylamine fixée sur un polymère, gel de silice ou billes de verre poreuses.

Un intérêt des composés homogènes ou mixtes selon la présente invention, comprenant une chaîne d'oligo-thio-nucléotides, est aussi de pouvoir envisager leur utilisation indépendamment d'agents effecteurs, notamment d'agents intercalants, puisque ces nouvelles substances oligonucléotides assurent la reconnaisance de la séquence d'acide nucléique complémentaire, notamment d'ARN, avec une stabilité très accrue ; la fonction de l'agent intercalant étant d'augmenter la stabilité du complexe d'hybridation, sa présence n'est plus obligatoire.

Les composés selon l'invention, par leur grande affinité spécifique des séquences oligothionucléotidiques pour des séquences nucléiques complémentaires, sont en effet supérieurs aux oligonucléotides actuels dont l'affinité pour les séquences complémentaires est plus faible.

Les composés, selon la présente invention, sont donc utilisables de manière plus efficace, à titre de sondes d'hybridation, mais également comme composants de purification pour des séquences déterminées d'ADN ou d'ARN.

Ces composés peuvent également être utilisés pour détecter des mutations au niveau d'un ADN ou d'un ARN de manière plus efficace.

Les composés de la présente invention, par la propriété des séquences oligothionucléotidiques de se fixer fortement sur la séquence nucléique complémentaire, puis d'induire la coupure de la chaîne d'acides nucléiques en ce site, notamment lorsqu'ils sont couplés à un agent de coupure, sont utilisables à titre de nucléases artificielles spécifiques de séquences. Ils peuvent être utilisés comme réactifs en biologie moléculaire ou en génie génétique.

La présence de la fonction hydroxyle en 2', le cas échéant, permet en outre d'envisager leur utilisation comme ribozymes artificiels, que ce soit sous forme de séquences oligothioribonucléotidiques homogènes ou mixtes, c'est-à-dire associées à des oligonucléotides de type ADN ou ARN.

L'objet de la présente invention est également l'application des composés selon l'invention pour réaliser le blocage spécifique des gènes cellulaires ou d'agents pathogènes préalablement choisis (virus, bactéries, parasites).

Les composés selon l'invention sont donc aussi utilisables à titre de médicaments.

Les nouveaux produits de formule générale (I) selon l'invention et les produits de formule générale (I) dans laquelle L représente un atome d'oxygène, R et R' représentent chacun un atome d'hydrogène et B représente une base nucléique naturelle forment des complexes d'hybridation avec les séquences complémentaires d'ARN beaucoup plus stables que ceux qui sont formés avec l'ADN.

En particulier, les produits de formule (I) dans lesquels J = OH (oligothioribonucléotides) s'apparient de manière plus stable avec des séquences d'ARN complémentaires que les produits de formule (I) dans lesquels J = H (oligothiodésoxyribonucléotides).

Compte tenu du fait que les cibles des molécules anti-sens en thérapies sont les gènes ARN messagers, cette propriété des oligothioribonucléotides est tout à fait intéressante.

Cette différence de stabilité des complexes d'hybridation permet une inhibition préférentielle des ARNs messagers et des ARNs viraux sans risque important de provoquer des effets indésirables au niveau de l'ADN du génome.

De plus, vis-à-vis des ARNs, les produits selon l'invention forment généralement des complexes plus stables que ceux qui sont obtenus à partir des oligonucléotides naturels.

Les composés oligothioribonucléotides selon l'invention, notamment de formule (I) (J = OH), présentent en outre un certain nombre de caractéristiques intéressantes, notamment pour une application comme agent antisens :

- la configuration bêta naturelle est respectée,
- ils sont isoélectriques des ARN naturels,
- ils n'ont pas de chiralité au niveau de la liaison phosphodiester,
- ils sont plus lipophiles que les analogues naturels à cause de la présence de l'atome de soufre.

Les séquences des oligothionucléotides, couplés ou non à un agent intercalant ou à un groupement activable chimiquement ou photochimiquement, comportant un enchaînement de nucléosides pyrimidiques sont capables de se fixer sur une double hélice d'ADN ou d'ARN comportant une séquence de bases puriques adjacentes associées par liaison hydrogène à la séquence complémentaire des bases pyrimidiques. Cette fixation implique la formation locale d'une triple hélice dans laquelle les bases pyrimidiques de l'oligothionucléotide forment des liaisons hydrogène (de type Hoogsteen ou Hoogsteen inversé) avec les bases puriques de la double hélice. Dans ce contexte, les oligothionucléotides forment des complexes plus stables que les oligonucléotides correspondants et ils permettent d'induire des réactions irréversibles (pontage, modification ou coupure) sur une double hélice d'ARN ou d'ADN.

Les nouveaux produits de formule générale (I) selon l'invention et les produits de formule générale (I) dans laquelle L représente un atome d'oxygène, R et R' représentent chacun un atome d'hydrogène et B représente une base nucléique naturelle, qui possèdent la propriété de se fixer fortement sur la séquence nucléique complémentaire, ou oligomères mixtes en comprenant, peuvent être utilisés comme sondes pour détecter la présence d'une chaîne de nucléotides complémentaires. Cette détection est facilitée par la présence d'un groupe fluorescent ou d'un groupe biotine dans ces molécules.

La structure non naturelle des oligothionucléotides confère des propriétés d'antigénicité rendant ainsi possible la préparation d'anticorps spécifiques dirigés contre des oligothionucléotides éventuellement couplés à un agent intercalant ou contre leurs complexes avec un ADN ou un ARN naturels.

Dans un but diagnostique ou pronostique, les oligothionucléotides, éventuellement couplés à un agent intercalant, ou oligommères mixtes en comprenant, peuvent être attachés de façon covalente à un support solide. Le couplage à un marqueur luminescent ou générateur d'une réaction colorée, luminescente ou fluorescente permet de les utiliser comme sondes de séquences d'ADN ou d'ARN dans un but diagnostique ou pronostique.

Les oligothionucléotides selon l'invention sont beaucoup plus résistants vis-à-vis des nucléases que les dérivés de nucléosides naturels, Cette stabilité vis-à-vis de l'hydrolyse enzymatique permet l'utilisation des produits selon l'invention, notamment de formule générale (I), ou oligomères mixtes en comprenant, dans des expérimentations in vivo ou in vitro en présence de nucléases. De ce fait, les oligothionucléotides selon l'invention présentent des avantages incontestables sur les dérivés de bêta-nucléosides déjà connus.

Les composés oligothioribonucléotides de formule (I) pour lesquels J = OH sont plus résitants à la dégradation enzymatiques que les composés oligothiodésoxyribonucléotides de formule I pour lesquels J = H.

Dès lors, comme on l'a vu, un autre objet de la présente invention concerne l'application des nouveaux produits selon l'invention, notamment de formule générale (I), et des produits de formule générale (I) dans laquelle L représente un atome d'oxygène, R et R' représentent chacun un atome d'hydrogène et B représente une base nucléique naturelle, en particulier les produits pour lesquels J = OH, ou oligomères mixtes en comprenant, au blocage spécifique de gènes cellulaires ou d'agents pathogènes préalablement choisis (virus, bactéries, parasites), en particulier les ARNm.

Les nouveaux produits selon l'invention, notamment de formule générale (I), et les produits de formule générale (I) dans laquelle L représente un atome d'oxygène, R et R' représentent chacun un atome d'hydrogène et B représente une base nucléique naturelle, en particulier les produits pour lesquels J = OH, ou oligomères mixtes en comprenant, sont particulièrement utiles comme médicaments permettant de bloquer les gènes indésirables exogènes (virus, bactéries, parasites) ou endogènes (gènes cellulaires, oncogènes), en particulier les ARNm. L'expression de ces gènes peut être bloquée en agissant soit directement sur l'ADN ou l'ARN porteur de l'information génétique, soit sur l'ARN messager, copie du gène, en bloquant alors toute traduction par hybridation ou par hybridation puis pontage ou modification ou coupure de l'ARN messager ou viral choisi comme cible.

Ce blocage peut être réalisé en utilisant un produit selon l'invention, notamment de formule générale (I), ou un produit de formule générale (I) dans laquelle L représente un atome d'oxygène, R et R' représentent chacun un atome d'hydrogène et B représente une base nucléique naturelle, en particulier les produits pour lesquels J = OH, ou oligomères mixtes en comprenant, dont la séquence est complémentaire de celle d'une région non complexée d'un ARN ou d'un ADN et, en particulier, d'un ARN messager. L'hybridation, suivie ou non du pontage, de la modification ou de la coupure de l'ARN messager ou viral, empêche la synthèse de l'ARN ou de la protéine correspondante ou l'expression des fonctions virales ou parasitaires.

Si cet ARN ou cette protéine est vital pour le virus, la bactérie ou le parasite, les produits selon l'invention, notamment de formule générale (I), et les produits de formule générale (I) dans laquelle L représente un atome d'oxygène, R et R' représentent chacun un atome d'hydrogène et B représente une base nucléique naturelle, en prticulier les produits pour lesquels J = OH, ou oligomères mixtes en comprenant, constitueront des médicaments à activité antivirale, antibactérienne ou antiparasitaire.

Si cet ARN ou cette protéine n'est pas vital pour l'organisme, il est possible d'en supprimer sélectivement les effets. Dans ce cas, les produits selon l'invention, notamment de formule générale (I), et les produits de formule générale (I) dans laquelle L représente un atome d'oxygène, R et R' représentent chacun un atome d'hydrogène et B représente une base nucléique naturelle, en particulier les produits pour lesquels J = OH, ou oligomères mixtes en comprenant, constitueront soit des médicaments à activité antitumorale lorsque le gène visé ou son ARN messager code pour une protéine impliquée dans la transformation cellulaire, soit des médicaments capables de supprimer le caractère de résistance aux antiviraux, aux antibiotiques ou aux antiparasitaires lorsque la protéine codée est responsable de l'inactivation des antibiotiques, des antiviraux ou des antiparasitaires.

Des effets cytotoxiques spécifiques peuvent être obtenus par action d'un produit selon l'invention, notamment de formule (I), ou d'un produit de formule générale (I) dans laquelle L représente un atome d'oxygène, R et R' représentent chacun un atome d'hydrogène et B représente une base naturelle, en particulier les produits pour lesquels J = OH, ou oligomères mixtes en comprenant, sur une fonction cellulaire indispensable à la cellule cible.

Les oligomères mixtes d'oligothioribonucléotides selon l'invention, comprenant une séquence oligonucléotidique d'ADN où l'oxygène intracyclique est rétabli, sont particulièrement intéressants dans une utilisation comme agent antisens. Ces oligomères mixtes sont susceptibles d'être hautement sélectifs dans la mesure où la "fenêtre" de structure ADN est seule substrat de la RNAse H après appariement de l'antisens avec sa séquence cible complémentaire d'$ARN_m$, la dite RNAse H induisant alors la coupure du complexe ADN/ARN.

D'autres caractéristiques et avantages de la présente invention apparaîtront à la lumière des exemples qui vont suivre.

La Figure 1 représente la courbe de stabilité thermique du duplex bêta$dT_5(S_rT)dT_6$/bêta$dC_2A_{12}C_2$ comparativement à celle du duplex naturel bêta$dT_{12}$/bêta$dC_2A_{12}C_2$.

La Figure 2 représente la courbe de digestion enzymatique de bêta($S_r$T)d$T_{11}$.

Dans la description qui suit, l'abréviation rS ou $S_r$ précède un thioribonucléotide.

I-SYNTHESE DE THIONUCLEOTIDES.

I . 1- Les premières synthèses de thiosucres ont été effectuées entre 1960 et 1970. Deux exemples sont donnés:

La synthèse de derivés du 4-thio-D-ribofurannose se fait selon le schéma 1 :

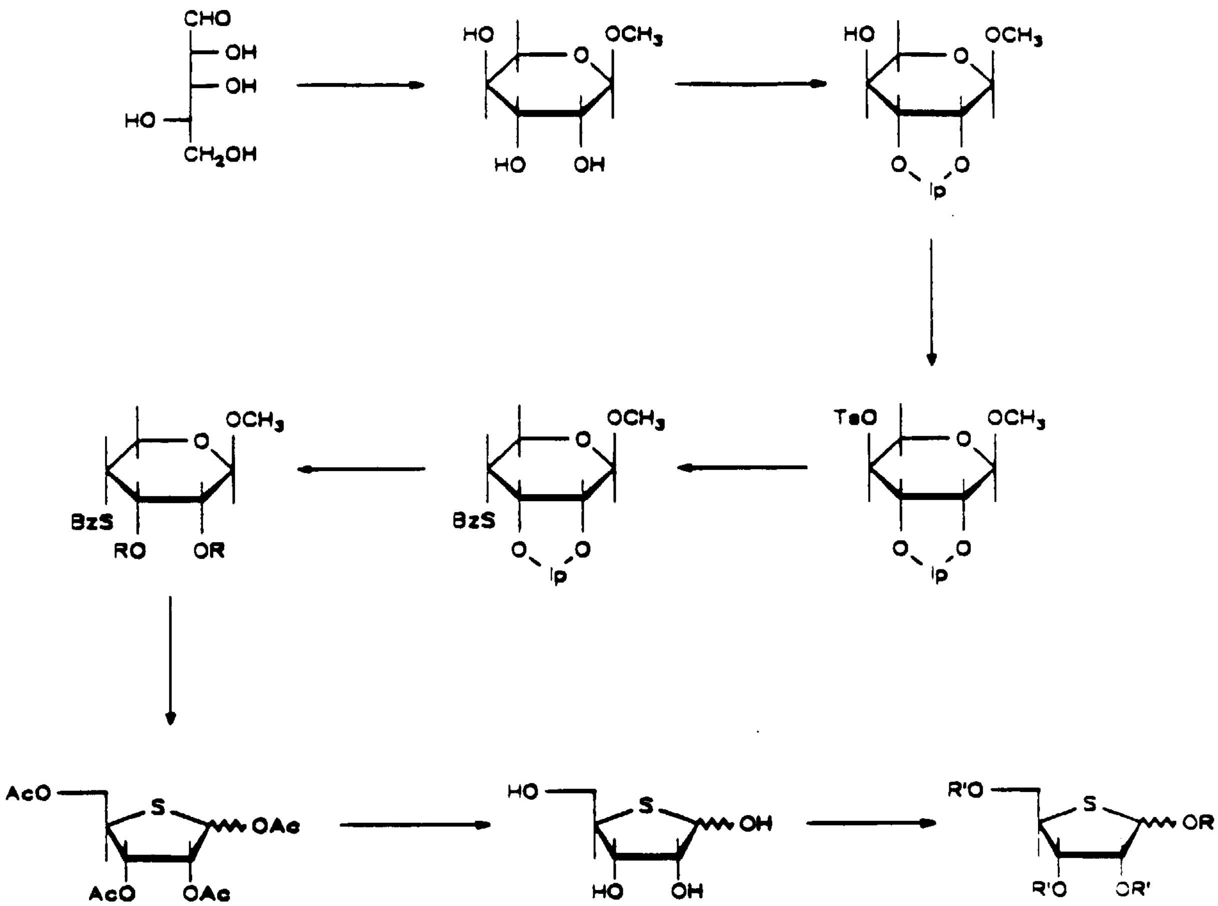

SCHEMA 1

Ce composé est obtenu en 8 étapes à partir du L-Lyxose avec un rendement global de 6 %.

- R. L. WHISTLER, W. E. DICK, T. R. INGLE, R. M. ROWELL et B. URBAS, *J. Org. Chem.*, 1964, **29**, 3723-3725.
- B. URBAS et R. L. WHISTLER, *J. Org. Chem.*, 1966, **31**, 813-816.
- E. J. REIST, D. E. GUEFFROY et L. GOODMAN, *J. Am. Chem. Soc.*, 1964, **86**, 5658-5663.
- R. L. WHISTLER et J. N. BeMILLER dans "*Methods in Carbohydrate Chemistry*", R. L. WHISTLER et M. L. WOLFROM, Eds., Academic Press, Inc., New york, 1962. **Vol. I**, p.79.

**Les dérivés du 4-thio-2-désoxy-D-ribofurannose** ont été obtenus selon le schema 2 :

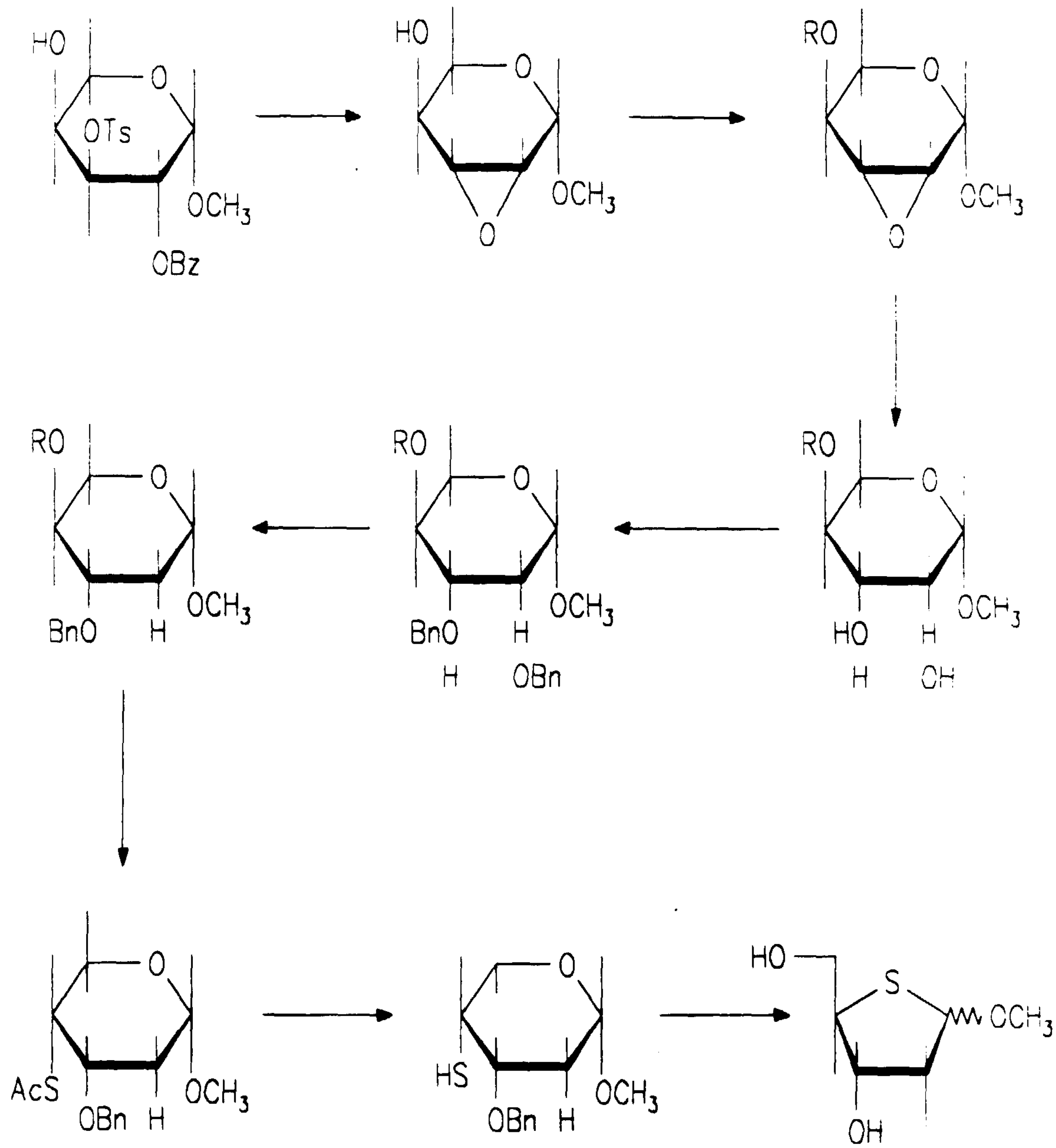

SCHEMA 2

Cette synthèse en 14 étapes conduit au methyl 2-désoxy 4-thio-D-erythro-pentofurannoside avec un rendement global de 8 %.

- Y. L. FU et M. BOBEK, *J. Org. Chem.*, 1976, **41**, 3831-3834.
- Y. L. FU et M. BOBEK, dans *"Nucleic Acid Chemistry"*, L. TOWSEND et R. S. TIPSON,Eds., 1978. **Part I** pp. 183-194.
- U. G. NAYAK et R.L. WHISTLER *Liebigs Ann. Chem.*, 1970, **741**, 131-138.

De façon semblable, **les dérivés du 4-thio-D-arabinofurannose** ont été obtenus:

- R. L. WHISTLER, U. G. NAYAK et A. W. PERKIN Jr., *J. Org., Chem.*, 1970. **35**, 519-521.

I. 2. Les nucléosides correspondants ont ensuite été synthétisés:

**a) En serie 4'-thio-2'-désoxy-D-ribofurannose**

- Y. L. FU et M. BOBEK dans *"Nucleic Acid Chemistry",* L. TOWSEND, R. L. TIPSON, Eds., John Wiley & Sons, New York, 1978, p. 317.

**b) En serie 4'-thio-D-ribofurannose**

- E. J. REIST, D. E. GUEFFROY et L. GOODMAN, *J. Am. Chem. Soc.,* 1964, **86**, 5658-5663.
- E. J. REIST, D. E. GUEFFROY et L. GOODMAN, *Chem. Ind.,* **1964**, 1364-1365.
- B. URBAS et R. L. WHISTLER, *J. Org. Chem.,* 1966, **31**, 813-816.
- M. BOBEK, R. L. WHISTLER et A. BLOCH, *J. Med. Chem.,* 1970, **13**, 411-413
- M. BOBEK, A. BLOCH, R. PARTHASARATHY et R. L. WHISTLER, *J. Med. Chem.,* 1975, **18**, 784- 787.
- M. BOBEK, R. L. WHISTLER et A. BLOCH, *J. Med. Chem.,* 1972, **15**, 168-171.
- N. OTOTANI et R. L. WHISTLER, *J. Med. Chem..* 1974, **17**, 535-537.
- M. W. PICKERING, J. T. WITKOWSKI et R. K. ROBBINS, *J. Med. Chem.*, 1976, **19**, 841-842.
- A. K. M. ANISUZZAMAN et M. D. AMIN, *J. Bangladesh Acad. Sci.,* 1978, **2**, 59-64
- D. J. HOFFMAN et R. L. WHISTLER, *Biochemistry,* 1970, **9**, 2367 - 2370.

**c) Dans d'autres series:**

- R.G.S. RITCHIE et W. A. SZAREK, *J. Chem. Soc. Chem. Commun.,* **1973**, 686.
- E. J. REIST, L. V. FISCHER et L. GOODMAN, *J. Org. Chem.,* 1968, **33**, 189-
- R. L. WHISTLER, L. W. DONER et U. G. NAYAK, *J. Org. Chem.,* 1971, **36**, 108- 110.

Ces nucléosides sont obtenus par les voies traditionnelles de la synthèse des nucléosides en série oxygénée.

**Soit par la méthode aux sels de métaux lourds:**

Traitement de l'hétérocycle chloromercurique avec le chlorosucre correspondant.

- M. BOBEK, R. L. WHISTLER et A. BLOCH, *J. Med. Chem.,* 1972, **15**, 168-171.
- D. E. GUEFFROY et L. GOODMAN, *Chem. & Ind.,* **1964**, 1364-1365.
- E. J. REIST, M. BOBEK, R. L. WHISTLER et A. BLOCH,*J. Med Chem.,* 1970, **13**, 411-413.
- E. J. REIST, D. E. GUEFFROY et L. GOODMAN, *J. Am. Chem.Soc..* 1964. **86**, 5658-5663.
- E. J. REIST, L. V. FISCHER et L. GOODMAN, *J. Org. Chem..* 1968, **33**, 189-192.

**Soit par la méthode de HILBERT et JOHNSON**,

- par condensation de la base pyrimidinique avec le thiochlorosucre correspondant.
- B. URBAS et R. L. WHISTLER, *J. Org. Chem..* 1966, **31**, 813-816.
- par condensation des dérivés silylés des bases et des thiochlorosucres correspondants.
- M. BOBEK, A. BLOCH, R. PARTHASARATHY et R. L. WHISTLER, *J. Med., Chem.*, 1975, **18**, 784-787.
- R. L. WHISTLER, L. W. DONER et U. G. NAYAK, *J. Org. Chem.,* 1971, **36**, 108-110
- N. OTOTANI et R. L. WHISTLER, *J. Med. Chem.,* 1974, **17**, 535-537.

- **par la reaction de fusion** du 3-cyano 1,2,4 triazole et du 1,2,3,5 tétra-O-acétyl-4-thio-D-ribofurannose.

- M. V. PICKERING, J. T. WITKOWSKI et R. K. ROBINS, *J. Med. Chem.,* 1976, **19**, 841-842.

- **par la méthode de VORBRUGGEN et NIEDBALLA** par condensation de l'adenine et du 1,2,3,5 tétra-O-acétyl thioribofurannose en présence de catalyseur FRIEDEL et CRAFTS.

- A. K. M. ANISUZZAMAN et M. D. AMIN, *J. Bangladesh Acad. Sci.,* 1978, **2**, 59-64.

**Depuis peu, la synthèse de thionucléosides a de nouveau connu de l'intérêt.**
Ainsi, le 2',3',5' tri-O-benzyl 4'-thio-β-D-xylofurannosyl uracile a été obtenu par une voie originale selon le schema 3:

- M. W. BREDENKAMP, C. W. HOLZAPFEL et A. D. SWANEPOEL, *Tétrahedron Lett.*, 1990, **31**, 2759-2762.
- M. W. BREDENKAMP, C. W. HOLZAPFEL et A. D. SWANEPOEL, *S. Afr. J. Chem.*, 1991, **44**, 31-33.

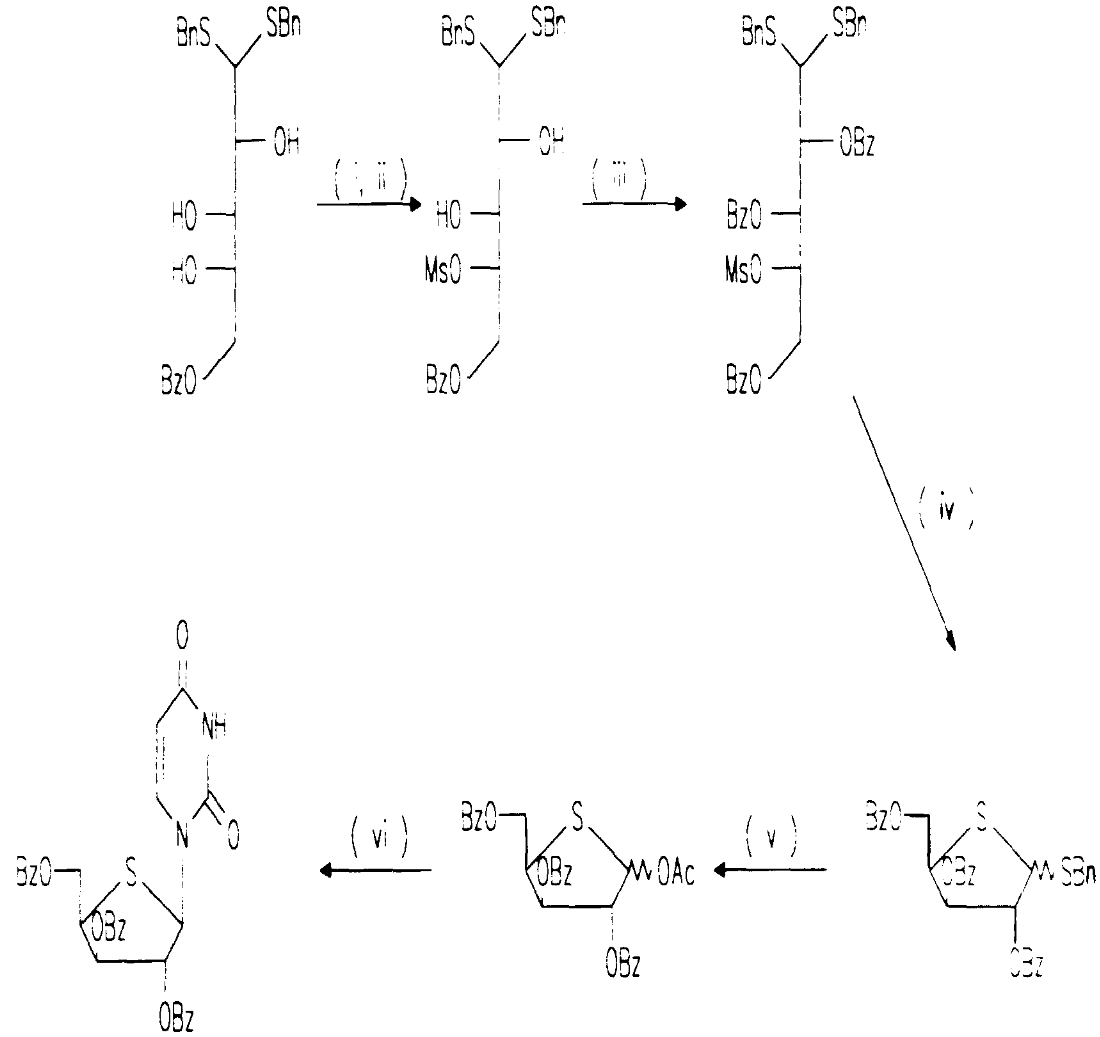


(i = oxyde de dibutyl étain; ii = MsCl; iii = BnCl; iv = iodure de tétra-butylammonium, carbonate de barium; v = $Hg(OAc)_2$ / AcOH; vi = Méthode d'HILBERT JOHNSON modifiée.)

SCHEMA 3

La synthèse d'analogues pyrimidiniques de 4'-thio 2'-désoxy nucléosides a été publiée.

- M. R. DYSON, P. L. COE et R. T. WALKER, *J. Med. Chem.,* 1991, **34**, 2782-2786. par la méthode de HORTON et MARKOVS.
- D. HORTON et R. A. MARKOVS, *Carbohydrate Res.,* 1980, **80**, 356. Elle est illustrée par le schéma 4 :

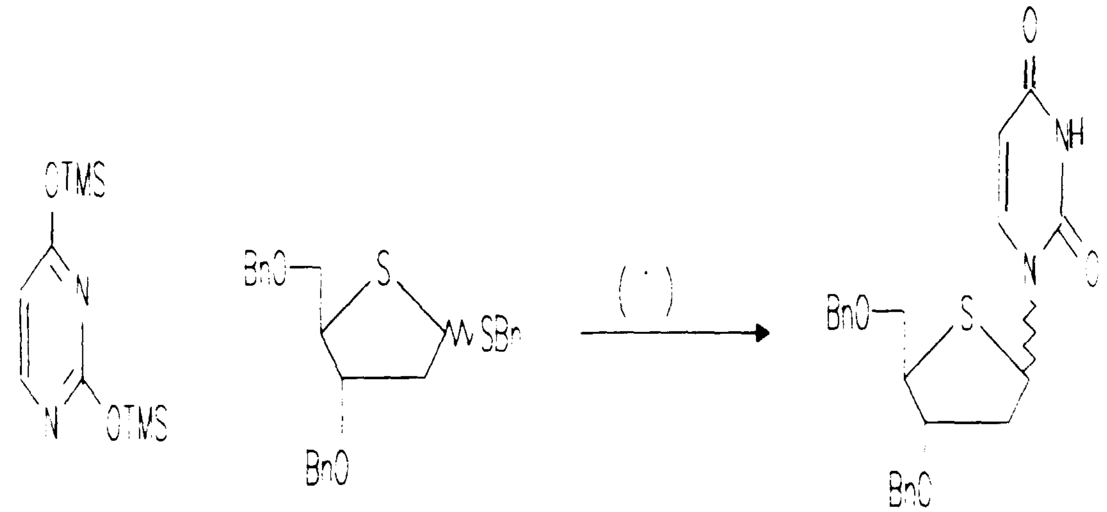

(i = $HgCl_2$. $CdCO_3$)

SCHEMA 4

D'une façon semblable, les 2'-désoxy-4'-thiocytidine, 2'-désoxy-4'-thiouridine et 4'-thiothymidine ont été obtenus par J. A. SECRIST III et Coll. (J. A. SECRIST, K. N. TIWARI. J. M. RIORDAN et J. A. MONTGOMERY, *J. Med. Chem.,* 1991, **34**, 2361-2366); (J. A. MONTGOMERY et J. A. SECRIST III, PCT Int. Appl. WO 9104.033 ) à partir du 2-désoxy-4-thio-β-D-érythro-pentofurannose synthétisé selon BOBEK et Coll. (Y. L. FU, M. BOBEK, *J. Org. Chem.,* 1976, **41**, 3831-3834.) en utilisant le TMSTf comme catalyseur de couplage.

La synthèse du 4'-thio 2'-désoxy-D- ribofurannose a fait l'objet d'un brevet et de publications :

- European Patent Application, 0421 777 A1. R. T. WALKER.
- M. R. DYSON, P. L. COE et R. T. WALKER, *J. Chem. Soc. Chem. Comm.*, **1991**,741-742.
- M. R. DYSON, P. L. COE et R. T. WALKER, *Carbohydrate Res.,* 1991, **216**, 237-248.

Elle est décrite sur le schéma 5 :

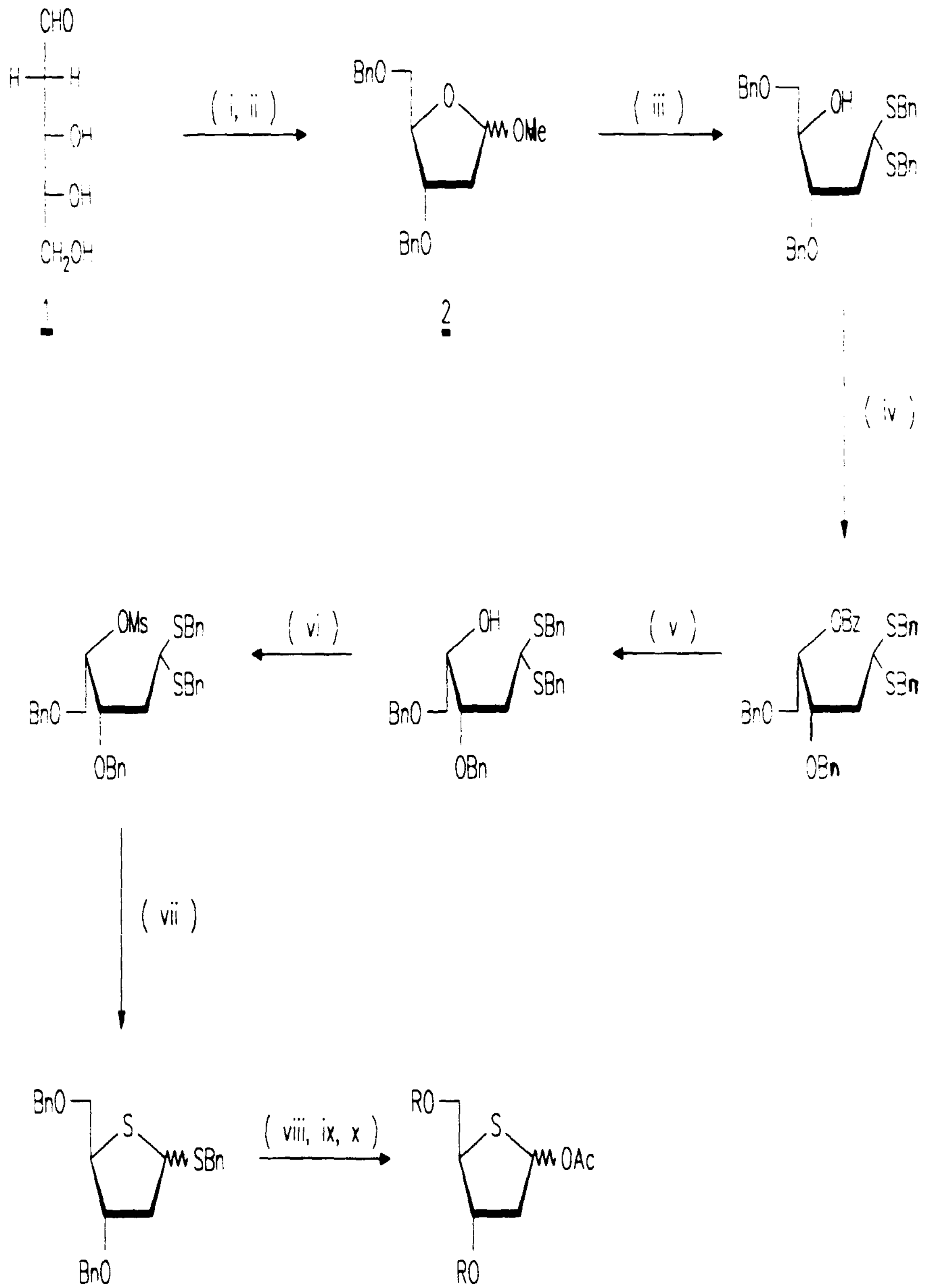


(i = HCl / MeOH, ii = BnBr / THF, iii = BnSH / HCl, iv = $Ph_3P$ / DEAD / BzOH / THF, v = MeONa / MeOH / $CH_2Cl_2$, vi = MsCl / Pyr., vii = NaI / $CO_3Ba$ / Acetone. viii = $BCl_3$ / $CH_2Cl_2$, ix = p-toluoylCl, x = $Ac_2O$ / $H_2SO_4$)

SCHEMA 5

Des ribothionucléosides dérivés des pyrimidines ont fait l'objet du même brevet. Mais la synthèse du thioribofurannose de départ a été réalisée selon la méthode de E. J. REIST. - E. J. REIST, D. E. GUEFFROY et L. GOODMAN, *J. Am. Chem. Soc.*, 1964, **84**, 5658.

I. 3.SYNTHESE DES SYNTHONS DU 4'-THIO-BETA-D-RIBOFURANNOSE NECESSAIRES A L'OBTENTION DE THIOOLIGOMERES.

On a réalisé la synthèse du L-thioLyxofurannose et du D-thioRibofurannose en 7 étapes à partir du D-ribose et du L-lyxose respectivement avec 29 et 21 % de rendement respectivement. Cette synthèse est illustrée sur le schéma 6.

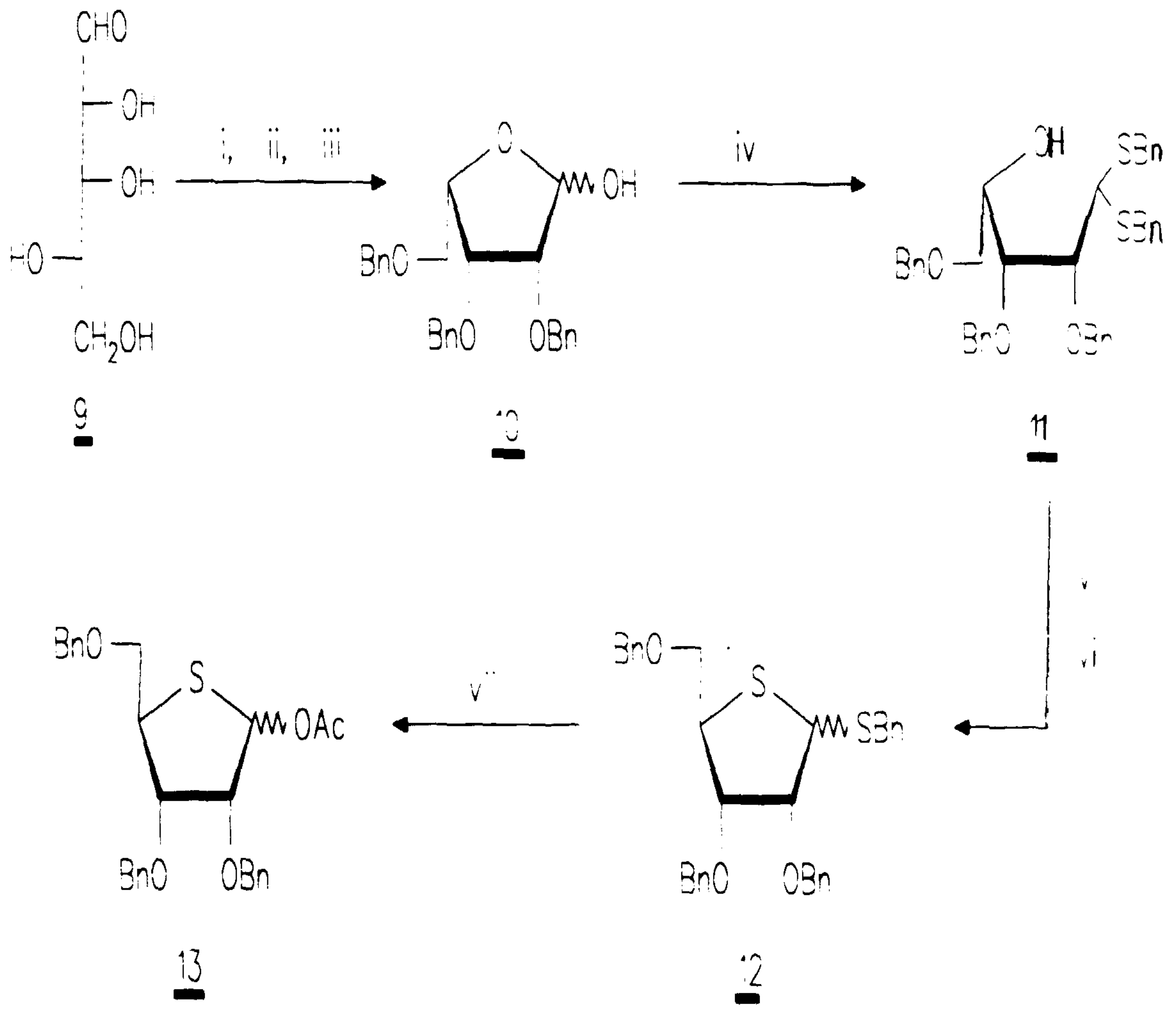


9

10

11

13

12

(i = MeOH, HCl: ii = BnBr, KOH; iii = HCL,$H_2O$.dioxane; iv = BnSH. HCl; v = MesCL, pyr.; vi = $NBu_4I$, $BaCO_3$; vii = $Hg(OAc)_2$)

SCHEMA 6

La synthèse pour obtenir le thio-D-ribofurannose utilise le L-lyxose comme produit de départ comme dans la synthèse de WHISTLER ( page 19). Cette stratégie est appelée stratégie C1---C4. En effet, l'atome de soufre est tout d'abord introduit en position anomère et une substitution nucléophile SN2 entre l'atome de soufre porté par le carbone anomère (C1) et le carbone C4 porteur d'un OH activé par un groupement Mesyl (Ms) est ensuite effectuée. Cette stratégie appliquée à la synthèse du 4-thio-D-ribofurannose et du 4-thio-L-lyxofurannose ressemble à celle que WALKER a utilisé pour parvenir à la série 2-desoxy-D-ribofurannose ( Brevet Eur. Pat. 0421 777 A 1 page 6). Par exemple, le produit 5 (Schéma 5) de WALKER est semblable à notre produit 10 (Schema 6) en série L-Lyxofurannose.

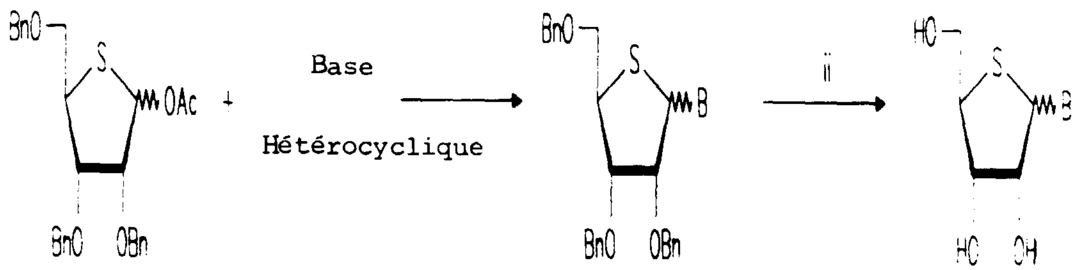


13

B = T 14α, 14β — B = T 18α, 18β

B = U 15α, 15β — B = U 19α, 19β

B = C 16α, 16β — B = C 20α, 20β

B = A 17α, 17β — B = A 21α, 21β

(i = BSA, TMSTf, $CH_3CN$; ii = $BBr_3$, $CH_2Cl_2$)

SCHEMA 7

La synthèse des thionucléosides de l'uracile, de la thymine et de la cytosine a été effectuée en utilisant le bis-triméthylsilyl acétamide (BSA) comme agent silylant et le triméthylsilyl trifluorométhane sulfonate (TMSTf) comme agent de couplage. Les nucléosides O et β ont été obtenus avec 74 , 77 et 70 % de rendement respectivement . La synthèse des thionucleosides de l'adenine a été réalisée par le SnC14 dans l'acetonitrile. Les nucleosides O et β sont obtenus avec 58 % de rendement (Schéma 7).

**II. SYNTHESE D'OLIGO-THIONUCLEOTIDES SUR SUPPORT SOLIDE.**

La synthèse supportée des β oligo-thioribonucléotides a été effectuée à l'aide d'un synthétiseur GENE modèle APPLIED BIOSYSTEM 318A selon la méthode aux phosphoramidites.

Elle est semblable à celle décrite pour la série β oligodésoxyribonucléotide naturelle par CARUTHERS et Coll. (S. L. BEAUCAGE et M. H. CARUTHERS, *Tétrahedron Lett.,* 1981, **22**, 1859-1862; L. J. Mc BRIDE et M. H. CARUTHERS, *Tétrahedron Lett.,* 1983, **24**, 245-248.)

et à celle décrite par USMAN et Coll.pour les oligoribonucléotides (N. USMAN, K. K. OGILVIE, M. Y. JIANG et R. J. CEDERGREN, *J. Am. Chem. Soc.,* 1987, **109**, 7845-7854.).

Cette synthèse a nécessité:

I) La préparation de synthons β thioribonucléoside phosphoramidites **5** correspondant aux bases T, U, C, A et G.

II) La fonctionnalisation du support solide incorporant un dérivé β thioribonucléosidique.

III) L'élongation de la chaîne oligothioribonucléotidique à l'aide d'un synthétiseur automatique.

IV) Enfin, au terme du nombre requis de cycles de synthèse, l'oligothionucléotide a été décroché de son support, déprotégé et purifié.

**II.1 - Synthèse du phosphoramidite des bêta-thioribonucléoside 26 a-d.**

II. 1. 1 Synthèse des dérivés 5'-DmTr-2'-O-TBDMS (Schéma 8)

Le problème majeur dans la synthèse chimique des oligoribonucléosides est la présence de l'hydroxyle en 2' dans le cycle ribose ou thioribose qui demande une protection sélective.

La sélection d'un groupement protecteur pour l'hydroxyle en 2' doit correspondre à plusieurs critères, il doit être stable:

- sous les conditions de couplage pendant la déprotection des groupements protecteurs en 5' pour permettre l'extension de la chaîne.
- pendant l'oxydation dans le cas de la méthode aux phosphites triesters.
- pendant le masquage qui suit le couplage.
- pendant la déprotection des groupements protecteurs des amines exocycliques.
- pendant la déprotection des phosphates.
- et dans le cas des synthèses sur support solide pendant le clivage de l'oligomère du support.

Finalement ce groupement protecteur doit être enlevé sous des conditions très douces pour éviter une attaque de la fonction hydroxyle en 2' libérée sur la liaison phosphodiester adjacente.

Un nombre important de stratégies pour protéger les hydroxyles en 2' ont été développées et illustrent les difficultés dans le choix du schéma de protection des groupements hydroxylés. (C. B. REESE, *Nucléosides Nucléotides,* 1985, **4**, 117-127; R. KIERZECK, M. H. CARUTHERS, C. E. LONGFELLOW, D. SWINTON, D. H. TURNIER et S. M. FREIER, *Biochemistry*, 1986, **25**, 7840-7846; S. IWAI et E. OHTSUKA, *Nucleic Acids Res.*, 1988, **16**, 9443-9456;

C. LEHMANN, Y. Z. XU, C. CHRISTODOULOU, Z. K. TAN et M. J. GAIT*, Nucleic Acids Res.,* **1989**, **17**, 2379-2390; T. TANAKA, S. TAMATSUKURI, et M. IKEHARA, *Nucleic Acids Res.,* 1986, **14**, 6265-6279.).

L'utilisation de groupements protecteurs alkylsilyl et notamment le *tert*-butyldiméthylsilyl (TBDMS) pour l'hydroxyle en 2' a facilité la synthèse des oligoribonucléotides. Le groupement TBDMS est suffisament stable dans des conditions acides ou basiques et est facilement enlevé par le fluorure de *tétra*-butyl ammonium dans le THF pour être utilisé dans la stratégie en phase solide (N. USMAN, K. K. OGILVIE, M. Y. JIANG et R. L. LEDERGEN, *J. Am. Chem. Soc.,* 1987, 109, **7845-7854**; N. USMAN, K. NICOGHOSIAN, R. L. CEDERGREN et K. K. OGILVIE, *Proc. Natl. Sci. USA,* 1988, **85**, 5764-5768.; S. A. SCARINGE, C. F. FRANCKLYN et N. USMAN, Nucleic Acids Res., 1990, **18**, 5433-5441, K. K. OGILVIE et M. J. NEMER, *Tétrahedron Lett.,* 1980, **21**, 4159, R. T. PON et K. K. OGILVIE, *Nucléosides Nucléotides,* 1984, **3**, 485; R. T. PON et K. K. OGILVIE, *Tétrahedron Lett.,* 1984, **25**, 713.).

On a donc appliqué en série β oligothioribonucléotide le schéma de protection utilisé pour les nucléosides oxygénés (Schéma 8).

A savoir les protections :

- Benzoyl pour l'adénine et la cytosine, isoButyl pour la guanine en tant que protection des groupements amino exocycliques.
- Diméthoxytrityl (DmTr) pour protéger les hydroxyles primaires en 5'.
- *tert*-butyldiméthymsilyl (TBDMS) pour protéger l'hydroxyle secondaire en 2'.
- Methoxy pour la protection des phosphates.

La première étape permet de protéger l'hydroxyle libre en 5' par un groupement acide labile le DmTr (Schéma 8). Ainsi le dérivé nucléosidique **22** est traité par le chlorure de diméthoxytrityle ( 1,27 équivalents molaires) dans la pyridine anhydre sous atmosphère inerte. Après traitement usuel du mélange réactionnel, le dérivé diméthoxytritylé **23** est purifié par chromatographie sur gel de silice et isolé avec des rendements variant de 70 à 80 %.

Les caractéristiques physico-chimiques de ces dérivés **23** ( R.M.N., Masse) sont en accord avec les strutures proposées.

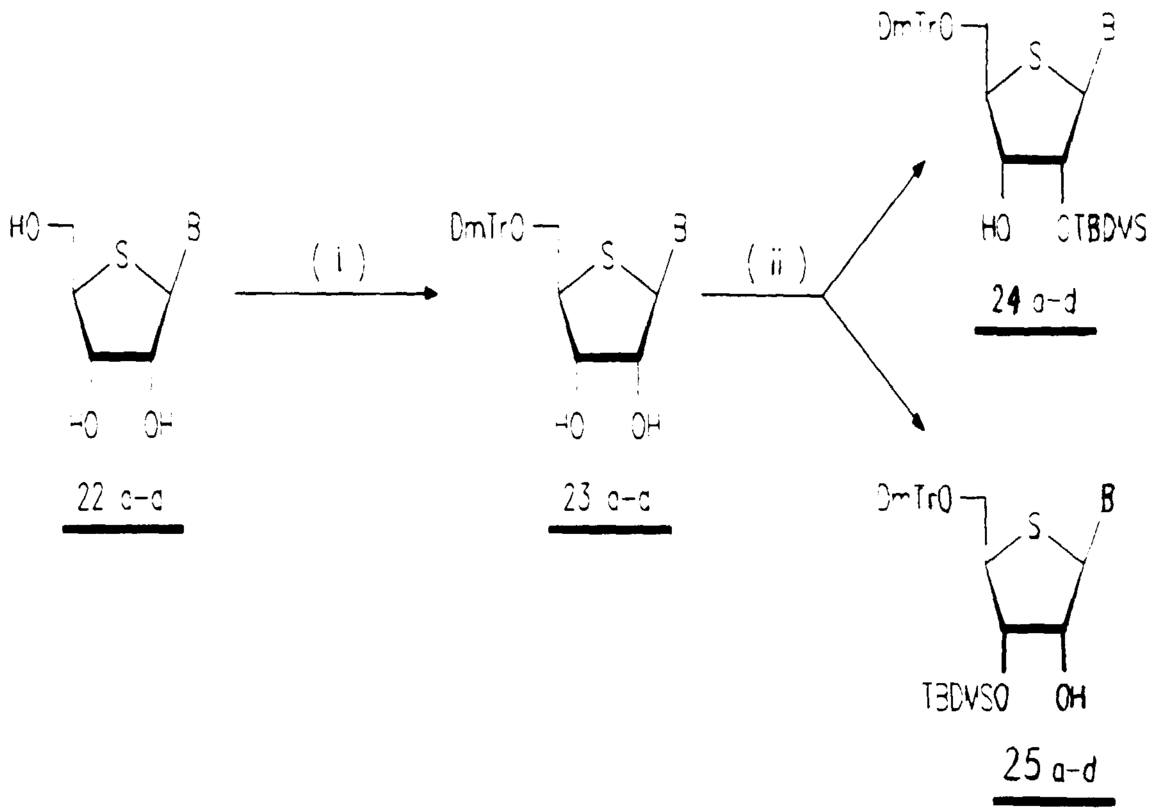


22 a-e

23 a-e

24 a-d

25 a-d

( i = DmTrCl. pyr. ii = TBDMSCl. pyr.)

Les abréviations ont les significations suivantes:

a- B = T = Thymine

b- B = U = Uracile.

c- B = CBz = N-Benzoyl-4-Cytosine.

d- B = ABz = N-Benzoyl-6-Adenine.

e- B = GPal = N-Palmitoyl-2-Guanine.

Dmtr = Diméthoxy-4,4'-trityl. iPr = Isopropyl. Bz = Benzoyl. TBDMS = tert-butyldiméthylsilyl. $(iPr)_2P(Cl)OMe$ = Chloro. (N.N diisopropylamino.méthoxyphosphine)

SCHEMA 8

La deuxième étape (Schéma 8) consiste en la protection de l'hydroxyle secondaire en 2' Or, durant la silylation un mélange de 2'-O-silyl **24** et de son régioisomère 3'-O-silyl **25** est obtenu (K. K. OGILVIE et D. W. ENTWISTLE, *Carbohyd. Res.*, 1981, **89**, 203-210, K. K. OGILVIE, A. L. SCHIFMAN et C. L. PENNEY, *Can. J. Chem.*, 1979, **57**, 2230, G. H. HAXIMELAHI, Z. A. PROBA et K. K. OGILVIE, *Can J. Chem.*, 1982, **60**, 1106.). Ces isomères doivent être séparés avec beaucoup de soins sur colonne de gel de silice afin d'obtenir le dérivé 2'-O-TBDMS **24** avec une pureté supérieure à 99,95 % mesurée par CLPH.

En effet, si ce n'est pas le cas, les étapes ultérieures nous conduiront à des mélanges d'oligothionucléotide 5'-3' attendus avec l'isomère indésirable 5'-2'. En conséquence, la pureté du 5'-O-DmTr-2'-O-silyl **24** est primordiale pour une bonne synthèse.

Ainsi, l'obtention des composés 5'-DmTr-2'-O-TBDMS **24** a tout d'abord été réalisée selon la méthode d'OGILVIE et Coll. (K. K. OGILVIE, A. L. SCHIFMAN et C. L. PENNEY, *Can. J. Chem.*, 1979, **57**, 2230.) qui consiste à condenser **23** avec du chlorure de *tert*-butyldiméthylsilyle (TBDMSC1) (1.2 equivalent molaire) dans la pyridine en présence d'imidazole (2,6 equivalents molaires). En fin de réaction, on obtient un mélange de dérivé 2'-O-TBDMS **24** et de son isomère 3'-O-TBDMS **25**. Le composé attendu **24** est séparé du mélange par chromatographie sur colonne de gel de

silice.
Il est ensuite possible par une réaction d'équilibration d'obtenir **<u>24</u>** à parir de son isomère 3'-O-TBDMS **<u>25</u>** (S. S. JONES et C. B. REESE, *J. Chem. Soc. Perkin Trans* I, **1979**, 2762.). Cette migration du groupement TBDMS est intramoléculaire et s'effectue *via* un intermédiaire possédant un atome de silicium pentavalent. L'isomérisation du dérivé 3'-O-TBDMS obtenu précédemment après une première séparation chromatographique est effectuée dans l'éthanol à température ambiante pendant une nuit. Les deux isomères de position ainsi obtenus sont à nouveau séparés par chromatographie sur colonne de gel de silice. Cette opération d'isomérisation suivie d'une purification est répétée trois fois. Cependant les rendements en 5'-DmTr-2'-O-TBDMS **<u>24</u>** obtenus par cette méthode (40 à 50 % selon les bases) ne nous ont pas paru satisfaisants. Nous en avons utilisé une autre, plus récente, mise au point par OGILVIE ( G. H. HAKIMELAHI, Z. A. PROBA et K. K. OGILVIE, *Can. J. Chem.*., **1982**, 60, 1106.) qui permet l'introduction préférentielle du TBDMS en position 2' des ribonucléosides β. Cette technique préconise l'utilisation de sels d'argents.

Ainsi le 5'-diméthoxytrityl **<u>23</u>** est condensé avec du chlorure de TBDMS (1,3 équivalents molaires) en présence de nitrate d'argent (1.2 équivalent molaires) et de 3,7 équivalents de pyridine dans le THF anhydre. Après purification par chromatographie sur colonne de gel de silice on obtient 56 % de rendement en **24** et 27 % en 5'-DmTr-3'-O-TBDMS **25**. En conséquence, le 5'-DmTr-2'-O-TBDMS **<u>24</u>** est obtenu dans ces nouvelles conditions en une seule étape avec un meilleur rendement mais la sélectivité d'introduction du groupement TBDMS en 2' n'est pas améliorée.

II.1.2 Synthèse des dérivés phosphoramidites **<u>26 a-d</u>** (Schéma 9)

Les quatre β thioribonucléosides phosphoramidites N-protégés **<u>26 a-d</u>** sont ensuite obtenus à partir des 5'-DmTr-2'-O-TBDMS β thioribonucléosides N-déprotégés correspondants **<u>24</u>** (Schéma 9).

Le dérivé nucléosidique **<u>24</u>** a été traité par du chloro N, N diisopropylamino méthoxy phosphine ( 2,5 équivalents molaires) en présence de N,N,N diisopropyl éthylamine (DIEA) dans le dichlorométhane sous atmosphère inerte. Le dérivé phosphoramidite **<u>26</u>** a été purifié par chromatographie sur gel de silice et lyophilisé dans le benzène, les phosphoramidites **<u>26 a-d</u>** sont obtenus avec des rendements variant de 78 à 80 % selon la nature de la base.

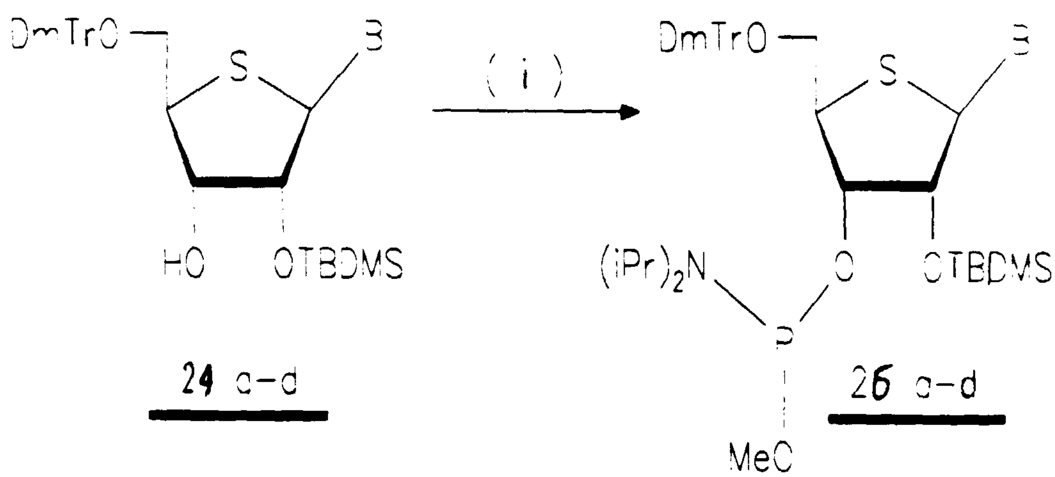


24 a-d

26 a-d

i = $(iPr)_2P(Cl)OMe$. DIEA. DMAP. $CH_2Cl_2$)

SCHEMA 9

Les analyses des spectres de RMN du $^{31}P$ et du $^{1}H$ de **<u>26</u>** montrent clairement que nous obtenons une seule paire de diastéréoisomères ce qui établit la pureté régioisomérique des composés obtenus.
En effet, quelques auteurs (R. KIERZEK, M. H. CARUTHERS, C. E. LONGFELLOW, D. SWINION, D. H. TURNER et S. M. FREIER, *Biochemisitry,* 1986, 25. 7840-7846.) avaient émis l'hypothèse que les groupements silylés en position 2' n'étaient pas stables dans les conditions de la phosphorylation. Cependant il a été montré (W. K. KOHLER, W. SCHLOSSERM, G. CHARUBALA et W. PFLEIDLERER, *Chemisitry and Biology of Nucléosides and Nucléotides,* Academic Press, New York, 1978, pp 347-358: K. K. OGILVIE et D. W. ENTWISTLE, *Carbohydrate Res.,* 1981, **89**, 203-210; N. USMAN, K. K. OGILVIE, M. Y. JLANG et R. J. CEDERGREN, *J. Am. Chem. Soc.,* 1987, **109**, 7845-7854.) que les groupes alkylsilyles migrent dans les solvants pratiques comme le méthanol ou dans les solutions aqueuses comme

le mélange pyridine/eau, mais ces groupements silylés sont stables dans les solvants anhydres et notamment dans les bases non protiques comme la pyridine anhydre (K. K. OGILVIE, D. W. ENTWISTLE, *Carbohydrate Res.,* 1981, **89**, 203-210; W. KOHLER, W. SCHLOSSER, G. CHARUBALA et W. PFLEIDERER, *Chemisitry and Biology of Nucleosides and Nucleotides,* Academic Press, New York, 1978, pp 347-358.). D'autre part, il a été clairement démontré en série ribonucléotide que l'utilisation des 2'-O-silyl ribonucléosides dans la synthèse d'oligoribonucléotides conduit exclusivement à des liaisons 5'-3' (K. K. OGILVIE, M. J. NEMER, *Tetrahedron Lett.,* 1980, **21**, 4159; R. T. PON et K. K. OGILVIE, *Tetrahedron Lett.,* 1984, **25**, 713; R. T. PON, et K. K. OGILVIE, *Nucleosides Nucleotides*, 1984, **3**, 485;P. J. GAREGG, I. LINDH, J. STAMINSKI, et R. STROMBERG, *Tetrahedron Lett.,* 1986, **27**, 4055-4058). La synthèse de phosphoramidites n'est pas stéréospécifique et la formation en quantités égales des deux diastéréoisomères est due à la chiralité de l'atome de phosphore de configuration R ou S. La formation de ces deux produits n'est pas gênante dans la mesure où les réactions d'oxydation ultérieures suppriment cette chiralité.

II. 2. PARTIE EXPERIMENTALE

**Méthode générale pour la préparation des 5'-O-diméthoxytrityl N-acyl 4'-thio-β-D-ribonucléosides 23 a-d.**

A une solution de N-acyl β-D-thioribonucléoside **23 a-d** (1mmole) dans la pyridine anhydre (5.6ml) est ajouté du chlorure de diméthoxytrityle (1.27mmole, 430mg). Le mélange réactionnel est agité à température ambiante sous atmosphère inerte pendant 90 à 120 min puis du méthanol (1ml) est ajouté. Après 10 minutes d'agitation supplémentaire, le mélange réactionnel est versé sur une solution aqueuse saturée de bicarbonate de sodium (25ml) et les produits sont extraits avec du dichlorométhane (2 x 20ml). Les phases organiques sont lavées avec de l'eau (2 x 100ml) puis séchées sur sulfate de sodium et filtrées. Le filtrat est évaporé sous pression réduite et le résidu obtenu est chromatographié sur une colonne de gel de silice. L'élution est effectuée avec un mélange $CH_2Cl_2$/MeOH : 98/2 en présence de triéthylamine 1%; les fractions contenant le produit sont évaporées à sec et les nucléosides **23 a-d** sont obtenus sous forme d'une huile.

**1-[4'-thio-5'-O-diméthoxytrityle-β-D-ribofurannosyl]Thymine 23 a.**

Rendement: 68 %.

Spectrométrie de masse FAB>0 NOBA m/z = 577 [M+H]+, 303 [DmTr]+ RMN $^1$H (DMSO$d_6$, 360MHz) δ 11,24, ( s, 1H, NH); 7,44, ( s, 1H, $H_6$); 6.87-7.30, (m, 13 H, H Aromatiques); 5,83, (d, 1H, $H_{1'}$, $J_{1',2'}$ = 7,0); 5,53, (d, 1H, $OH_{2'}$, $J_{OH,2'}$ = 5,8); 5,30, (d, 1H, $OH_{3'}$, $J_{OH,3'}$ = 4,7); 4,14, (dd, 1H, $H_{2'}$, $J_{2',1'}$ = 6.9, $J_{2',3'}$ = 3,0); 4,00, (t,1 H, $H_{3'}$, $J_{3',2'}$ = 3,0, $J_{3',4'}$ = 3.0); 3,69, (s, 6H, OMe); 3,30, (dd, 1H, $H_{4'}$); 3,13, ( m, 2H, $H_{5'}$, $H_{5''}$); 1,60, (s, 3H, $CH_3$).

**1-[4'-thio 5'-O-diméthoxytrityle β-D-ribofurannosyl]Uracile 23 b.**

Rendement 70%.

RMN $^1$H (DMSO$d_6$, 360 MHz) δ 11.31, (s, 1 H, NH); 7.70, (d, 1H, $H_6$, $J_{6,5}$ = 8.0), 7.40-6 89, (m, 13 H, H aromatiques du groupement dmTr); 5.84, (d, 1H, $H_{1'}$, $J_{1',2'}$ = 5.7), 5.53, (d, 1H, $OH_{2'}$, $J_{OH2',2'}$ = 5.6), 5.48, (d, 1H, $H_5$, $J_{5,6}$ = 8.0); 5.26, (d, 1H, $OH_{3'}$, $J_{OH3',3'}$ = 4.45); 4.02, (m, 2H, $H_{2'}$, $H_{3'}$); 3.74, (s, 6H, OMe); 3.31, (m, 3H, $H_{4'}$, $H_{5'}$, $H_{5''}$). Spectromètrie de masse FAB>0 NOBA m.z = 563 [M+H]+, 303 [DmTr]+

**1-[4'-thio 5'-O-diméthoxytrityle β-D-ribofurannosyl]N-4-benzoylCytosine 23 c**

Rendement 69 %.

RMN $^1$H (DMSO$d_6$, 230 MHz) δ 11.32, (s, 1H, NH); 8.40, (d, 1H, $H_6$, $J_{6,5}$ = 7.5); 8.01, (d, 2H, Ho du groupement benzoyle); 7.63, (d, 1H, $H_5$, $J_{5,6}$ = 7.3); 7.44, (m, 17H, H aromatiques du groupement DmTr et Hm.p du groupement benzoyle); 5.88,(d, 1H, $H_{1'}$, $J_{1',2'}$ = 4.1); 5.73, (d, 1H, $OH_{2'}$, $J_{OH,2'}$ = 5.2); 5.27,(d, 1H, $OH_{3'}$, $J_{OH,H3'}$ = 5.7), 4.06,(m, 1H, $H_{2'}$,$J_{2',1'}$ = 4.0, $J_{2',3'}$ = 3.7, $J_{2',OH}$ = 5.2), 4.03,(m, 1H, $H_{3'}$, $J_{3'2'}$ = 3.7, $J_{3',4'}$ = 5.3, $J_{3',OH}$ = 5.7); 3.76, (s, 6H, $OCH_3$ du groupement DmTr); 3.43,(m, 3H, $H_{4'}$, $H_{5'}$et $H_{5''}$),

Spectrométrie de masse FAB>0 NOBA m/z = 666[M+H]+,517[M+H- ØCONH$_2$]+, 303 [DmTr] +

**1-[4'-thio 5'-O-diméthoxytrityle β-D-ribofurannosyl]N6-benzoylAdénine 23 d**

Rendement 70 %

RMN $^1$H (DMSO$d_6$, 230 MHz) δ 11.25,( s, 1H, NH); 8.63, (s, 1H, $H_8$); 8.57, (s, 1H, $H_2$); 8.04, (d, 2H, Ho du groupement benzoyle); 7.51, (m, 13H, H aromatiques du groupement DmTr); 6.92, (d, 3H, Hm,p du groupement benzoyle); 5.98, (d, 1H, $H_{1'}$, $J_{1',2'}$ = 5.8); 5.75, (d, 1H, $OH_{2'}$, $J_{OH,2'}$ = 3.5); 5.45, (d, 1H, $OH_{3'}$, $J_{OH,3'}$ = 2.5); 4.70, (m, 1H, $H_{2'}$), 4.26, (m,

1H, $H_{3'}$); 3.74,( s, 6H, $OCH_3$ du groupement DmTr); 3.53, (m, 3H, $H_{4'}$,$H_{5'}$ et $H_{5''}$). Spectrométrie de masse FAB>0 NOBA m/z = 690 [M+H]+. 303 [DmTr]+

**Méthode générale pour la préparation des 2'-O-*tert*-butyldiméthylsilyl-4'-thio-5'-O-diméthoxytrityl-N-acyl-β-D-ribonucléosides 24 a-e et de leur isomère 3'-TBDMS 25 a-e.**

A une solution de 5'-O-diméthoxytrityl 4'-thio N-acyl β-D-ribonucléoside **23 a-d** (1mmole) dans le THF anhydre (10ml) est ajouté du nitrate d'argent (1.2mmole, 203mg). Le mélange réactionnel est alors agité 5 minutes à température ambiante avant l'addition du chlorure de *tert*-butyldiméthylsilyle (TBDMSCl, 1.3mmole, 195mg) et de la pyridine anhydre (3.7mmole, 0.193ml). L'agitation à température ambiante est alors prolongée pendant 24 h, puis la solution hétérogène est filtrée avant d'être versée dans une solution aqueuse de bicarbonate de sodium à 5% (30ml). Les produits sont extraits avec du dichlorométhane (3x20ml) et les phases organiques sont lavées avec de l'eau puis séchées sur sulfate de sodium et filtrées. Le filtrat est évaporé à sec et le résidu est chromatographié sur une colonne de gel de silice en utilisant comme éluant un mélange de $CH_2Cl_2$/AcOEt : 95/5 puis de $CH_2Cl_2$/AcOEt:80/20 . Les fractions contenant le produit désiré (2'-O-TBDMS, **24 a-d**) sont regroupées et évaporées à sec. L'isomère 3'-O-TBDMS **25 a-d** est ensuite obtenu.

**1-[2'-O-*tert*-butyldiméthylsilyl-4'-thio-5'-O-diméthoxytrityl-β-D-ribofurannosyl] Thymine 24 a.**

Rendement: 33 %.
Spectrométrie de masse FAB>0 NOBA m/z = 691 [M+H]+, 713 [M+Na]+.RMN $^1H$ (DMSO$d_6$, 300MHz); δ 11,36, ( s, 1 H, NH); 7,58, ( s, 1 H, $H_6$); 7,33-6,90. ( m, 13 H, H Aromatiques); 5,89, ( s, 1 H, $H_{1'}$,$J_{1',2'}$ = 6,1); 5,27, ( m, 1 H, $OH_{3'}$); 4,20, ( dd, 1 H, $H_{2'}$,$J_{2',1'}$ = 6,10, $J_{2',3'}$ = 3,5); 3.97, (m, 1 H, $H_{3'}$); 3,73, ( s, 6 H, OMe); 3,36, ( m, 1 H, $H_{4'}$), 3,25, ( m, 2 H, $H_{5'}$, $H_{5''}$); 1.58, ( s, H, $CH_3$); 0.81, ( s, 9 H. *tert*-butyl); 0,0, ( d, 6 H, $Me_2Si$).

**1-[2'-O-*tert*-butyldiméthylsilyl-4'-thio-5'-O-diméthoxytrityl-β-D-ribofurannosyl] Uracile 24 b.**

Rendement 56% .
$T_f$= 114°C.
RMN $^1H$ (DMSO$d_6$, 360 MHz); δ 11.20, (s, 1 H, NH); 7.81, (d, 1 H, $H_6$, $J_{6,5}$ = 8.1); 7.30-6.89, (m, 13 H, H aromatiques du groupement dmTr) 5.84, (d, 1 H, $H_{1'}$, $J_{1',2'}$ = 5.5); 5.50, (d, 1 H, $H_5$, $J_{5,6}$ = 8.1); 5.23, (d, 1 H, $OH_{3'}$, $J_{OH3',3'}$ = 4.7); 4.11, (q, 1 H, $H_{2'}$,$J_{2',1'}$ = 5.5, $J_{2',3'}$ = 3.5); 3.95, (m, 1 H, $H_{3'}$); 3.74, (s, 6 H, OMe); 3.38, (m, 2 H, $H_{4'}$, $H_{5'}$ $J_{4',5'}$ = 3.28, (m, 1 H, $H_{5''}$); 0.82, (s, 9 H, *tert*-butyl); 0.05, (m, 6 H, $Me_2Si$).
Spectrométrie de masse FAB>0 NOBA m/z = 677 [M+H]+, 619 [M+H-tert-butane]+. 303 [dmTr]+

**1-[2'-O-*tert*-butyldiméthylsilyl-4'-thio-5'-O-diméthoxytrityl-β-D-ribofurannosyl] N4-benzoylCytosine 24 c.**

Rendement 32%.
RMN $^1H$ (DMSO$d_6$, 250 MHz); δ 11.31, (s, 1H, NH); 8.51, (d, 1H, $H_6$, $J_{6,5}$ = 7.3); 8.01, (d, 2H, Ho du groupement benzoyle); 7.51, (m, 14H, H aromatiques du groupement DmTr et $H_5$); 6.93,( d, 3H, Hm,p du groupement benzoyle); 5.93,(d, 1H, $H_{1'}$, $J_{1',2'}$ = 3.0); 5.20,(d, 1H, $OH_{3'}$, $J_{OH,H3'}$ = 5.0); 4.14,(m, 1H, $H_{2'}$); 4,00,(m, 1H, $H_{3'}$); 3.76,(s, 6H, $OCH_3$ du groupement DmTr); 3.38,(m, 3H, $H_{4'}$, $H_{5'}$et $H_{5''}$); 0.86, (s, 9H, *tert*-butyl); 0.60, (d, 6H, $Me_2Si$).
Spectrométrie de masse FAB>0 NOBA m/z 780 $[M+H]^+$, 303 $[DmTr]^+$

**1-[2'-O-*tert*-butyldiméthylsilyl-4'-thio-5'-O-diméthoxytrityl-β-D-ribofurannosyl] N6-benzoylAdénine 24 d.**

Rendement 35 %
RMN $^1H$ (DMSO$d_6$, 250 MHz); δ 11.25,( s, 1H, NH); 8.64, (s, 1H, $H_8$); 8.61, (s, 1H, $H_2$); 8.04, (d, 2H, Ho du groupement benzoyle); 7.51, (m, 13H, H aromatiques du groupement DmTr); 6.95, (d, 3H, Hm,p du groupement benzoyle); 6.01, (d, 1H, $H_{1'}$, $J_{1',2'}$ = 5.8); 5.48, (d, 1H, $OH_{3'}$, $J_{OH,3'}$ = 2.5); 4.70, (m, 1H, $H_{2'}$); 4.26, (m, 1H, $H_{3'}$); 3.75,( s, 6H, $OCH_3$ du groupement DmTr); 3.53, (m, 3H, $H_{4'}$,$H_{5'}$ et $H_{5''}$), 0.82, (s, 9H, *tert*-butyl); 0.50, (d, 6H, $Me_2Si$).
Spectrométrie de masse FAB>0 NOBA m/z 804 $[M+H]^+$, 303 $[M+H]^+$

**1-[3'-O-*tert*-butyldiméthylsilyl-4'-thio-5'-O-diméthoxytrityl β-D-ribofurannosyl] Thymine 25 a.**

Rendement 13%.
RMN $^1H$ (DMSO$d_6$, 360 MHz); δ 11.34, (s, 1H; NH); 7.46, (s, 1H, $H_6$); 7.89-7.30, (m. 13H, H aromatiques); 5.87,(d, 1H, $H_{1'}$, $J_{1',2'}$ = 7.8); 5.46, (d, 1H, $OH_{2'}$, $J_{OH,2'}$ = 5.1); 4.13, (m, 1H, $H_{3'}$); 4.11,(m, 1H, $H_{2'}$, $J_{2',1'}$ = 7.8, $J_{OH,2'}$= 5.1); 3.73,

(s, 6H, OMe), 1.66, (s, 3H, $CH_3$); 0.84,(s, 9H, *tert*-butyl); 0.50,(d, 6H,$Me_2Si$).

**1-[3'-O-*tert*-butyldiméthylsilyl-4'-thio-5'-O-diméthoxytrityl β-D-ribofurannosyl] Uracile 25 b**.

Rendement 27%
Tf= 109°C.
RMN $^1H$ ($DMSOd_6$, 360 MHz); δ 11.30,(s, 1 H, NH); 7.70, (d, 1 H, $H_6$, $J_{6,5}$ = 8.0); 7.30-6.89, (13 H, H aromatiques du groupe-ment dmTr); 5.84, (d, 1 H, $H_{1'}$, $J_{1',2'}$ = 7.3); 5.57, (d, 1 H, $H_5$, $J_{5,6}$ = 8.0); 5.46, (d, 1 H, $OH_{2'}$, $J_{OH2',2'}$ = 5.1); 4.12, (t, 1 H, $H_{3'}$ $J_{3',2'}$ = 3.1, $J_{3',4'}$ = 3,1); 4.03, (m, 1 H, $H_{2'}$); 3.74, (s, 6 H, OMe); 3.41, (m, 1 H, $H_{4'}$); 3.21, (m, 2 H, $H_{5'}$, $H_{5''}$); 0.08, (s, 9 H, *tert*-butyl); 0.02, (m, 6 H, $Me_2Si$).
Spectrométrie de masse FAB>0 NOBA m/z 677 [M+H]+. 619 [M+H-tert-butane]+, 303 [dmTr]+.
Ce composé a été mis en présence avec une solution éthanol/triéthylamine 0.25% (v/v) pendant une 12 h à température ambiante pour donner en proportion égale un mélange **24 b** et **25 b**.

**1-[3'-O-*tert*-butyldiméthylsilyl-4'-thio-5'-O-diméthoxytrityl β-D-ribofurannosyl] N4-benzoyl Cytosine 25 c.**

Rendement 31 %.
RMN $^1H$ ($DMSOd_6$, 250 MHz); δ 11.33, (s, 1H, NH); 8.40, (d, 1H, $H_6$, $J_{6,5}$ = 7.4); 8.01, (d, 2H, Ho du groupement benzoyle); 7.60, (m, 14H, H aromatiques du groupement DmTr et $H_5$); 6.92, ( d, 3H, Hm,p du groupement benzoyle); 5.92,(d, 1H, $H_{1'}$, $J_{1',2'}$ = 5.3); 5.60,(d, 1H, $OH_{2'}$, $J_{OH,2'}$ = 5.0); 4.15,(m, 2H, $H_{2'}$, $H_{3'}$); 3.75,(s, 6H, $OCH_3$ du groupement DmTr); 3.38,(m, 3H, $H_{4'}$, $H_{5'}$et $H_{5''}$); 0.79, (s, 9H, *tert*-butyl); 0.00, (d, 6H, $Me_2Si$). Spectrométrie de masse FAB>0 NOBA m/z 780 $[M+H]^+$, 303 $[DmTr]^+$

**1-[3'-O-*tert*-butyldiméthylsilyl-4'-thio-5'-O-diméthoxytrityl β-D-ribofurannosyl] N6-benzoyl Adénine 25 d.**

Rendement 29%.
RMN $^1H$ ($DMSOd_6$, 250 MHz); δ 11.25,( s, 1H, NH); 8.64, (s, 1H, $H_8$); 8.61, (s, 1H, $H_2$); 8.04, (d, 2H, Ho du groupement benzoyle); 7.51, (m, 13H, H aromatiques du groupement DmTr); 6.95, (d, 3H, Hm,p du groupement benzoyle); 6.01, (d, 1H, $H_{1'}$, $J_{1',2'}$ = 5.8); 5.79, (d, 1H, $OH_{2'}$, $J_{OH,2'}$ = 3.5); 4.70, (m, 1H, $H_{2'}$); 4.26, (m, 1H, $H_{3'}$); 3.75,( s, 6H, $OCH_3$ du groupement DmTr); 3.53, (m, 3H, $H_{4'}$,$H_{5'}$ et $H_{5''}$); 0.82, (s, 9H, *tert*-butyl); 0.01, (d, 6H, $Me_2Si$).
Spectrométrie de masse FAB>0 NOBA m/z 804 $[M+H]^+$, 303 $[M+H]^+$

**Méthode générale de préparation des β-D-ribonucléoside phosphoramidites 26 a-d.**

A une solution des composés **24 a-d** (1 mmole) dans du dichlorométhane anhydre (3.8ml) sont ajoutés sous atmosphère inerte d'argon la N,N,N-diisopropyléthylamine (4 mmoles, 0.7ml), la chloro N,N-diisopropylaminométhoxyphosphine (2.5mmole), 0.48ml) et la 4-N,N-diméthylaminopyridine (0.2mmole, 24.4mg). Le mélange réactionnel est agité de 40 à 90 minutes à température ambiante puis est dilué avec de l'acétate d'éthyle (35ml). La solution résultante est lavée avec de la saumure (4x50ml) puis avec de l'eau (2x50ml). La phase organique est séchée sur sulfate de sodium, filtrée et évaporée sous pression réduite. Le résidu est chromatographié sur colonne de gel de silice et l'élution est réalisée à l'aide d'un mélange de cyclohexane, de dichlorométhane et de triéthylamine (100/0/0.1 à 50/50/0.1). Les produits **26 a-d** sont obtenus sous la forme d'une poudre blanche après lyophilisation dans le benzène.

**1-[2'-O-*tert*-butyldiméthylsilyl-3'-N,N-diisopropylméthoxyphosphoramidite-4'thio-5'-O-diméthoxytrityle-β-D-ribofurannosyl]Thymine 26 a** (mélange diastéréoisomérique)

Rendement: 89%.
RMN $^{31}P$ ($CD_3CN$); δ 151,32 et 150,03.
RMN $^1H$ : δ 7.99, (s, 1 H, NH), 7.65, (d, 1 H, $H_6$); 7,35-6.80, (m, 13 H, H aromatiques ); 5.97, (m, 1 H, $H_{1'}$); 4,25, (m, 1 H, $H_{2'}$), 4.13, (m, 1 H, $H_{3'}$); 3.78, (s, 6 H, OMe); 3.68, (m, 1 H, $H_{4'}$); 3.54, (m, 2 H, $H_{5'}$, $H_{5''}$); 3.35 (m, 3 H, MeO du groupement phosphoramidite); 3.25, (m, 2 H, CH des groupements isopropyle); 1,60, (m, 3 H, $CH_3$); 1.15, (m, 12 H, $CH_3$ des groupements isopropyle); 0.86, (s, 9 H, *tert*-butyl); 0.05, (m, 6 H, $Me_2Si$).Spectromètrie de masse FAB>0 NOBA m/z = 852 $[M-H]^+$

**1-[2'-O-*tert*-butyldiméthylsilyl-3'-N,N-diisopropylméthoxyphosphoroamidite-4'thio-5'-O-diméthoxytrityle-β-D-ribofurannosyl] Uracile 26 b** (mélange diastéréoisomérique)

Rendement 78%.

RMN $^{31}P$ ($CDCl_3$); δ 150.65 et 150.50.
RMN $^{1}H$ ($CD_3CN$,250 MHz); δ 7.99, (s, 1 H, NH), 7.22, (m, 1 H, $H_6$); 7.30-6.89, (m, 13 H, H aromatiques du groupement DmTr); 5.90, (m, 1 H, $H_{1'}$); 5,50, (m, 1 H, $H_5$); 4,13, (m, 2 H, $H_{2'}$, $H_{3'}$); 3.80, (s, 6 H, OMe du groupe DmTr); 3.58, (m, 3 H, $H_{4'}$, $H_{5'}$, $H_{5''}$); 3.42, (m, 2 H, 2 CH des groupements isopropyle); 3.30 (m, 3 H, MeO du groupement DmTr); 1.19, (m, 12 H, $CH_3$ des groupements isopropyle); 0.84, (s, 9 H, *tert*-butyl); 0.05, (m, 6 H, $Me_2Si$).
Spectrométrie de masse FAB>0 NOBA m/z = 534 [M+H-DmTrH]$^+$

**1-[2'-O-*tert*-butyldiméthylsilyl-3'-N,N-diisopropylméthoxyphosphoroamidite-4'thio-5'-O-diméthoxytrityle-β-D-ribofurannosyl] N4-benzoyl Cytosine 26 c.**

Rendement 75%.
RMN $^{31}P$ ($CD_3CN$); δ 151,30 et 150,05
Spectrométrie de masse FAB>O PEG m/z 941 [M+H]$^+$, 637 [M+H-DTrH], 303 [DmTr]$^+$

**1-[2'-O-*tert*-butyldiméthylsilyl-3'-N,N-diisopropylméthoxyphosphoroamidite-4'thio-5'-O-diméthoxytrityle-β-D-ribofurannosyl] N6-benzoyl Adénine 26 d.**

Rendement 71% .
RMN $^{31}P$ ($CDCl_3$); δ 151,26 et 150,01
Spectrométrie de masse FAB>O PEG m/z 964 [M+H]$^+$, 600 [M+H-DmTrH], 303 [DmTr]$^+$.

**II. 3 Fonctionalisation du support solide (Schéma 10)**.

Le support solide ou LCA-CPG ( Long Chain Alkylamine Controlled Pore Glass) **P-1** a tout d'abord été activé par une solution d'acide trichloroacétique à 3 % dans le dichlorométhane à température ambiante pendant 2 à 3 heures afin de libérer le plus grand nombre de groupements amino, ce qui conduit au maximum de sites réactifs sur la surface des billes de verre. Le support ainsi activé **P-2** est alors fonctionnalisé par couplage avec de l'anhydride succinique dans la pyridine en présence de 4-DMAP ce qui conduit à **P-3**. Dans l'étape suivante, le nucléoside 3'-silylé **25** est utilisé puisqu' il a été obtenu pendant la réaction de silylation des thioribonucléosides et n'est pas nécessaire pour la synthèse d'oligothionucléotides contenant la liaison phosphodiester 3'-5'. Ainsi, **P-3** activé par le 1-(3-diméthylaminopropyl) 3-éthylcarbodiimide (DEC) en présence de 4-DMAP est mis en réaction dans la pyridine anhydre avec le 5'-O-DmTr-3'-OTBDMS β-D-thioribonucléoside **25**. Les sites non utilisés du support sont masqués à l'aide de pipéridine. Une dernière étape de masquage à l'anhydride acétique en présence de collidine dans le THF conduit à **P-6**. La quantité de nucléoside fixée sur le support est déterminée par spectrophotométrie U. V. en mesurant la quantité de cation diméthoxytrityle libéré après un traitement par une solution à 10 % d'acide trichloroacétique dans le dichlorométhane. Le degré de fonctionnalisation varie de 21 à 38 μmole par gramme de support suivant la nature de la base nucléosidique.

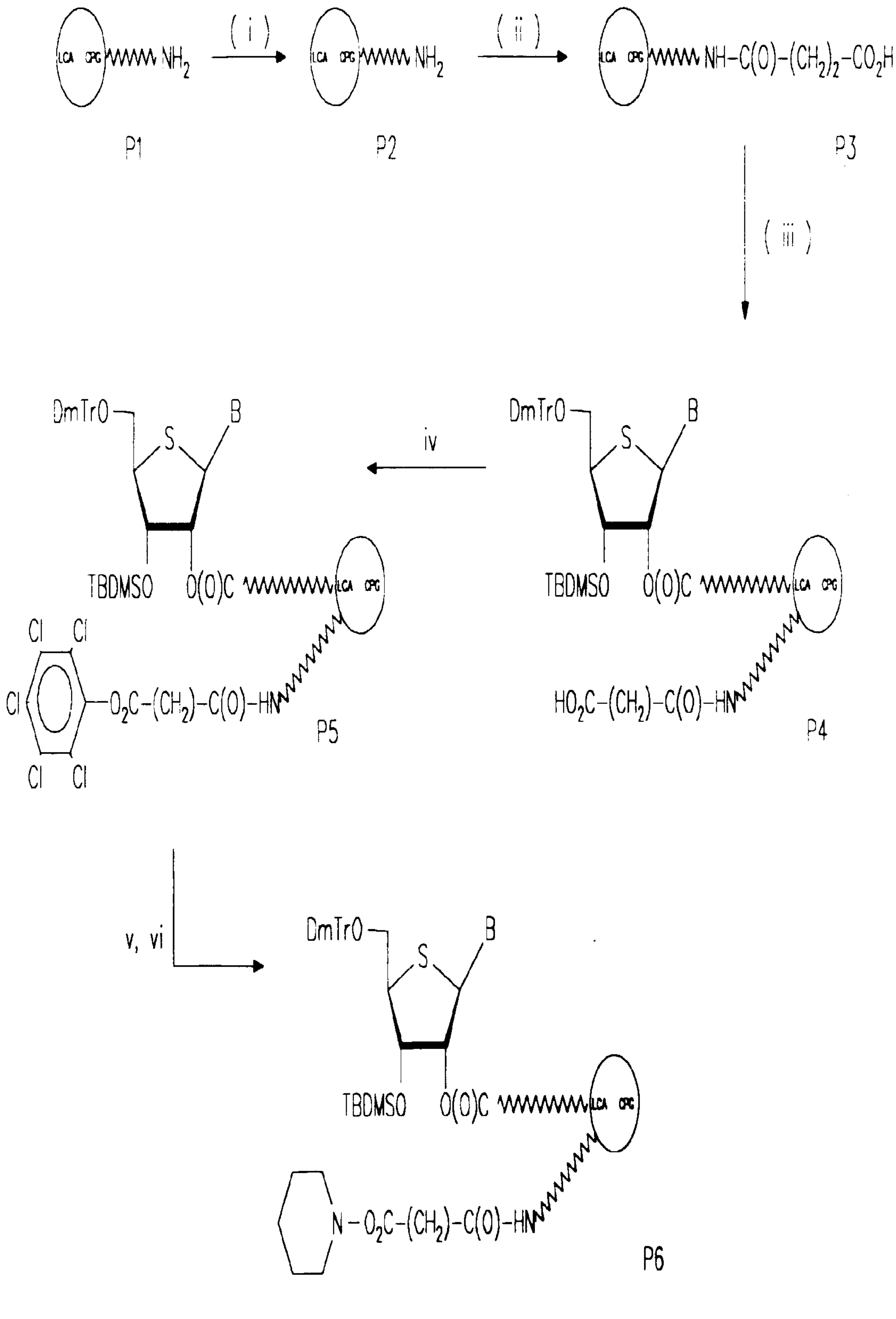


( i = TCA3%, $CH_2Cl_2$; ii = anhydride succinique, DMAP, Pyr.; iii = 5'-o-DmTr-4'-thio-3'-o-TBDMS-ß-D-ribonucléoside, DMAP, $NEt_3$, DEC Pyr.; iv = pentachlorophénol; v = pipéridine; vi = $Ac_2O$ 0.5M dans THF )

SCHEMA 10

**II. 4. Synthèse automatisée des thiooligonucléotides (Schéma 11).**

Les thiooligonucléotides ont été synthétisés sur un synthétiseur à ADN (Applied Biosystems modèle 381 A). Le cycle d'élongation comporte quatre étapes importantes :

Détritylation du nucléoside ou de l'oligonucléotide fixé sur le support solide.

Condensation du phosphoramidite activé par le tétrazole sur l'hydroxyle 5' libre du nucléoside ou de l'oligonucléotide.

Masquage des fonctions 5'-hydroxyles qui n'ont pas réagi.

Oxydation des fonctions phosphitetriester en phosphotriester.

SCHEMA 11 - DEPROTECTION ET DECROCHAGE

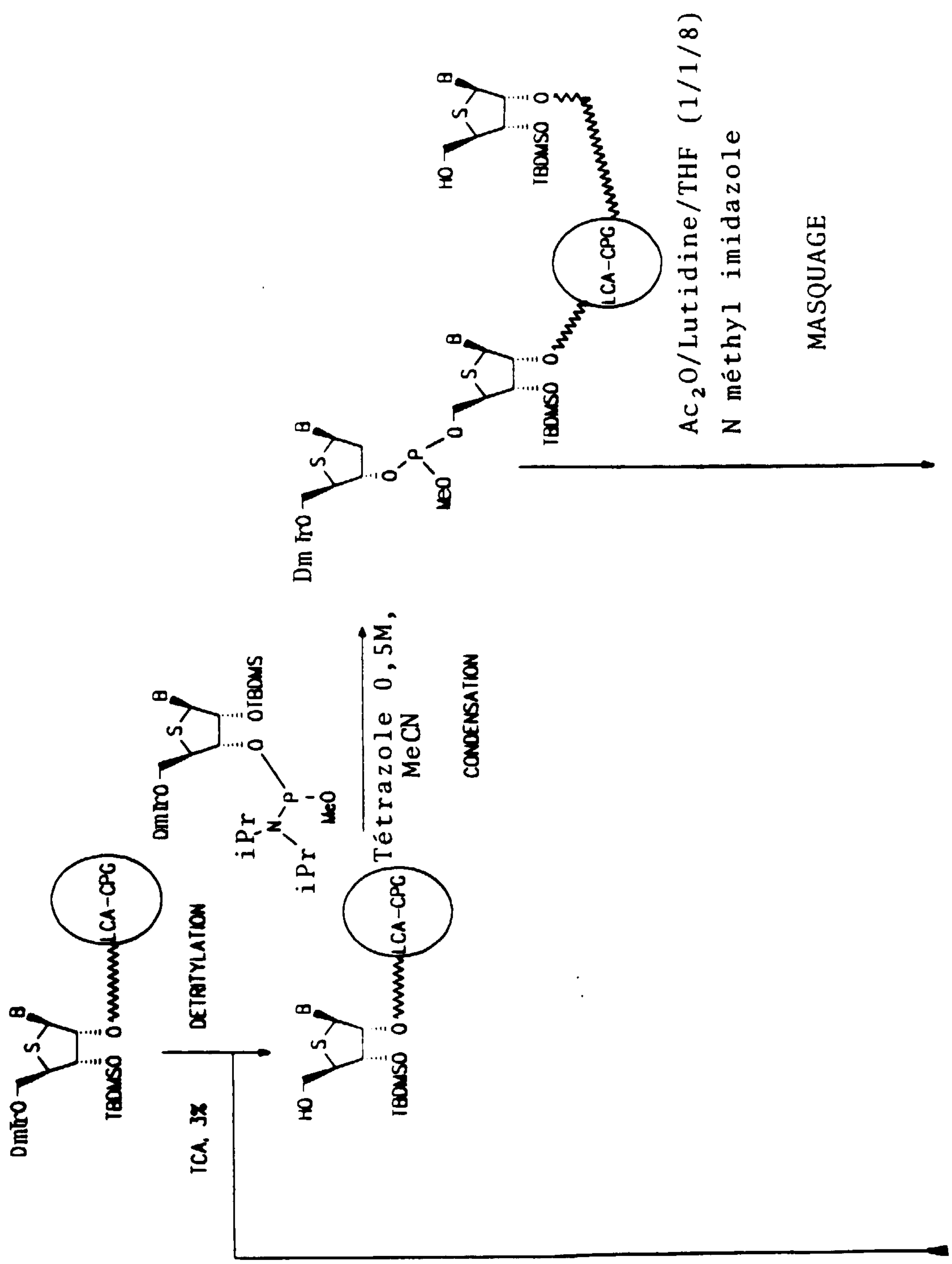

SCHEMA 11 (suite)

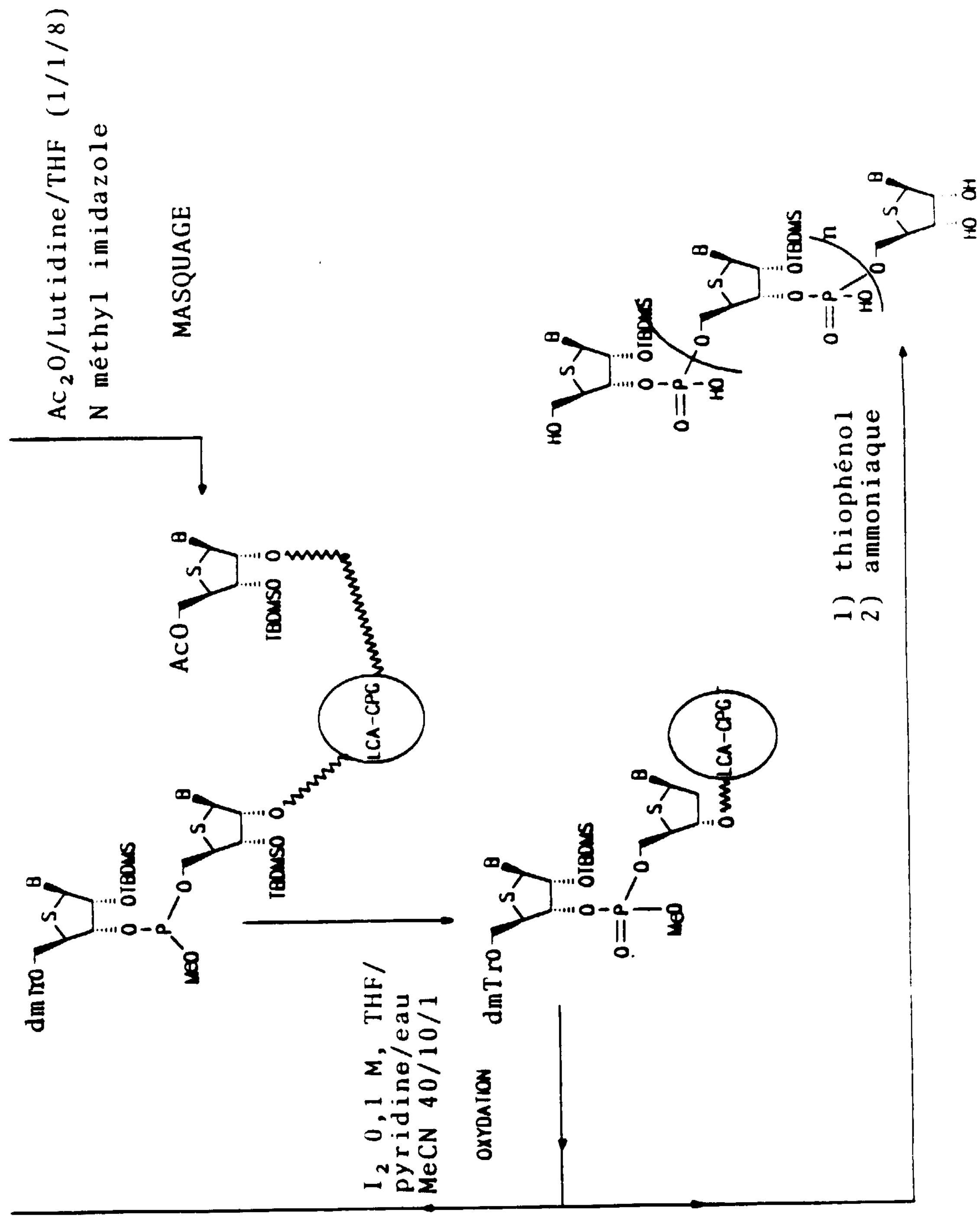


Chaque synthèse a été effectuée sur une colonne contenant 40 mg de support solide. Le rendement de chaque incorporation est de 98 % évalué en mesurant la concentration des cations diméthoxytrityles récupérés après chaque couplage par traitement du support à l'acide trichloroacétique.

Des lavages sont effectués entre chaque étape, et la durée d'un cycle d'élongation est de 21,1 minutes pour la synthèse de l'homododécamère 4'-thio-β-D-ribonucléotide (β**rSU$_{12}$**) ( Tableau 1).

TABLEAU 1:

| Cycle d'élongation utilisé pour la synthèse du β**rSU$_{12}$**. | | | |
|---|---|---|---|
| Numéro | Etape | Temps (sec.) | Solvant et Réactifs |
| 1 | Séchage et Lavage | 24 | MeCN-Argon |
| 2 | Détritylation | 80 | TCA à 3% dans $CH_2Cl_2$ |
| 3 | Séchage et Lavage | 74 | MeCN-Argon |
| 4 | Couplage | 915 | amidite 0.1M(24eq.)-MeCN+tétrazole 0.5M(10eq.)+MeCN |
| 5 | Séchage | 45 | Argon |
| 6 | Masquage | 23 | Méthylimidazole-THF $Ac_2O$/lutidine/THF: 1 1 8 |
| 7 | Séchage | 11 | Argon |
| 8 | Oxydation | 43 | $I_2$ 0.1M dans THF Pyr H2O 40/10/1 |
| 9 | Lavage | 53 | MeCN |

**II. 5. Décrochage, Déprotection et Purification du thiooligomère.**

Après avoir réalisé les étapes de déprotection des méthoxyphosphate diesters au thiophénol, le décrochage du dodécamère du support solide par l'ammoniaque, la déprotection des hydroxyles par le TBAF, le thiooligomère a été dessalé sur une colonne d'exclusion G-25 DEAE Sephadex où sur une colonne ionique A-25 Sephadex DEAE et purifié par CLHP en phase inverse.

II.6.. PARTIE EXPERIMENTALE.

**Exemple I : Synthèse de l'homododécamère βrsU$_{12}$.**

I-1 Synthèse du support solide:

Le support solide **P-1** (2,415 g) est mis en suspension avec une solution (50 ml) d'acide trichloroacétique à 3 % dans $CH_2Cl_2$ anhydre. Après 4 h 15 d'agitation, 50 ml d'une solution de triethylamine et de diisopropylamine (9:1) est ajoutée et après 5 mn d'agitation la solution est filtrée. Le support solide **P-2** ainsi obtenu est lavé par du dichlorométhane (2 x 50 ml) puis par de l'éther (50 ml) et mis à sécher.

Le support solide **P-2** est ensuite mis en suspension dans de la pyridine anhydre (14,5 ml) en présence d'anhydride succinique (0,48 g) et de DMAP (0,0805 g). La suspension est agitée pendant 20 heures puis filtrée. Le support solide **P-3** est alors lavé avec de la pyridine anhydre (50 ml), du dichlorométhane (2 x 50 ml) et de l'éther (2 x 50 ml) puis mis a sécher sous vide.

**Condensation du support solide P-3 avec le 1-[3'-O-*tert*-butyldiméthylsilyl-2'-hydroxy-4'thio-5'-O-diméthoxytrityle-β-D-ribofurannosyl]Uracile 25 b**

Un mélange de **P-3** (2,415 g) de 1-[3'-O-*tert*-butyldiméthylsilyl-2'-hydroxy-4'thio-5'-O-diméthoxytrityle-β-D-ribofurannosyl]Uracile **25 b** (333 mg, 1 éq.) de la DMAP (29,5 mmg, 0,5 éq.) de la triethylamine (0,146 mg, 202 µl, 0,003 éq.) et du DEC (920 mg, 10 eq.) dans 29 ml de pyridine anhydre est agité 3 jours à température ambiante.

On ajoute ensuite du pentachlorophénol (321 mg, 2,5 éq.) et on laisse sous agitation 24 heures supplémentaires. Le support solide est alors filtré, rincé avec de la pyridine anhydre (2 x 50 ml) puis avec du dichlorométhane (2 x 50 ml) et de l'éther (2 x 50 ml).

Immédiatement après, on traite le support solide **P-5** par 25 ml de pipéridine. Après agitation à température ambiante pendant 24 heures, le support solide est filtré, lavé par du dvichlorométhane (2 x 50 ml) puis de l'éther (2 x50 ml) et mis à sécher sous vide.

Le support solide sec (2,415 g) est mis en présence d'une solution d'anhydride acétique (0,5 M) dans le THF (15 ml) et d'une solution de collidine (0,5 M) dans le THF (15 ml). Après 4 heures de réaction, le support solide est lavé avec de la pyridine anhydre (2 x 50 ml), du dichlorométhane (2 x 50 ml), du THF (2 x 50 ml) et de l'éther (2 x 50 ml). **P-6** ainsi obtenu est mis à sécher sous vide.

I-2) Détermination du taux de fonctionnalisation du support solide **P-6**.

Cette détermination s'effectue par dosage des groupements DmTr libérés en milieu acide. On pèse exactement 38.0 mg de **P-6** qui sont mis en suspension dans 3,4 ml d'une solution d'acide **para**-toluène sulfonique (0,1 M) dans l'acétonitrile. Le mélange est agité 15 mn puis sonique pendant 2 mn.

On ajuste alors le volume à 10 ml avec la solution 0,1 M d'acide **para**-toluène sulfonique dans l'acétonitrile. On prélève 0.3 ml de cette solution à laquelle on ajoute 5 ml de la solution 0,1 M d'acide *para*-toluène sulfonique. La lecture de la DO (0.317 unité de DO) à 500 nm nous permet de calculer le degré de fonctionnalisation du support **P-6** qui est dans ce cas de 21 μmole/gramme de support solide.

I-3) Synthèse automatisée de l'homododécamère β**rSU$_{12}$**.

La synthèse est effectuée sur une colonne contenant 39,8 mg de support solide fonctionna lisé à 21 μmole/g de résine, soit 0,835 μmole (1 éq) de 4'-thiouridine.

Chaque cycle d'élongation nécessite un excès de synthon phosphoramidite (20 μmole, 24 éq) soit 240 μmole au total pour la synthèse du dodécamère. Le synthétiseur prelevant 200μl d'une solution 0,1 M de synthon dans l'acétonitrile par incorporation.

La durée d'un cycle d'élongation est de 21,1 mn (Tableau 1).

L'étape de couplage a été considérablement augmentée (900 s contre 45 s dans le cas d'un désoxynucléoside) à cause de la moindre réactivité du synthon ribonucléotide.

Le dosage des cations diméthoxytrityles libérés à chaque étape de détritylation a permis d'évaluer le rendement moyen d'incorporation de l'unité thioribonucléotidique à 98,7 %.

I-4) Décrochage, déprotection et purification du thio homododécamère β**rSU$_{12}$**.

Le support solide portant le thio-homododécamère β**rSU$_{12}$** est traité par 5 ml d'une solution de thiophénol, triéthylamine, dioxane 1, 2 (1/2/2) pendant une demi-heure à température ambiante.

La solution de thiophénol est alors filtrée et le support lavé par une solution d'ammoniaque 32 % dans l'ethanol 95 (3/1: v/v) (3 x 500μl) pour décrocher le thio-homododecamere du support solide. La solution ainsi obtenue est évaporée, reprise par 500 μl d'eau puis lyophilisée.

L'oligomère est alors dissout dans 300 μl d'une solution de TBAF (1,1 M) dans le THF. Le mélange réactionnel est laissé sous agitation pendant 24 heures. La réaction est alors stoppée avec 300 μl d'une solution aqueuse d'acétate d'ammonium (0,05 M).

La solution est évaporée à sec, coévaporée 3 fois avec de l'eau et le résidu chromatographié sur colonne de gel d'exclusion Sephadex G-25. Les fractions contenant le thiooligomère sont rassemblées, évaporées à sec et analysées par CLPH.

Analyse et Purification du thio-oligomère β**rSU$_{12}$** obtenu.

L'analyse CLPH du thiooligomère a été effectuée sur une colonne Beckman C-18 RP 3 μ x LODS Ultrasphère dans les conditions de gradient suivantes:

A: 10 % d'acétonitrile dans une solution aqueuse 0,05 M d'acétate de triéthylammonium.
B: 15 % d'acétonitrile dans une solution aqueuse 0,05 M d'acétate de triéthylammonium.

Gradient:

A -----------B---------B---------A
20'    10'    5'

Temps d'analyse: 30'.

Dans ces conditions, on constate que le thiooligomère est pur à 92,73 %.

On effectue alors une purification de β**rSU$_{12}$** sur colonne semi-préparative Nucléosyl avec un gradient:

A--------------B----------B-----------A
15'    10'    5'

On obtient dans ces conditions le β**rSU$_{12}$** avec une pureté de 94,42 % suffisante pour les études ultérieures.

**Exemple II: Synthèse du dodècamère β ( SrT)$_1$ dT$_{11}$**.

II-1) Synthèse du support solide.

Le support solide fonctionnalisé à 1 μmole pour 35 mg de résine de 2'-désoxythymidine ainsi que les synthons 1-[ 5'-O-DmTr-3'-O-N,N-diisopropylamino cyanoéthyl phosphine, 2'-désoxy-β-D-ribofurannosyl thymine sont commercialisés par Applied Biosystems.

II-2) Synthèse automatisée de β **( SrT)$_1$ dT$_{11}$**.

La synthèse est effectuée sur une colonne contenant 35 mg de support solide soit 1 μmole de 2'-désoxythymidine. Chaque cycle d'élongation nécessite un excès de synthons phosphoramidite (20 μmoles,20 éq) soit 220 μmole de synthons 2'-désoxythymidine et 20 μmoles de synthon 4'-thio-thymidine 5. Le synthétiseur prélevant 200 μmoles par incorporation d'une solution 0,1 M de chaque synthon dans l'acétoninile. La duree d'un cycle d'incorporation d'une unité désoxythymidine est de 5,5 mn .Alors qu'elle est de 20,1 mn pour un cycle d'incorporation du synthon 4'-thiothymidine.

L'augmentation de la durée du cycle d'incorporation du synthon chimère est due à l'étape de couplage de 909' contre 36' pour le synthon 2'-désoxythymidine.

Le dosage des cations diméthoxytrityles libérés à chaque étape de détritylation a permis d'évaluer le rendement moyen d'incorporation de l'unité 2'-désoxythymidine à 98,5 % et 93,5 % pour le synthon 4'-thiothymidine.

II-3) Décrochage,déprotection et purification du β **(SrT)$_1$ dT$_{11}$**

Le support solide portant l'oligomére β **(SrT)$_1$ dT$_{11}$** est traité par une solution d'ammoniaque (32 %) dans l'ethanol 95 (3/1, v/v) (3 x 500 μl) pendant 3 cycles de 20 mn chacun afin de décrocher l'oligomère de son support, puis le dodécamère est incubé pendant 2 hr dans une étuve sèche dans le même mélange afin de déprotéger les fonctions cyanoéthyl et méthoxy.

L'oligomére est ensuite évaporé sous vide, repris par 500 μl d'eau et lyophilisé.

La déprotection des groupements TBDMS est effectuée selon le protocole mis au point dans l'exemple I.

Le résidu obtenu est chromatographié sur colonne de gel de DEAE Sephadex A-25. Les fractions contenant l'oligomére sont rassemblées, évaporées à sec et analysées par CLPH.

Analyse et Purification du β **(SrT)$_1$ dT$_{11}$** par CLPH.

L'analyse CLPH de l'oligomère a été effectuée sur une colonne Beckman C 18 RP 3 μ Ultrasphère dans les conditions de gradient suivantes :

A: 6 % d'acétonitrile dans un tampon acétate de triéthyl ammonium 0,05 M.

B : 20 % d'acétonitrile dans un tampon acétate de triéthyl ammonium 0,05 M.

A-------------------B----------------B

15'                    10'

Temps d'analyse : 25 mn.

Dans ces conditions, l'oligomère est pur à 49,3 %.

une purification de β **( SrT)$_1$ dT$_{11}$** est alors éffectuée par CLPH semi-préparative avec une colonne Nucleosyl dans les conditions de gradient suivantes:

A----------------B---------------B

15'                5'

Le 12-mer présente alors un temps de rétention de 12,99 mn et une pureté supeneure a 99 %.

**II.4.: Synthèse du dodécamère β $dT_5$ (SrT) $dT_6$**

II-4.1) Synthèse du support solide.

Le support solide défini dans l'exemple II a été utilisé.

II-4.2) Synthèse automatisée de β **$dT_5$ (SrT) $dT_6$**.

Les mêmes conditions de synthèse mises au point pour la synthèse de β **$(SrT)_1$ $dT_{11}$** ont été utilisées.

Le dosage des cations diméthoxytrityles libérés à chaque étape de détritylation a permis d'évaluer le rendement moyen d'incorporation de l'unité 2'-désoxythymidine à 99,3 % et à 96,1 % pour le synthon 4'-thiothymidine.

II-4.3) Décrochage et purification du β **$dT_5$ (SrT) $dT_6$.**

Le traitement de cet oligomère a été effectué selon le protocole mis au point dans l'exemple II. L'analyse et la purification CLHP, réalisée dans les mêmes conditions de gradient révèle un temps de rétention de 13,18 minutes et une pureté spectrophotométrique a 260nm de 100 %.

III. SYNTHESE AUTOMATISEE DE 4'-THIO-OLIGORIBONUCLEOTIDES ET OLIGOMERES MIXTES.

On décrit la synthèse d'un 4'-thio-ribo oligomère homogène 1 dirigé sur la séquence d'épissage acceptrice du gène *tat* du HIV. On montre qu'il est ensuite possible d'obtenir des oligomères mixtes comprenant une séquence limitée d'ADN présentant des liaisons phosphodiesters (séquence 4) ou phosphorothioates (séquence 5).

De telles séquences mixtes 5'-(4'-S-ARN / ADN / 4'-S-ARN)-3' 4 et 5 peuvent être utilisées dans une approche antisens et sont susceptibles d'être hautement sélectives dans la mesure où la "fenêtre" de structure ADN sera seule substrat de la RNAse H après appariement de l'antisens avec une cible complémentaire. Il est à nota que la "fenêtre" ADN (phosphodiester ou phosphorothioate) peut être de longueur variable - dans les séquences 4 et 5 il y a six désoxynucléotides - et que cette "fenêtre" peut être incluse à n'importe quelle position de l'oligomère, même aux extrémités 5' et / ou 3'.

Complémentairement, cette possibilité ouvre la voie à la conception de ribozymnes artificiels (homogènes ou non) comportant des séquences d'oligo-ribonucléotides 4'-S-ARN

On a synthétisé un certain nombre de 4'-thio-oligoribonucléotides homogénes (4'-S-ARN) sur un synthétiseur à ADN (Applied Biosystems modèle 381A) en utilisant le procédé général décrit précédemment.

C'est le cas notamment: du

**4'-Sr-(A-C-A-C-C-C-A-A-U-U-C-U) 1**

du

**4'-Sr-(U-U-U-U-U-U). (4'-$SrU_6$). 2**

et du

**4'-Sr-(U-U-U-U-U-U-3'-P-$CH_2$-$CH_2$-$CH_2$-OH). (4'-$SrU_6$-3'n-prOH) 3.**

L'expression 4'-Sr veut dire : oligoribonucléotide de configuration β où l'oxygène du cycle furannose des sucres est remplacé par un atome de soufre.

3'n-prOH signifie qu'une fonction n-propanol est fixée sur le groupement phosphate introduit à l'extrémité 3' de l'oligoribonucléotide.

De plus, des oligomères mixtes comprenant des séquences d'oligodesoxynucleotides phosphodiester ou phosphorothioates ont été obtenus et les modifications apportées au cycle d'élongation sont décrites en prenant comme exemple les séquences suivantes :

**4'-Sr-(U-U-U)-d(T-T-T-T-T-T)-4'-Sr-(U-U-U) / $P_{11}$,**

$$[4'\text{-}SrU_3\text{-}dT_6\text{-}4'\text{-}SrU_3\ /\ P_{11}]\ \underline{4}$$

et

$$4'\text{-}Sr\text{-}(U\text{-}U\text{-}U)\text{-}d(T\text{-}T\text{-}T\text{-}T\text{-}T\text{-}T)\text{-}4'\text{-}Sr\text{-}(U\text{-}U\text{-}U)/P_3PS_5P_3,$$

$$[4'\text{-}SrU_3\text{-}dT_6\text{-}4'\text{-}\ SrU_3/P_3PS_5P_3]\ \underline{5}$$

ou 4'-Sr a déjà été défini comme précédemment, $P_x$ correspond au nombre de liaisons phosphodiester entre les unités nucléotidiques et $PS_y$ au nombre de liaisons phosphorothioate entre les unités nucléotidiques.

Les thio-oligoribonucléotides ont été synthétisés sur un synthétiseur à ADN (Applied Biosystems modèle 381 A).

*III-1) Synthèse des 4'-thiooligoribonucléotides: 4'-Sr-(A-C-A-C-C-C-A-A-U-U-C-U)*1. *4'-SrU*$_6$, **2**, *et 4'-SrU*$_6$*-3'n-prOH* **3**

Le cycle d' élongation comporte quatre étapes importantes :

a) Détritylation du nucléoside ou de l'oligonucléotide fixé sur le support solide dans le cas de **4'-SrU$_6$** et de **4'-Sr-(A-C-A-C-C-C-A-A-U-U-C-U)**, ou bien détritylation du bras n-propanol fixé sur le support solide ou de l'oligonucléotide fixé sur le lien n-propanol dans le cas de **4'-SrU$_6$-3'n-prOH**.

b) Condensation du phosphoramidite activé par le tétrazole sur l'hydroxyle 5' libre du nucléoside ou de l'oligonucléotide dans le cas de **4'-SrU$_6$** et de **4'-Sr-(A-C-A-C-C-C-A-A-U-U-C-U)** ou bien sur l'hydroxyle libre du bras n-propanol ou sur l'hydroxyle en 5' de l'oligonucléotide dans le cas de **4'-SrU$_6$-3'n-prOH**.

c) Masquage des fonctions 5'-hydroxyles qui n'ont pas réagi.

d) Oxydation des fonctions phosphitetriester en phosphotriester.

Chaque synthèse a été effectuée sur une colonne contenant 40 mg de support solide. Le rendement de chaque incorporation est de 98 % évalué en mesurant la densité optique des cations diméthoxytrityles libérés après chaque couplage par traitement du support à l'acide trichloroacétique.

Des lavages sont effectués entre chaque étape, et la durée d'un cycle d'élongation est de 21,46 minutes pour la synthèse des trois oligomères précités. ( Tableau 2).

TABLEAU 2

| Cycle d'élongation utilisé pour la synthèse de **4'-Sr-(A-C-A-C-C-C-A-A-U-U-C-U) 1, 4'-SrU$_6$ 2, et 4'-SrU$_6$-3'n-prOH, 3.** | | |
|---|---|---|
| **ETAPES** | **TEMPS (secondes)** | **SOLVANTS ET REACTIFS** |
| Lavage-séchage | 25 | $CH_3CN$/ARGON. |
| Couplage | 915 | Nucléoside phosphoramidite 0.1 M (20 eq.) dans $CH_3CN$ + tetrazole 0,5 M (10 eq.) dans $CH_3CN$. |
| Lavage-séchage | 41 | $CH_3CN$/ARGON. |
| Oxydation | 36 | $I_2$ (0,1 M) dans THF/Pyr/$H_2O$ (40/10/1). |
| Lavage-séchage | 59 | $CH_3CN$/ARGON. |
| Masquage | 23 | Méthylimidazole-THF : 1 $Ac_2O$/lutidine/THF : 1/1/8 : 1. |
| Lavage-séchage | 71 | $CH_3CN$/ARGON. |
| Détritylation | 98 | TCA à 3% dans $CH_2Cl_2$. |
| Lavage-séchage | 33 | $CH_3CN$/ARGON. |

***III-2 Synthèse des oligomères mixtes : 4'-SrU$_3$-dT$_6$-4'-SrU$_3$ / P$_{11}$ 4 et 4'-SrU$_3$-dT$_6$-4'-SrU$_3$ / P$_3$PS$_5$P$_3$.***

*III-2-1 Oligonucléotide 4'-SrU$_3$-dT$_6$-4'-SrU$_3$ P$_{11}$**4** .*

La synthèse automatisée de cet oligonucléotide reprend les quatre étapes décrites au II-1 Chaque synthèse a été effectuée sur une colonne contenant 40 mg de support solide. Le rendement de chaque incorporation, est de 98 %. La durée d'un cycle d'élongation par monomère 4'-Sr-U est de 21,43 minutes contre 6,73 minutes pour un monomère dT. Cette différence est dûe à l'augmentation du temps de couplage d'une entité 4'-Sr-U qui est de 915 secondes contre 36 secondes pour une unité dT (tableau 3).

TABLEAU 3 :

| Cycle d'élongation utilisé pour la synthèse de **4'-SrU$_3$-dT$_6$-4'-SrU$_3$ / P$_{11}$, 4**. | | |
|---|---|---|
| **ETAPES** | **TEMPS (secondes)** | **SOLVANTS ET REACTIFS** |
| Lavage-séchage | 25 | $CH_3CN$/ARGON. |
| Couplage 4'-Sr-U<br>dT | 915<br>36 | Nucléoside phosphoramidite 0,1 M (20 eq.) dans $CH_3CN$ + tetrazole 0,5 M (10 eq.) dans $CH_3CN$. |
| Lavage-séchage | 41 | $CH_3CN$/ARGON. |
| Oxydation | 36 | $I_2$ (0,1 M) dans THF / Pyr / $H_2O$ (40/10/1). |
| Lavage-séchage | 59 | $CH_3CN$/ARGON. |
| Masquage | 23 | Méthylimidazole-THF : 1 $Ac_2O$/lutidine/THF:1/1/8 : 1. |
| Lavage-séchage | 71 | $CH_3CN$/ARGON. |
| Détritylation | 98 | TCA à 3% dans $CH_2Cl_2$. |
| Lavage-séchage | 33 | $CH_3CN$/ARGON. |

*III-2-2 Oligonucléotide 4'-SrU$_3$-dT$_6$-4'-SrU$_3$ P$_3$PS$_5$P$_3$.**5**.*

Les mêmes conditions de synthèse automatisée ont été appliquées à ce dodécamère mixte comportant à la fois des liaisons phosphodiesters et phosphorothioates. La synthèse a été effectuée à l'échelle d'une µmole soit sur une colonne contenant environ 40 mg de support solide fonctionnalisé à 21 µmole / g. Le rendement moyen d'incorporation est de 98 %.

Lors de l'introduction d'une liaison internucléotidique de type phosphorothioate, l'étape d'oxydation définie au paragraphe IV-2-1 (étape 4, Tableau 3 ) a été remplacée par une étape de sulfurisation de 51 secondes utilisant le reactif de Beaucage ("dérivés de sulfone carboxythioanhydride du benzène") dissout dans l'acétonitrile à une concentration de 0.05 M.

Les autres étapes du cycle d'élongation demeurent inchangées.

***III-3 Décrochage, Déprotection et Purification des 4'-thiooligoribonucléotides (4'-S-ARN).***

Après avoir réalisé les étapes de déprotection des méthoxyphosphotriesters en phosphodiesters par traitement au thiophénol, les 4'-thiooligoribonucléotides ont été séparés des supports solides par un traitement à l'ammoniaque. Les groupements TBDMS introduits sur les hydroxyles en 2' ont été déprotégés par une solution de TBAF dans le THF. Les thiooligomères sont ensuite dessalés sur une colonne d'exclusion DEAE Sephadex G-25 puis purifiés par CLHP en phase inverse.

III-4 PARTIE EXPERIMENTALE.

**Exemple I : Synthèse des hexamères : 4'-SrU$_6$ 2 et 4'-SrU$_6$-3'n-prOH, 3.**

*I-1 Synthèse des supports solides:*

Le support solide nécessaire à la synthèse de **4'-SrU$_6$, 2** est le même que celui utilisé pour la synthèse de β**rSU$_{12}$**.

La synthèse de **4'-SrU$_6$-3'n-prOH 3** nécessite l'utilisation du support solide universel:

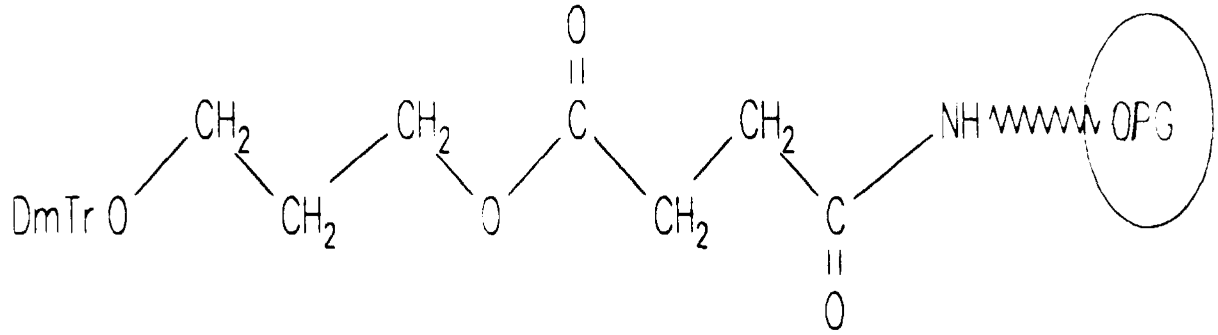

A partir de l'intermédiaire **P-3** décrit dans le Brevet n° 92 01275, un mélange de 1-O-DmTr-propanol-3 (1 éq.), de DMAP (29,5 mg, 0,5 éq.), de triethylamine (0,146 mg, 0,003 éq.) de DEC (920 mg, 10 éq.) et de **P-3** (2,415 g) est agité dans 29 ml de pyridine anhydre pendant trois jours à température ambiante. Du pentachlorophénol (321 mg, 2,5 éq.) est ajouté et l'agitation du mélange réactionnel est poursuivie pendant 24 heures supplémentaires. Le support solide est alors filtré, rincé avec de la pyridine anhydre (2 x 50 ml) puis avec du dichlorométhane (2 x 50 ml) et de l'éther (2 x 50 ml).

Le support solide est ensuite traité par la pipéridine, puis par une solution d'anhydride acétique et de collidine dans le THF selon le protocole défini précédemment.

***I-2) Détermination du taux de fonctionnalisation du support solide.***

Cette détermination a été éffectuée en utilisant le mode opératoire décrit précedemment. Le taux de fonctionnalisation du support solide a été évalué à 16,4 µmole de n-propanol par gramme de résine.

***I-3-) Synthèse automatisée des hexamères 4'-SrU$_6$ 2 et 4'-SrU$_6$-3'n-prOH 3.***

*I-3-1) Synthèse automatisée de l'hexamère 4'-SrU$_6$* **2**.

La synthèse est effectuée sur une colonne contenant 39,4 mg de support solide fonctionnalisé à 21 µmole/g de résine, soit 0,827 µmole (1 éq) de 4'-thiouridine. Chaque cycle d'élongation nécessite un excès de synthon phosphoramidite (16,75 µmole, 20,3 eq) soit 100,5 µmole au total en solution 0,1 M dans l'acétonitrile.La durée du cycle d'élongation définie dans le tableau 1 est de 21,46 minutes.

Le dosage des cations diméthoxytrityles libérés à chaque étape de détritylation a permis d'évaluer le rendement moyen d'incorporation d'une unité thioribonucléotidique à 98,3 %.

*I-3-2) Synthèse automatisée de l'hexamère 4'-SrU$_6$-3'n-prOH* **3**.

La synthèse est effectuée sur une colonne contenant 36,2 mg de support solide fonctionnalisé à 16,4 µmole de n-propanol par gramme de résine soit 0,82 µmole (1 eq.) de n-propanol.

La synthèse totale de **4'-SrU$_6$-3'n-prOH, 3** nécessite 95,9 mg de synthon phosphoramidite (114,8 µmole, 20 eq.) dissout dans 1,14 ml d'acetonitrile.La durée d'un cycle d'élongation est de 21.46 minutes.

Le rendement moyen par couplage est de 98,3 %.

***I-4) Décrochage, déprotection et purification de 4'-SrU$_6$ 2 et 4'-SrU$_6$-3'-n-prOH 3.***

Le traitement utilisant successivement du thiophénol, de l'ammoniaque et une solution de TBAF, commun aux deux hexamères est décrit précedemment.

*I-4-1) Analyse et Purification de l'hexamère 4'-SrU$_6$* **2**.

L'analyse CLPH du thiooligomère a été effectuée sur une colonne SFCC Nucleosyl C-18 N125 2P 789 dans les conditions de gradient suivantes :

A: 10 % d'acétonitrile dans une solution aqueuse 0.05 M d'acétate de triéthylammonium.
B: 15 % d'acétonitrile dans une solution aqueuse 0.05 M d'acétate de méthylammonium

Gradient:

A-------20'------ B-----10'-------- B
> >

Temps d'analyse : 30 minutes, débit : 1 ml / min.

Dans ces conditions, l'hexamère **4'-SrU$_6$, 2** présente une pureté spectrophotométrique à 260 nm de 95,3 %.

Une purification en CLPH préparative sur une colonne nucleosyl semi-préparative avec un débit de 2 ml / min et un éluant isocratique de 12 % d'acétonitrile dans une solution aqueuse d'acétate de triéthylammonium 0,05 M nous permet d'obtenir le **4'-SrU$_6$, 2** avec une pureté spectroscopique de 97,7 %.

*I-4-2) Analyse et purification de l'hexamère 4'-SrU$_6$-3'n-prOH* **3**.

L'analyse est effectuée dans les mêmes conditions de colonne et de gradient que pour l'hexamère 4'-SrU$_6$.

Le **4'-SrU$_6$-3'n-prOH, 3** a été obtenu avec une pureté spectroscopique à 260 nm de 51,6 %. Cette pureté est portée à 92 % après purification par CLPH preparative effectuée dans un gradient de 10 % à 13 % d'acétonitrile dans une solution aqueuse d'acetate de triéthylammonium 0,05 M en 15 minutes, suivi d'un pallier à 13 % d'acétonitrile dans une solution aqueuse d'acétate de triéthylammonium 0,05 M pendant 10 minutes.

**Exemple II: Synthèse du dodècamère mixte : 4'-SrU$_3$-dT$_6$-4'-SrU$_3$ / P$_{11}$ 4.**

***II-1) Synthèse du support solide.***

Le support solide utilisé pour cette synthèse est identique celui nécessaire à la synthèse du β**rSU$_{12}$**

***II-2) Synthèse automatisée du 4'-SrU$_3$-dT$_6$-4'-SrU$_3$ / P$_{11}$ 4.***

La synthèse du dodècamère est effectuée sur une colonne contenant 39,3 mg de support solide soit 0,819 µmole de 4'-thiouridine. Chaque cycle d'élongation nécessite un excès de synthons 4'-thiouridine phosphoramidite (16,75 µmoles, 20,3 éq) soit 100,5 µmole de synthons 4'-thio-uridine dissout dans 1,2 ml d'acétonitrile (0,083 M) et un excès de synthon désoxythymidine phosphoramidite (20 eq., 16,38 µmole) soit 98,28 µmole de dT en solution 0,1 M dans l'acétonitrile. La durée d'un cycle d'incorporation d'une unité désoxythymidine est de 6,73 mn, alors qu'elle est de 21,43 mn pour un cycle d'incorporation du synthon 4'-thioribouridine.

Le dosage des cations diméthoxytrityles libérés à chaque étape de détritylation a permis d'évaluer le rendement moyen d'incorporation à 98.0 %.

***II-3) Décrochage,déprotection et purification du 4'-SrU$_3$-dT$_6$-4'-SrU$_3$ / P$_{11}$ 4.***

Les étapes de décrochage de l'oligomère du support solide et de déprotection des liaisons internucléotidiques phosphotriester au thiophenol sont identiques à celles décrites dans l'exemple I.

*II-3-1) Analyse et Purification du 4'-SrU$_3$-dT$_6$-4'-SrU$_3$ P$_{11}$* **4** *par CLPH.*

L'analyse CLPH de l'oligomère **4** a été effectuée sur une colonne SFCC Nucléosyl C18 N125 2P789 dans un gradient à 10 % à 15 % d'acétonitrile dans une solution aqueuse d'acétate de triéthylammonium 0.05 M pendant 20 minutes (debit : 1 ml /min, temps d'analyse : 30 min). Dans ces conditions, le dodécamère mixte présente une pureté spectrophotométrique à 260 nm de 92.5 %.

Une purification par CLPH semi-préparative sur une colonne Nucleosyl avec un débit de 2 ml / min dans le même gradient augmente la pureté spectrophotométrique de la séquence mixte **4'-SrU$_3$-dT$_6$-4'-SrU$_3$ / P$_{11}$, 4** à 99,5 %.

**EXEMPLE III- Synthèse automatisée du 4'-SrU$_3$-dT$_6$-4'-SrU$_3$ / P$_3$PS$_5$P$_3$ 5.**

***III-1) Synthèse du support solide.***

Le support solide utilisé est le même que celui décrit dans l'exemple I.

***III-2) Synthèse automatisée du 4'-SrU$_3$-dT$_6$-4'-SrU$_3$ / P$_3$PS$_5$P$_3$ 5.***

La synthèse du dodécamère est effectuée sur une colonne contenant 39.5 mg de support fonctionnalisé soit 0,831 µmole (1 eq) de 4'-thiouridine. Chaque cycle d'élongation nécessite un excés de synthon 4'-thiouridine phosphoroamidite (16.75 µmole. 20.3 eq.) soit 100.5 µmole de 4' Sr-U au total et un excès de 20 eq. (16.38 µmole) de synthon désoxythymidine soit 98.28 µmole pour la synthèse entière.

La durée d'un cycle d'incorporation d'une désoxythymidine est de 7.7 minutes contre 21.43 minutes pour l'incorporation d'un 4'-thiouridine. Le dosage des cations dimethoxytrityles libérés indique un rendement moyen d'incorporation des synthons de 98.7 %.

***III-3) Décrochage, déprotection et purification du 4'-SrU$_3$-dT$_6$-4'-SrU$_3$/P$_3$PS$_5$P$_3$ 5.***

Le traitement de cet oligomère est identique à celui défini dans l'exemple I. L'analyse et la purification CLHP, réalisée sur une colonne SFCC Nucléosyl C18 N125 2P789 dans un gradient de 10 % à 20 % d'acétonitrile dans une solution aqueuse d'acétate de triéthylammonium 0.05 M pendant 20 minutes (débit : 1 ml /min. temps d'analyse 30 min). montre une pureté spectrophotométrique à 260 nm de 85 %.

La purification par CLPH semi-préparative sur une colonne Nucléosyl semi-préparative avec un débit de 2 ml / mm dans des conditions isocratiques à 19 % d'acétonitrile dans une solution aqueuse d'acétate de triethylammonium 0,05 M augmente la pureté de l'oligomère à 99.1 %.

**Exemple IV : Synthèse du dodécamère anti"*tat*" :**

**4'-Sr-(A-C-A-C-C-C-A-A-U-U-C-U) 1.**

***IV-1) Synthèse du support solide.***

Le support solide utilisé pour cette synthèse automatisée est identique à celui décrit dans l'exemple I.

***IV-2) Synthèse automatisée du dodécamère anti"tat":***

***4'-Sr-(A-C-A-C-C-C-A-A-U-U-C-U) 1.***

La synthèse de cet hétérododécamètre complémentaire du site accepteur d'épissage du gène *tat* du VIH est effectuée sur une colonne contenant 38.5 mg de support solide fonctionnalisé soit sur 0,808 µmole de 4'-thiouridine (1 eq.). Chaque cycle d'élongation utilise un excés de 25,24 eq (20,4 µmole) en synthon 4'-thioadénosine phosphoroamidite, 13,5 eq (10,97 µmole) en synthon 4'-thiocytidineet 30 eq. (24,25 µmole) en synthon 4'-thiouridine.

La durée d'un cycle d'incorporation est de 20,71 minutes et le dosage des cations diméthoxytrityles indique un rendement moyen d'incorporation des synthons phosphoramidites de 93,6 %.

***IV-3) Décrochage, déprotection et purification du 4'-Sr-(A-C-A-C-C-C-A-A-U-U-C-U) 1.***

Le support solide portant le dodécamère 4'-Sr-(A-C-A-C-C-C-A-A-U-U-C-U), 1 est traité par 5 ml d'une solution de thiophénol / triéthylamine / dioxane (1/2/1) pendant une demi heure à température ambiante. La solution de thiophénol est alors filtrée et le support solide est lavé avec 3 x 3 ml de méthanol, puis par une solution d'ammoniaque 32 % dans l'éthanol 95 (3/1 : v/v) (3 x 500 µl) afin de décrocher l'oligomère de son support. La solution obtenue est mise à incuber dans une étuve sèche thermostatée à 55 °C pendant 5 heures afin de déprotéger les groupements protecteurs des bases hétérocycliques. Elle est ensuite évaporée, reprise dans 500 µl d'eau et lyophilisée. L'oligomère lyophilisé est alors dissout dans 300 µl d'une solution de TBAF (1.1 M) dans le THF. Le mélange réactionnel est laissé à température ambiante pendant 24 heures. La réaction est alors stoppée avec 300 µl d'une solution aqueuse d'acétate d'ammonium 0.05 M. La solution est évaporée à sec. co-evaporée avec 3 portions de 500 µl d'eau et la résidu est dessalé sur une colonne de gel d'exclusion DEAE Sephadex G-25. Les fractions contenant le 4'-thiooligomère sont rassemblées, évaporées à sec, redissoutes dans 1,2 ml d'eau et filtré sur filtre millipore avant d'être analysée par CLPH.

***IV-4) Analyse et purification du 4'-Sr-(A-C-A-C-C-C-A-A-U-U-C-U) 1.***

L'analyse CLPH analytique du dodécamère **1** a été éffectuée sur une colonne SFCC Nucléosyl C18 N125 2P789

dans un gradient de 10% à 15 % d'acétonitrile dans une solution aqueuse d'acétate de triéthylammonium 0,05 M avec un débit de 1 ml / min et un temps d'analyse de 30 minutes. Le chromatogramme indique une pureté spectrophotométrique à 260 nm de 44,9 %. Une purification par CLPH semi-préparative sur une colonne Nucléosyl C18 semi-préparative en utilisant le même gradient avec un débit de 2 ml / min et un temps d'analyse de 30 minutes amène la pureté spectroscopique du **4'-Sr-(A-C-A-C-C-C-A-A-U-U-C-U), 1** à 100 %.

### IV. PROPRIETE D'HYBRIDATION DES β-4'-THIO-OLIGONUCLEOTIDES.

La spectrophotométrie U. V. permet d'étudier les acides nucléiques et de mettre en évidence la formation de duplex, la température de fusion étant une des caractéristiques d'un duplex. L'empilement et l'appariement des bases des acides nucléiques sont accompagnés d'une diminution de l'absorbtion U.V. (Hypochromicité). Ainsi par spectrophotométrie U.V. il est possible de controler la formation ou la dissociation d'un duplex. (W SANGER. *Principles of Nucleic Acid Structure.* Springer-Verlag,.New-York, 1984. pp. 141-149). Lorsque la température d'une solution contenant une double hélice (ADN ou ARN) est lentement augmentée, l'absorbtion U.V. croit nettement tout au long de la dissociation. Le point d'inflexion de la courbe d'allure sygmoïdale de la variation d'absorbance en fonction de la température est appelée température de fusion ou Tm et correspond à la co-existence de brins séparés et de brins appariés dans un rapport 50 / 50.

L'analyse des courbes de fusion des duplex β-4'-thiooligoribonucléotide / β-oligodésoxyribonucléotide et β-4'-thiooligoribonucléotide / β-oligoribonucléotide permettra de définir la stabilité thermique de tels duplex par la mesure de la température de fusion. Ces expériences d'hybridation sont menées en respectant une stoechiométrie 1 / 1 entre les deux brins complémentaires, en présence d'un tampon 1 M NaCl, 10 mM cacodylate de sodium. Une forte concentration en NaCl favorise en effet les appariements en solvatant les charges négatives autour des atomes de phosphore des deux oligonucléotide. Nous avons donc effectué ces expériences d'hybridation dans un autre tampon : 0.1 M NaCl . 10 mM cacodylate de sodium.

### IV. 1. RESULTATS ET DISCUSSION

**Exemples I et II: Expériences de fusion des duplex $\beta SrU_{12}$ /polyrA et $\beta rU_{12}$ / polyrA.**

Ces expériences permettent de mettre en évidence l'influence de l'atome de soufre en position 4' du dodécamère modifié sur la stabilité thermique du duplex en comparant les Tm obtenus avec ceux du duplex naturel.

Le tableau 4 résume les températures de fusion obtenues dans les deux expériences

Tableau 4

| Températures de fusion des duplex de dodécamères<br>Concentration 3 μM en chaque oligomère. | | |
|---|---|---|
| Concentration en NaCl | $\beta SrU_{12}$/polyrA °C Hypochromicité % | $\beta rU_{12}$/polyrA °C Hypochromicité % |
| 1M | 46°C 25% | en cours |
| 0.1M | 33°C 25% | en cours |

L'analyse de ces résultats montrent que le duplex $\beta SrU_{12}$ / polyrA présente une bonne stabilité thermique représentée par le Tm de 46°C obtenu à une concentration de 1 M en NaCl.

**Exemples III et IV : Expériences de fusion des duplex $\beta SrU_{12}$ / $\beta dC_2A_{12}$C2 et $\beta rU_{12}$ / $\beta dC_2A_{12}C_2$.**

Les températures de fusion obtenues dans les deux expériences sont résumées dans le tableau 5.

Tableau 5

| Températures de fusion des duplex de dodécamères<br>Concentration 3 μM en chaque oligomères | | |
|---|---|---|
| Concentration en NaCl | $\beta SrU_{12}/\beta dC_2A_{12}C_2$ °C Hypochromicité % | $\beta rU_{12}/\beta dC_2A_{12}C_2$ °C Hypochromicité % |
| 1M | 27°C 9,3% | en cours |
| 0.1M | 5°C 5,6% | en cours |

**Exemple IV : Expérience d'autohybridation de l'oligomère $\beta SrU_{12}$.**

Afin de vérifier les résultats précédents, il est nécessaire de s'assurer que le dodécamère $\beta SrU_{12}$ ne conduit à aucune autohybridation. Pour cela, on a effectué une expérience d'auto-hybridation du dodécamère $\beta SrU_{12}$ à deux concentrations en NaCl (1 M, et 0,1 M). Les courbes d'hybridation ne présentent pas l'allure sigmoïdale caractéristique mais obéissent à l'équation A = k+T où A est l'absorbance à 260 nm. T la température et k une constante. Aucun point d'inflexion ne peut être visualisé sur une telle droite Nous pouvons donc conclure que le dodécamère $\beta SrU_{12}$ ne s'auto-apparie pas.

**La comparaison des températures de fusion des duplex $\beta SrU_{12}$ / $\beta dC_2A_{12}C_2$ (Tableau 5) et $\beta SrU_{12}$ / polyrA (Tableau 4) montre une stabilité thermique de l'homoduplex ARN/ARN (Tm = 46° C) nettement supérieure à celle de l'hétéroduplex ARN/ADN (Tm = 27° c) d'autre part, aucune auto-hybridation de $\beta SrU_{12}$ n'a été constatée.**

**Exemple VI et VII: Expérience de fusion des duplex $\beta dT_5(SrT)dT_6$ / $\beta dC_2A_{12}C_2$ et $\beta dT_{12}$ / $\beta dC_2A_{12}C_2$**

Cette expérience permet d'évaluer la stabilité thermique du duplex $\beta dT_5(SrT)dT_6$ / $\beta dC_2A_{12}C_2$ comparativement à celle du duplex naturel $\beta dT_{12}$ / $\beta dC_2A_{12}C_2$. (Figure 1). Les températures de fusion de ces duplex sont indiquées dans le tableau 6.

Tableau 6

| Température de fusion des duplex $\beta dT_5(SrT)dT_6$ / $\beta dC_2A_{12}C_2$ et $\beta dT_{12}$ / $\beta dC_2A_{12}C_2$ Concentration en chaque oligomère 10 µM | | |
|---|---|---|
| Concentration en NaCl | $\beta dT_5(SrT)dT_6/\beta dC_2A_{12}C_2$ °C Hypochromicité % | $\beta dT_{12}/\beta dC_2A_{12}C_2$ °C Hypochromicité % |
| 1M | 40°C 18% | 45°C 20.5 % |

Au vu de ces résultats, il apparait que la stabilité thermique du duplex modifié est inférieure à celle du duplex naturel. Cependant la différence de 5°C existante entre les deux températures de fusion est trop faible pour conclure à un mésappariement. En effet certains auteurs (Y.KAWASE, S.IWAI, H.INOUE, K.MIURA et E.OKTSUKA. *Nucleic Acid Research,* 1986, **14**, 7727) ont montré que l'existence d'un seul mésappariement dans un duplex de 13 unités nucléotidiques se traduisait par une diminution de Tm supérieure à 10°C.

**On peut donc conclure que l'unité 4'-thio-nucléotidique s'apparie bien avec la 2'-désoxyadénosine mais avec une affinité inférieure à celle de la 2'-désoxythymidine comme le confirme la différence de Tm de 5° C obtenue (Tableau 6)**.

**IV. 2. PARTIE EXPERIMENTALE**

**Méthode Générale pour les expériences d'hybridation**

*A - Dosage des oligomères complémentaires.*

Afin de réaliser l'expérience d'hybridation, il est nécessaire de connaître la concentration en chaque oligomère pour respecter la stoechiométrie 1:1 entre les deux brins complémentaires. Selon la loi de BEER-LAMBERT A = εlc. les absorbances des oligomères ont été mesurées à 80°C afin d'éliminer le phénomène d'empilement des bases créant une perturbation de l'hyperchromicité due à l'existence de structures tertiaires. On peut donc connaitre les coefficients d'extinction moléculaire des dodécamères à 80°C selon la relation $\varepsilon^{265}(SrU_{12}) = 12\varepsilon^{265}(rU)$.

*B - Conditions d'hybridation.*

Les expériences d'hybridation sont effectuées avec un spectrophotomètre UVIKON 810 (KONTRON), couplé à un microordinateur IBM compatible. La température est controlée par un programmateur de température HUBER PD 415 connecté à un bain thermostaté (HUMER MINISTAT). Les cuves U.V. sont en quartz de 1 cm de long et une circulation continuelle d'azote est utilisée pour les températures inférieures à 20°C. Avant l'expérience, 3 µM (Exemples I à V) ou 10 µM (Exemples VI et VII) d'oligomères complémentaires sont mis en présence dans une quantité suffisante pour 1000 µl de tampon 1 M NaCl et 10 mM cacodylate de sodium, ajusté à pH 7 (Exemples I à VII). Ce mélange est chauffé à 80°C pendant 1 heure, puis refroidit jusqu'à - 20°C selon une pente de température de 0,5°C par minute. Les valeurs d'absorbance et de température sont collectées toutes les 30 secondes.

La même expérience est effectuée en utilisant une quantité suffisante pour 1000 µl de tampon d'hybridation 0,1 M NaCl, 10 mM cacodylate de sodium ajusté a pH 7 afin d'évaluer la stabilité thermique du duplex dans ce tampon favorisant moins les appariements

**Exemple I : Stabilité thermique du duplex $\beta SrU_{12}$ / polyrA**

*I-1 Dosage des oligomères*
Concentration de $\beta SrU_{12}$ = 0,351mM
Concentration de polyrA = 4,61 mM

*I-2 Conditions d'hybridation*
3 µM (8,55 µl) de $\beta SrU_{12}$ et 3 µM (7,79 µl) de polyrA sont mis en présence de 983,6 µl des tampons d'hybridation (1 M NaCl, 10 mM cacodylate de sodium ou 0,1 M NaCl, 10 mM cacodylate de sodium)

**Exemple II : Stabilité thermique du duplex $\beta rU_{12}$ / polyrA**

en cours

**Exemple III : Stabilité thermique du duplex $\beta SrU_{12}$ / $\beta dC_2A_{12}C_2$**

*III-1 Dosage des oligomères*
Concentration de $\beta SrU_{12}$ = 0,351 mM
Concentration de $\beta dC_2A_{12}C_2$ = 0,369 mM

*III-2 Conditions d'hybridation*
3 µM (8.55 µl) de $\beta SrU_{12}$ et 3 µM (8,11 µl) de $\beta dC_2A_{12}C_2$ sont mis en présence de 983,3 µl des tampons d'hybridation (1 M NaCl, 10 mM cacodylate de sodium ou 0.1 M NaCl. 10 mM cacodylate de sodium)

**Exemple IV: Stabilité thermique du duplex $\beta rU_{12}$ / $\beta dC_2A_{12}C_2$** (en cours)

*IV-1 Dosage des oligomères*
Concentration de $\beta rU_{12}$ =
Concentration de $\beta dC_2A_{12}C_2$ = 0,369 mM

*III-2 Conditions d'hybridation*
3 µM ( µl) de $\beta rU_{12}$ et 3 µM (8,11 µl) de $\beta dC_2A_{12}C_2$ sont mis en présence de µl des tampons d'hybridation (1 M NaCl, 10 mM cacodylate de sodium ou 0.1 M NaCl, 10 mM cacodylate de sodium)

**Exemple V: Expérience d'autohybridation de l' oligomére $\beta SrU_{12}$**

V-1 *Dosage des oligomères*
Concentration de $\beta SrU_{12}$ = 0,351 mM

*V-2 Conditions d'hybridation*
6 µM (17,10 µl) de $\beta SrU_{12}$ sont mis en présence de 986,9 µl des tampons d'hybridation (1 M NaCl, 10 mM cacodylate de sodium ou 0,1 M NaCl, 10 mM cacodylate de sodium)

**Exemple VI: Stabilité thermique du duplex $\beta dT_5(SrT)dT_6$ / $\beta dC_2A_{12}C_2$**

*VI-1 Dosage des oligomères*
Concentration de $\beta dT_5(SrT)dT_6$ = 1,33 mM
Concentration de $\beta dC_2A_{12}C_2$ = 0,369 mM

*VI-2 Conditions d'hybridation*
10µM (7,49µl) de $\beta dT_5(SrT)dT_6$ et 10 µM (22,5 µl) de $\beta dC_2A_{12}C_2$ sont mis en présence de 970 µl de tampon d'hybridation 1M NaCl, 10 mM cacodylate de sodium.

**Exemple VII: Stabilité thermique du duplex $\beta dT_{12}$ / $\beta dC_2A_{12}C_2$**

*VII-1 Dosage des oligomères*

Concentration de $\beta dT_{12}$= 1,196 mM

Concentranon de $\beta dC_2A_{12}C_2$ = 0,369 mM

*VII-2 Conditions d'hybridation*

10 µM (8,36 µl) de $\beta dT_{12}$ et 10 µM (22,5 µl) de $\beta dC_2A_{12}C_2$ sont mis en présence de 969,1 µl de tampon d'hybridation 1 M NaCl, 10 mM cacodylate de sodium.

**V. PROPRIETES D'HYBRIDATION DES 4'-S-ARN ET OLIGOMERES MIXTES.**

Les expériences d'hybridation sont menées en respectant la stoechiométrie 1/1 entre les deux brins complémentaires en présence d'un tampon 1 M NaCl, 10 mM cacodylate de sodium. Une forte concentration en NaCl favorise en effet les appariements en solvatant les charges négatives autour des atomes de phosphore des deux oligonucléotides. Nous avons effectué ces expériences d'hybridation dans un autre tampon 0,1 M NaCl, 10 mM cacodylate de sodium.

**V-1-RESULTATS ET DISCUSSION.**

Les expériences de fusion des duplex :

[4'-Sr$U_3$-d$T_6$-4'-Sr$U_3$ / $P_{11}$] / polyrA, [**4** / polyrA]

[4'-Sr$U_3$-d$T_6$-4'-Sr$U_3$ / $P_{11}$] / d($C_2A_{12}C_2$), [**4** / d($C_2A_{12}C_2$)]

[4'-Sr$U_3$-d$T_6$-4'-Sr$U_3$ / $P_3PS_5P_3$] /polyrA, [**5** / polyrA]

[4'-Sr$U_3$-d$T_6$-4'-Sr$U_3$ / $P_3PS_5P_3$] / d($C_2A_{12}C_2$), [**5** / d($C_2A_{12}C_2$)]

sont effectuées à deux concentrations en NaCl et permettent d'évaluer l'influence des liaisons phosphorothioates sur la stabilité d'hybridation comparativement aux oligonucléotides avec des liaisons phosphodiester normales (tableau 7).

TABLEAU 7

| Résultats des expériences de fusion. | | |
|---|---|---|
| NaCl concentration (M) | Duplex | Tm °C |
| 1 | **4**/poly rA | 45 |
| 1 | **5**/poly rA | 36 |
| 1 | **4**/d($C_2A_{1\ 2}C_2$) | 31 |
| 1 | **5**/d($C_2A_{12}C_2$) | 19 |
| 0.1 | **4**/poly rA | 32 |
| 0.1 | **5**/poly rA | 24 |
| 0.1 | **4**/d($C_2A_{12}C_2$) | 10 |
| 0.1 | **5**/d($C_2A_{12}C_2$) | 3 |

Le dodécamère mixte **5** présentant à la fois des liaisons internucléotidiques de type phosphodiester et phosphorothioate ($P_3PS_5P_3$) induit une diminution de la valeur du Tm d'environ 2°C par lien phosphorothioate. Par contre, l'oligomère **4** présente des valeurs de Tm qui sont équivalentes à celles obtenues pour le dodécamère $\beta rSU_{12}$ avec les deux séquences complémentaires poly rA et d($C_2A_{12}C_2$). Ces résultats (tableau 7) indiquent que les oligomères de séquence mixte **4** et **5** présentent une bonne stabilité thermique avec des séquences d'ARN complémentaires.

**PARTIE EXPERIMENTALE**.

**Méthode Générale pour les expériences d'hybridation**

*A - Dosage des oligomères complémentaires.*

Afin de réaliser l'expérience d'hybridation, il est nécessaire de connaître la concentration de chaque oligomére pour respecter la stoechiométrie 1:1 entre les deux brins complémentaires. Selon la loi de BEER-LAMBERT A = εlc,

les absorbances des oligomères ont été mesurées à 80°C afin d'éliminer le phénomène d'empilement des bases créant une perturbation de l'hyperchromicité dûe à l'existence de structures tertiaires. On peut donc connaître les coefficients d'extinction moléculaire des dodécamères à 80°C selon une relation semblable à $\varepsilon^{265}(SrU_{12}) \sim 12\ \varepsilon^{265}(SrU)$.

*B - Conditions d'hybridation*

Les expériences d'hybridation sont effectuées avec un spectrophotomètre UVIKON 810 (KONTRON), couplé à un microordinateur IBM compatible. La température est controlée par un programmateur de température HUBER PD 415 connecté à un bain thermostate (HUMER MINISTAT). Les cuves U.V, sont en quartz de 1 cm de long et une circulation continuelle d'azote est utilisée pour les températures inférieures à 20°C. Avant l'expérience, 3 µM (Exemples I à IV) d'oligomères complémentaires sont mis en présence dans une quantité suffisante pour 1000 µl de tampon 1 M NaCl et 10 mM cacodylate de sodium ajusté a pH 7 (Exemples I à VII). Ce mélange est chauffé à 80°C pendant 1 heure, puis refroidit jusqu'à - 20°C selon une pente de température de 0.5°C par minute. Les valeurs d'absorbance et de température sont collectées toutes les 30 secondes.

La même expérience est effectuée en utilisant une quantité suffisante pour 1000 µl de tampon d'hybridation 0,1 M NaCl, 10 mM cacodylate de sodium ajusté à pH 7 (Exemples I et IV) afin d'évaluer la stabilité thermique du duplex dans ce tampon favorisant moins les appariements

**Exemple I : Stabilité thermique du duplex [4'-SrU$_3$-dT$_6$-4-SrU$_3$ / P$_{11}$] / polyrA, 4/polyrA.**

*I-1 Dosage des oligomères*
Concentration de **4** = 0,821 mM
Concentration de polyrA = 4,61 mM.

*I-2 Conditions d'hybridation*
3 µM (3.65 µl) de **4** et 3 µM (7,79 µl) de polyrA sont mis en présence de 988,5 µl des tampons d'hybridation (1 M NaCl, 10 mM cacodylate de sodium ou 0,1 M NaCl, 10 mM cacodylate de sodium).

**Exemple II : Stabilité thermique du duplex 4'-SrU$_3$-dT$_6$-4'-SrU$_3$ / P$_{11}$ / dC$_2$A$_{12}$C$_2$, 4/dC$_2$A$_{12}$C$_2$**

*II-1 Dosage des oligomères*
Concentration de **4** = 0,821 mM
Concentration de $dC_2A_{12}C_2$ = 0,369 mM

*II-2 Conditions d'hybridation*
3 µM (3.63 µl) de **4** et 3 µM (8,11 µl) de $dC_2A_{12}C_2$ sont mis en présence de 988,2 µl des tampons d'hybridation (1 M NaCl, 10 mM cacodylate de sodium ou 0,1 M NaCl, 10 mM cacodylate de sodium)

**Exemple III: Stabilité thermique du duplex [4'-SrU$_3$-dT$_6$-4'-SrU$_3$ / P$_3$PS$_5$P$_3$] / polyrA, 5 / polyrA**

*III-1 Dosage des oligomères*
Concentration de 5 = 0,40 mM.
Concentration de polyrA = 4.61 mM.

*III-2 Conditions d'hybridation*
3 µM (7,5 µl) de **5** et 3 µM (7,79 µl) de polyrA sont mis en présence de 984,7 µl des tampons d'hybridation (1 M NaCl, 10 mM cacodylate de sodium ou 0,1 M NaCl, 10 mM cacodylate de sodium)

**Exemple IV: Stabilité thermique du duplex [4'-SrU$_3$-dT$_6$-4'-SrU$_3$ / P$_3$PS$_5$P$_3$] / dC$_2$A$_{12}$C$_2$, 5 / polyrA**

*IV-1 Dosage des oligomères*
Concentration de **5** = 0,40 mM
Concentration de $dC_2A_{12}C_2$ = 0,369 mM

*IV-2 Conditions d'hybridation*
3 µM (7,5 µl) de **5** et 3 µM (8,11 µl) de $dC_2A_{12}C_2$ sont mis en présence de 984,4 µl des tampons d'hybridation (1 M NaCl, 10 mM cacodylate de sodium ou 0,1 M NaCl, 10 mM cacodylate de sodium).

## VI. ETUDES ENZYMATIQUES

La reconnaissance des acides nucléiques par des enzymes est en majeure partie d'ordre structural. Le remplacement de l'hétéroatome naturel du sucre d'un oligonucléotide confére à celui-çi une forte résistance à la dégradation par les nucléases:

L'étude de l'hydrolyse des 4'-thio-oligoribonucléotides β par ces nucléases permet de confirmer que le remplacement de l'oxygène intracyclique par un atome de soufre confère bien une meilleure stabilité enzymatique.

**Exemple I: Etude de la dégradation enzymatique du dodécamère β(SrT)dT$_{11}$ par la phosphodiestérase de rate de veau.**

La phosphodiestérase de rate de veau, est une exonucléase qui dégrade les oligoméres à partir de leurs extremités 5' libre libérant ainsi des nucléotides 3' phosphate.

Cette étude nous permettra d'évaluer la résistance à la dégradation enzymatique induite par la présence en position 5' d'une unité 4'-thionucléotidique dans l'oligomére β(SrT)dT$_{11}$ comparativement à la dégradation enzymatique de l'homologue naturel oxygéné βdT$_{12}$. La stabilité comparative des deux dodécamères βdT$_{12}$ et β(SrT)dT$_{11}$ envers l'hydrolyse par la phosphodiestérase de rate de veau est présentée dans le tableau 8.

La différence de comportement entre les deux dodécamères est importante, en effet seulement 23% de l'oligomère β(SrT)dT$_{11}$ a été hydrolysé en 25 minutes alors que dans le même laps de temps le 12-mer βdT$_{12}$ a été dégradé à 93%.

Tableau 8

| Cinétique de dégradation de βdT$_{12}$ et β(SrT)dT$_{11}$ par la phosphodiestérase de rate de veau | | |
|---|---|---|
| Dodécamère | Dégradation en 0 min | Dégradation en 25 min |
| βdT$_{12}$ | 0% | 93% |
| β(SrT)dT$_{11}$ | 0% | 23% |

L'évolution au cours du temps de la dégradation enzymatique des deux dodécamères a été suivie par CLHP. Nous avons pu tracer la courbe de digestion enzymatique de β(SrT)dT$_{11}$ en fonction du temps : $A = A_0 e^{-kt}$ (Figure 2, A) où:

- $A_0$ est l'aire initiale du 12-mer
- A est l'aire du 12-mer à l'instant t
- t est le temps
- k est la constante de vitesse de premier ordre de la dégradation

Cette courbe (Figure 2) nous permet de calculer, en utilisant le logiciel de régression curviligne EUREKA, k = 0.016min$^{-1}$ et le temps de demi-vie du dodécamère $t_{1/2} = \ln 2/k = 43$ min

De la même façon, la courbe de digestion enzymatique de βdT$_{12}$ en fonction du temps $A = A_0 e^{-kt}$ a pu être tracée. (Figure 2). On constate que la cinétique de dégradation de βdT$_{12}$ est beaucoup plus rapide que celle de β(SrT)dT$_{11}$ ; en effet le temps de demi-vie s'établit à 1 minute pour le dodécamère naturel.

La cinétique de dégradation enzymatique de l'oligomére modifié β(SrT)dT$_{11}$ par l'extrémité 5' présente un temps de demi-vie nettement supérieur à celui de la dégradation du βSrU$_{12}$. Ces résultats montrent que l'introduction d'une unité thio-ribonucléotidique à l'extrémité 5' d'un oligomère stabilise ce dernier vis à vis de la phosphodiéstérase de rate de veau.

Une solution d'oligonucléotide (20 µl, 3 unités d'absorbance à 260 nm) est diluée dans un tampon Acétate d'ammonium 0,125 M (pH 7,0), d'EDTA 2,5mM et de Tween 80 0.0625% (80 µL). On ajoute une solution commerciale de phosphodiestérase de rate de veau (2 µl, concentration finale 0,008 unité/ml) et le mélange est incubé à 37°C. A des temps déterminés, des fractions aliquotes sont prélevées (5 µl), chauffées à 100°C pendant une minute et analysées par CLHP.

*Conditions d'analyse CLHP.*

Les analyses ont été effectuées sur une colonne PVDI ( Polyvinylimidazole SFCC-Shandon) protégée par une précolonne et un préfiltre. L'élution est réalisée selon un gradient linéaire en 20 minutes de 0,1 M à 0,4 M KCl dans 20 mM $KH_2PO_4$ à pH 6 contenant 20% d'acetonitrile. Le débit était fixé à 1 ml/min et la détection U.V. à 260 nm.

**EXEMPLE II : Etude des activités de type substrat des hexamères 4'-Sr$U_6$, 4'-Sr$U_6$-3'n-prOH comparativement aux caractères substrats des hexamères βr$U_6$ et αr$U_6$ vis-à-vis de différentes nucléases purifiées.**

Quatre nucléases typiques ont été utilisées :

- L'endonucléase S1, spécifique du simple brin d'ADN hydrolyse les liaisons phosphodiesters de façon aléatoire.
- La phosphodiestérase de rate de veau (CSP) est une 5'-exonucléase qui dégrade les oligonucléotides à partir de leur extrémité 5' et libère des nucléotides 3'-phosphates.
- La phosphodiestérase de venin de serpent (VSP) est une 3'-exonucléase-3' qui libère des nucléotides 5'-phosphate après avoir dégradé l'oligomère par son extrémité 3'.
- La ribonucléase A dégrade un oligoribonucléotide après les résidus pyrimidiques.

Ces quatre enzymes ont été obtenus auprès de Boehringer Mannheim.

De façon générale, une unité de densité optique à 260 nm de chaque hexamère est mis en présence d'une certaine concentration d'enzyme considérée puis est diluée en quantité suffisante pour 1000µl de tampon de digestion enzymatique.

Différents tampons de digestion ont été utilisés en fonction de la nature de l'enzyme (Tableau 9 ).

Tableau 9 :

| Concentrations en enzyme et tampons de digestion utilisés | | |
|---|---|---|
| Enzyme | Concentration finale en enzyme (unités d'activité enzymatique/ml) | Tampon de digestion enzymatique |
| CSP | $13.10^{-3}$ | EDTA 0.025 mM pH 7 $AcONH_4^+$ 0.125 mM |
| VSP | $6.10^{-4}$ | Tris, HCl 0.1 N pH = 9,4 $MgCl_2$ 0.01 M |
| S1 | 20 | AcONa 0.05N NaCl 0.3 M pH 4.7 AcZn 0.1 M |
| RNAse A | $2.10^{-2}$ | NaCl 300 mM Tris, HCl 10 mM pH 7.4 EDTA 5 mM |

L'échantillon mère est divisé en 10 fractions de 100 µl avant d'être congelé à -18°C

Une fraction aliquote de 100 µl est prélevée et incubée à l'étuve sèche thermostatée à 37°C pendant un temps t puis est analysée par CLHP analytique.

L'étude de l'aire du signal correspondant à l'hexamère permet de mesurer une cinétique de dégradation dont le temps de demi-vie est calculé (Tableau 10).

Tableau 10:

| Dégradations enzymatiques. | | | | |
|---|---|---|---|---|
| | Temps de demi-vie (min) | | | |
| Nucléases | **αr$U_6$** | **βr$U_6$** | **βSr$U_6$** | **βSr$U_6$3'$(CH_2)_3$OH** |
| 5'-exonucléase Phosphodiestérase de rate de veau | * | 17 | 3900 | - |
| 3'-exonucléase Phosphodiestérase de venin de serpent | 110 | 1 | 76 | 250 |
| Endonucléase S1 | * | 120 | 930 | - |
| Ribonucléase A | * | <1 | 670 | - |

* Aucune dégradation observée après 4 jours d'incubation

L'hexamère **4'-Sr$U_6$, 2** présente une grande résistance enzymatique comparée à celle du β **r$U_6$** vis à vis de quatre nucléases (CSP, $S_1$, RNase-A et VSP).

La résistance à la 3'-exonucléase est fortement augmentée par l'introduction d'une liaison phosphodiester propanol sur l'extrémité 3' du **4'-Sr$U_6$ 3'n-pr-OH, 3.**

CONCLUSION.

Les études ont montré que :

a) les 4'-thio-oligoribonucléotides homogènes ou mixtes, se lient avec une bonne stabilité thermodynamique avec un ARN complémentaire.

b) la substitution de l'oxygène cyclique par un atome de soufre induit une forte résistance à la dégradation enzymatique ( cf. la comparaison des temps de demie-vie de β-$rU_6$ et β-S-$rU_6$ )

L'ensemble de ces données implique que les 4'-thio-oligoribonucléotides peuvent être considérés comme agents antisens sous forme de séquences homogènes ou inclus dans des oligomères mixtes. De plus, la présence de la fonction hydroxyle en 2' permet d'envisager leur utilisation comme ribozymes artificiels que ce soit sous forme de séquences homogènes ou mixtes associées à des oligonucléotides de type divers ADN ou ARN modifiés ou non au niveau de l'enchaînement phosphate.

**Revendications**

**1.** Composés chimiques constitués par un oligo-4'-thio-2'désoxyribonucléotide ou un oligo 4'-thioribonucléotide, caractérisés en ce qu'ils comportent un enchaînement de 4'-thio-2'désoxyribonucléotides ou 4'-thioribonucléotides respectivement, enchaînement pouvant être facultativement lié à un agent effecteur, notamment un radical correspondant à un agent d'intercalation ou un radical chimique ou photoactivable, tel qu'un radical porteur d'une fonction réagissant directement ou indirectement avec les chaînes de nucléotides ou un radical dont la présence permet une détection facile et sensible.

**2.** Composés chimiques selon la revendication 1 constitués par un oligo 4'-thioribonucléotide ou un oligo 4'-thio-2'-désoxyribonucléotide, caractérisés en ce qu'ils comportent un enchaînement de 4-thioribonucléotides ou 4-thio-2'-déoxyribonucléotides respectivement non lié à un radical effecteur.

**3.** Composés selon la revendication 1 ou 2 caractérisés en ce qu'ils ont pour formule :

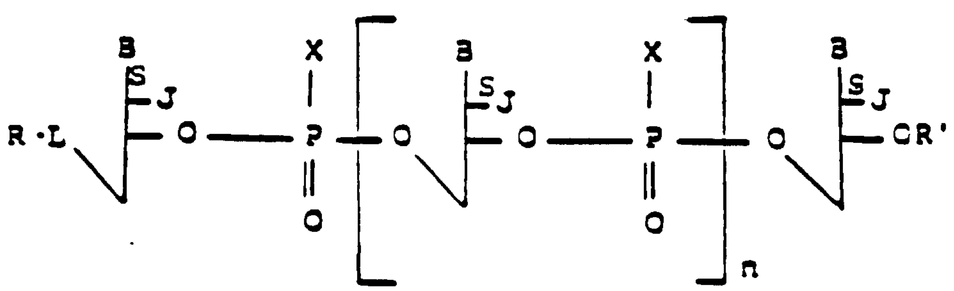

(I)

dans laquelle :

les radicaux B peuvent être identiques ou différents et représentent chacun une base d'un acide nucléique éventuellement modifiée, activable et/ou comportant un groupement intercalant et rattachée au cycle glycosidique selon une configuration anomérique alpha non naturelle ;
les radicaux X peuvent être identiques ou différents et représentent chacun un oxoanion $O^-$, un thioanion $S^-$, un groupe alkyle, un groupe alcoxy, un groupe thioalkyle, un radical alkyle ou alcoxy substitué par un hétérocycle azoté ou un groupement -Y-Z ;
R et R', qui peuvent être identiques ou différents, représentent chacun un atome d'hydrogène ou un groupement -Y-Z ou Y'-Z' ;
Y et Y' identiques ou différents représentent chacun un radical alcoylène droit ou ramifié -alk- ou un radical choisi parmi

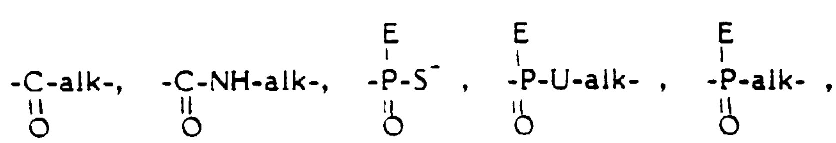

alk-O-alk

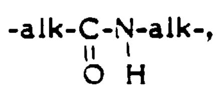, 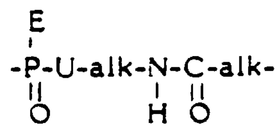 et 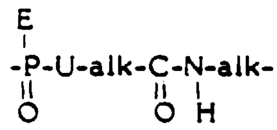

avec U = O, S ou N
E peut avoir les mêmes significations que X excepté Y-Z ou Y'-Z' ;
J représente un atome d'hydrogène ou un groupe hydroxy ;
Z et Z', identiques ou différents, représentent chacun CH ou un radical correspondant à un agent effecteur, notamment un radical correspondant à un agent d'intercalation ou un radical porteur d'une fonction qui réagit directement ou indirectement avec les chaînes de nucléotides ou un radical dont la présence permet une détection facile et sensible.
n est un nombre entier y compris 0 ;
L représente un atome d'oxygène, un atome de soufre ou un groupe -NH-.

4. Composés selon la revendication 3, caractérisés en ce que Y et Y' représentent un radical choisi parmi

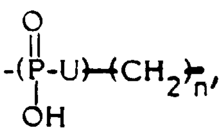 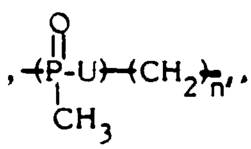 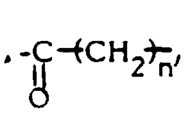 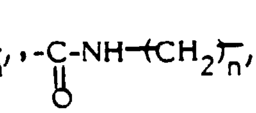

avec U = O, S ou N et n' est un nombre entier notamment de 1 à 10.

5. Composés selon la revendication 3 caractérisés en ce que L représente l'oxygène, R et R' représentent un atome d'hydrogène et B représente une base nucléique naturelle.

6. Composés selon l'une des revendications 3 à 5, caractérisés en ce que l'agent d'intercalation Z ou Z' est choisi parmi les composés polycycliques ayant une configuration plane.

7. Composés selon la revendication 6 caractérisés en ce que l'agent d'intercalation Z ou Z' est choisi parmi l'acridine et ses dérivés, la furocoumarine et ses dérivés, la daunomycine et les autres dérivés de l'anthracycline, la 1,10-phénanthroline, la phénanthridine et ses dérivés, la proflavine, les porphyrines, les dérivés du dipyrido [1,2-a:3'-d] imidazole, l'ellipticine ou l'ellipticinium et leurs dérivés et le diazapyrène et des dérivés.

8. Composés selon l'une des revendications 3 à 5, caractérisés en ce que les groupes chimiques réactifs Z ou Z' sont choisis parmi l'acide éthylène-diamine-tétraacétique, l'acide diéthylènetriaminepentaacétique, les porphyrines, la 1,10-phénanthroline, l'azido-4 acétophénone, l'éthylèneimine, la bêta-chloroéthylamine, le psoralène et leurs dérivés.

9. Composés selon l'une des revendications 3 à 8, caractérisés en ce que le radical B est un radical correspondant à la thymine, l'adénine; la 2-aminoadénine et ses dérivés substitués, la cytosine, la guanine et ses dérivés substitués, l'uracile, le bromo-5 uracile, l'azido-8 adénine, la 7-déazaadénine, la 7-déazaguanine, la 4-azido-cytosine, la 4-azido-thymine et les dérivés de ces bases comportant un groupement intercalant et/ou un groupement chimiquement ou photochimiquement activable.

10. Composés selon l'une des revendications 1 à 9, caractérisés en ce qu'il s'agit de composés oligothioribonucléotides de formule I pour lesquels J = OH.

11. Composés oligomères mixtes caractérisés en ce que ils sont constitués par des composés selon l'une des revendications 1 à 10 liés à des oligonucléotides de type ADN ou ARN modifiés ou non au niveau de l'enchaînement phosphate et dans lesquels l'hétéroatome intracyclique du cycle furannose des nucléotides est l'oxygène.

**12.** Composés oligomères mixtes caractérisés en ce que ils sont constitués par un composé selon l'une des revendications 1 à 10, notamment de formule (I), comprenant, à une ou à chacune de ses extrémités, une séquence oligonucléotidique de type ADN ou ARN modifiée ou non au niveau de l'enchaînement phosphate, dans lequel l'hétéroatome du cycle furannose des nucléotides est l'oxygène.

**13.** Composés oligomères mixtes selon la revendication 12 caractérisés en ce que ils sont constitués par un composé oligothioribonucléotidique selon l'une des revendications 1 à 10, comprenant une séquence de type ADN modifiée ou non au niveau de l'enchaînement phosphate dans les nucléotides de laquelle l'hétéroatome du cycle furannose des nucléotides est l'oxygène.

**14.** Composé oligomère mixte selon la revendication 13, caractérisé en ce que la dite séquence d'ADN, dans laquelle l'hétéroatome du cycle furannose est l'oxygène, est encadré par deux séquences du type thioribonucléotidique.

**15.** Procédé de préparation de composés selon l'une des revendications 1 à 13, caractérisé en ce qu'on applique des synthèses au phosphodiester, phosphotriester, phosphoramidite ou hydrogénophosphonate en utilisant des 4thionucléosides pour des séquences oligothionucléotidiques et en ce que l'on prépare les dérivés totalement protégés et que l'on élimine les groupements protecteurs.

**16.** Procédé de synthèse des composés sans agent effecteur selon l'une des revendications 2 à 13, caractérisé en ce que, pour préparer les séquences oligothionucléotidiques, on réalise une synthèse supportée selon la méthode au phospohoroamidite comportant

- une protection en 3' et 5' des 4'thionucléotides ou oligo-4'thionucléotides de départ avec par exemple du diméthoxytrityl en 5' et du diisopropylaminophosphoramidite de méthyle en 3',
- la fonctionnalisation d'un support solide incorporant un dérivé 4'thionucléosidique par un lien par exemple succinyle entre le groupement hydroxyle-3' du dérivé 4'thionucléosidique et un groupement amino du support solide,
- l'élongation de la chaîne oligothionucléotidique dans un réacteur synthétiseur,
- enfin, le décrochage, la déprotection et la purification de l'oligothionucléotide prolongé.

**17.** Procédé de synthèse de composés incorporant un agent effecteur selon l'une des revendications 3 à 10, caractérisé en ce que l'on part d'un oligothionucléotide sans agent effecteur, ou oligomère mixte en comprenant, protégé en 5', dont on fait réagir la terminaison OH 3' avec une fonction non protégée d'un bras difonctionnel dont la deuxième fonction est protégée et après déprotection du produit résultant, on lie ledit produit à l'agent effecteur par la deuxième fonction libre du bras difonctionnel.

**18.** Application des composés selon l'une des revendications 1 à 13, caractérisée en ce que lesdits composés sont utilisés à titre de sondes d'hybridation ou comme composants de purification pour des séquences déterminées d'ADN ou d'ARN en simple brin ou en double hélice dans un but diagnostique ou pronostique.

**19.** Application des composés selon l'une des revendications 1 à 13, caractérisée en ce que les composés sont utilisés pour détecter des mutations au niveau d'un ADN ou d'un ARN.

**20.** Application des composés selon l'une des revendications 1 à 13 caractérisée en ce que ces composés sont utilisés pour réaliser le blocage spécifique de gènes cellulaires ou d'agents pathogènes préalablement choisis, tels que des virus, bactéries ou des parasites à l'exclusion des méthodes selon l'Art. 52(4) CBE.

**21.** Application d'un dérivé selon l'une des revendications 1 à 13 pour bloquer sélectivement l'expression d'un gène soit par hybridation, soit par coupure sélective, soit par modification chimique du gène ou de son ARN messager d'origine cellulaire, virale, bactérienne ou parasitaire à l'exclusion des méthodes selon l'Art. 52(4) CBE.

**22.** Application d'un dérivé selon l'une des revendications 1 à 13 pour bloquer sélectivement l'expression d'un ARN viral soit par hybridation, soit par coupure, soit par modification chimique de l'ARN viral ou de son ARN messager à l'exclusion des méthodes selon l'Art. 52(4) CBE.

**23.** Application d'un dérivé selon l'une des revendications 1 à 13, caractérisée en ce qu'ils sont utilisables à titre de nucléases artificielles spécifiques de séquences ou ribozyme artificielle à l'exclusion des méthodes selon l'Art. 52 (4) CBE.

**24.** A titre de médicaments, des composés selon l'une des revendications 1 à 13, utilisables comme antiviraux, antitumoraux, antibiotiques ou antiparasitaires ou dans toute pathologie où un gène est anormalement exprimé soit par mutation, soit par dérégulation.

## Patentansprüche

**1.** Chemische Verbindungen, bestehend aus einem Oligo-4'-thio-2'-desoxyribonucleotid oder einem Oligo-4'-thioribonucleotid, dadurch gekennzeichnet, daß sie eine Kettenverknüpfung von 4'-Thio-2'-desoxyribonucleotiden bzw. 4'-Thioribonucleotiden aufweisen, wobei die Kettenverknüpfung gegebenenfalls mit einem Effektor-Agens, insbesondere einem Rest, der einem Interkalierungsmittel entspricht, oder einem chemischen oder photoaktivierbaren Rest wie einem Rest, der eine Funktion trägt, die direkt oder indirekt mit den Nucleoid-Ketten reagiert, oder einem Rest dessen Anwesenheit einen leichten und empfindlichen Nachweis erlaubt, verbunden sein kann.

**2.** Chemische Verbindungen nach Anspruch 1, bestehend aus einem Oligo-4'-thioribonucleotid oder einem Oligo-4'-thio-2'-desoxyribonucleotid, dadurch gekennzeichnet, daß sie eine Kettenverknüpfung von 4-Thioribonucleotiden bzw. 4-Thio-2'-deoxyribonucleotiden aufweisen, die nicht mit einem Effektor-Rest verbunden sind.

**3.** Verbindungen nach Anspruch 1 oder 2, dadurch gekennzeichnet, daß sie die Formel haben

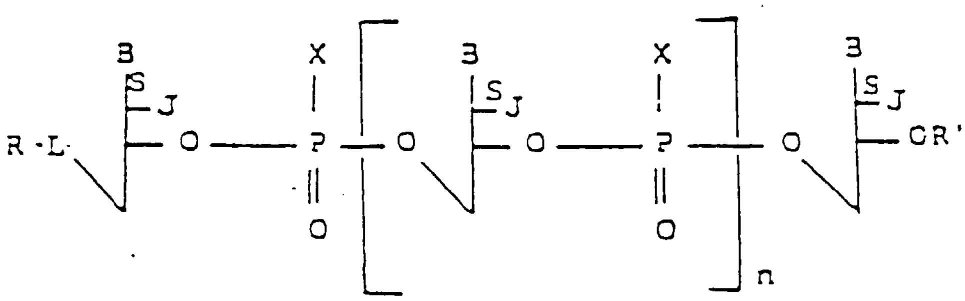

(I)

worin bedeuten:

die Reste B, die gleich oder verschieden sein können, jeweils eine Base einer Nucleinsäure, die gegebenenfalls modifiziert ist, aktivierbar ist und/oder eine Interkalierungs-Gruppierung trägt und in einer nicht-natürlichen α-Anomeren-Konfiguration an den glycosidischen Ring gebunden ist;

die Reste X, die gleich oder verschieden sein können, jeweils ein Oxoanion $O^-$, ein Thioanion $S^-$, eine Alkyl-, Alkoxy-, Thioalkyl-Gruppe, einen Alkyl- oder Alkoxy-Rest, der durch einen Stickstoff-haltigen Heterocyclus substituiert ist, oder eine Gruppierung -Y-Z;

R und R', die gleich oder verschieden sein können, jeweils ein Wasserstoffatom oder eine Gruppierung -Y-Z oder -Y'-Z';

Y und Y', die gleich oder verschieden sind, jeweils einen geradkettigen Alkylen- oder verzweigten -Alk-Rest oder einen Rest, ausgewählt aus der Gruppe

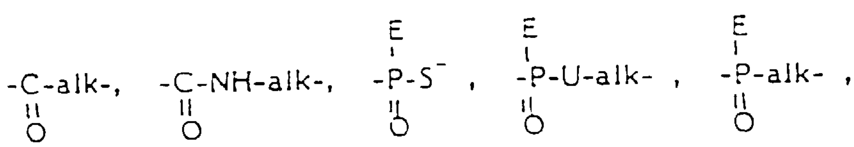

alk-O-alk

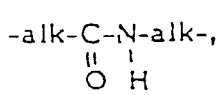, 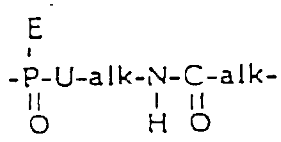 und 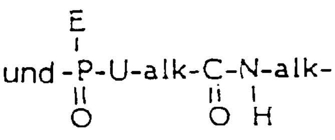

wobei U = O, S oder N und E die gleichen Bedeutungen wie X, ausgenommen die Bedeutung Y-Z oder Y'-Z', haben kann;

J — ein Wasserstoffatom oder eine Hydroxygruppe;

Z und Z', — die gleich oder verschieden sind, jeweils OH oder einen Rest, der einem Effektor-Agens entspricht, insbesondere einen Rest, der einem Interkalierungsmittel oder einem Rest entspricht, der eine Funktion trägt, die direkt oder indirekt mit den Nucleotid-Ketten reagiert, oder einen Rest, dessen Anwesenheit einen leichten und empfindlichen Nachweis erlaubt;

n — eine ganze Zahl einschließlich 0; und

L — ein Sauerstoffatom, ein Schwefelatom oder eine Gruppe -NH-.

**4.** Verbindungen nach Anspruch 3, dadurch gekennzeichnet, daß Y und Y' für einen Rest stehen, der ausgewählt wird aus der Gruppe

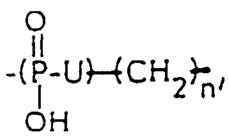, 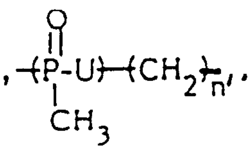, 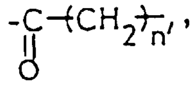, 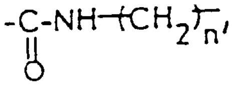,

worin U = O, S oder N und n' = eine ganze Zahl, insbesondere 1 bis 10.

**5.** Verbindungen nach Anspruch 3, dadurch gekennzeichnet, daß L für Sauerstoff steht, R und R' ein Wasserstoffatom bedeuten und B für eine natürliche Nucleinbase steht.

**6.** Verbindungen nach einem der Ansprüche 3 bis 5, dadurch gekennzeichnet, daß das Interkalierungs-Mittel Z oder Z' ausgewählt wird aus polycyclischen Verbindungen, die eine ebene Konfiguration haben.

**7.** Verbindungen nach Anspruch 6, dadurch gekennzeichnet, daß das Interkalierungs-Mittel Z oder Z' ausgewählt wird aus Acridin und seinen Derivaten, Furocumarin und seinen Derivaten, Daunomycin und anderen Derivaten von Anthracyclin, 1,10-Phenanthrolin, Phenanthridin und seinen Derivaten, Proflavin, Porphyrinen, Derivaten von Dipyrido[1,2-a;3'-d]imidazol, Ellipticin oder Ellipticinium und ihren Derivaten und Diazapyren und Derivaten davon.

**8.** Verbindungen nach einem der Ansprüche 3 bis 5, dadurch gekennzeichnet, daß die reaktionsfähigen chemischen Gruppen Z oder Z' ausgewählt werden aus Ethylendiamintetraessigsäure, Diethylentriaminpentaessigsäure, den Porphyrinen, 1,10-Phenanthrolin, 4-Azido-acetophenon, Ethylenimin, β-Chloroethylamin, Psoralen und ihren Derivaten.

**9.** Verbindungen nach einem der Ansprüche 3 bis 8, dadurch gekennzeichnet, daß der Rest B ein Rest ist, der entspricht dem Thymin, dem Adenin; dem 2-Aminoadenin und seinen substituierten Derivaten, dem Cytosin, dem Guanin und seinen substituierten Derivaten, dem Uracil, dem 5-Bromo-uracil, dem 8-Azido-adenin, dem 7-Deazaadenin, dem 7-Deazaguanin, dem 4-Azidocytosin, dem 4-Azido-thymin und den Derivaten dieser Basen, die eine Interkalierungs-Gruppierung und/oder eine chemische Gruppierung oder eine photochemisch aktivierbare Gruppierung aufweisen.

**10.** Verbindungen nach einem der Ansprüche 1 bis 9, dadurch gekennzeichnet, daß es sich dabei handelt um Oligothioribonucleotid-Verbindungen der Formel (I), in denen J = OH.

**11.** Gemischte oligomere Verbindungen, dadurch gekennzeichnet, daß sie bestehen aus Verbindungen nach einem der Ansprüche 1 bis 10, die gebunden sind an Oligonucleotide vom DNA- oder RNA-Typ, die im Bereich der Phosphat-Kettenverknüpfung modifiziert oder nicht-modifiziert sind und in denen das intracyclische Heteroatom des Furanose-Ringes der Nucleotide Sauerstoff ist.

**12.** Gemischte oligomere Verbindungen, dadurch gekennzeichnet, daß sie bestehen aus einer Verbindung nach einem der Ansprüche 1 bis 10, insbesondere der Formel (I), die an einem oder an jedem ihrer Enden eine Oligonucleotid-Sequenz vom DNA- oder RNA-Typ aufweist, die im Bereich der Phosphat- Kettenverknüpfung modifiziert oder nicht-modifiziert ist und in der das Heteroatom des Furanose-Ringes der Nucleotide Sauerstoff ist.

**13.** Gemischte oligomere Verbindungen nach Anspruch 12, dadurch gekennzeichnet, daß sie bestehen aus einer Oligothioribonucleotid-Verbindung nach einem der Ansprüche 1 bis 10, die eine Sequenz vom DNA-Typ aufweist, die im Bereich der Phosphat-Kettenverknüpfung modifiziert oder nicht-modifiziert ist und in deren Nucleotiden das Heteroatom des Furanose-Ringes der Nucleotide Sauerstoff ist.

**14.** Gemischte oligomere Verbindung nach Anspruch 13, dadurch gekennzeichnet, daß die genannte DNA-Sequenz, in der das Heteroatom des Furanose-Ringes Sauerstoff ist, von zwei Sequenzen vom Thioribonucleotid-Typ eingerahmt ist.

**15.** Verfahren zur Herstellung der Verbindungen nach einem der Ansprüche 1 bis 13, dadurch gekennzeichnet, daß man sie herstellt mit Phosphodiester, Phosphotriester, Phosphoamidit oder Hydrogenphosphonat unter Verwendung von 4-Thionucleosiden für die Oligothionucleotid-Sequenzen und daß man die vollständig geschützten Derivate herstellt und die Schutzgruppen entfernt.

**16.** Verfahren zur Synthese von Verbindungen ohne Effektor-Agens nach einem der Ansprüche 2 bis 13, dadurch gekennzeichnet, daß man zur Herstellung der Oligothionucleotid-Sequenzen eine Träger-Synthese nach der Phosphoroamidit-Methode durchführt, das umfaßt

- das Einführen einer Schutzgruppe in 3'- oder 5'-Stellung der Ausgangs-4'-Thionucleotide oder -Oligo-4'-thionucleotide beispielsweise mit Dimethoxytrityl in 5'-Stellung oder Methyldiisopropylaminophosphoramidit in 3'-Stellung;
- die Funktionalisierung eines festen Trägers durch Einarbeitung eines 4'-Thionucleosid-Derivats beispielsweise mittels Succinyl zwischen die 3'-Hydroxyl-Gruppe des 4'-Thionucleosid-Derivats und eine Aminogruppe des festen Trägers,
- die Verlängerung der Oligothionucleotid -Kette in einem Synthesereaktor und
- schließlich die Ablösung, die Schutzgruppenentfernung und die Reinigung des verlängerten Oligothionucleotids.

**17.** Verfahren zur Synthese von Verbindungen, die ein Effektor-Agens enthalten, nach einem der Ansprüche 3 bis 10, dadurch gekennzeichnet, daß man von einem Oligothionucleotid ohne Effektor-Agens oder einem gemischten Oligomer, das ein solches umfaßt und in 5'-Stellung geschützt ist, ausgeht, daß man dessen 3'-OH-Endgruppe mit einer nicht-geschützten Funktion eines difunktionellen Zweiges, dessen zweite Funktion geschützt ist, reagieren läßt, und daß man nach der Schutzgruppenentfernung aus dem resultierenden Produkt das genannte Produkt mit dem Effektor-Agens über die zweite freie Funktion mit dem difunktionellen Zweig verbindet.

**18.** Verwendung der Verbindungen nach einem der Ansprüche 1 bis 13, dadurch gekennzeichnet, daß die genannten Verbindungen als Hybridisierungssonden oder als Reinigungskomponenten für bestimmte Einfachstrang- oder Doppelhelix-DNA- oder RNA-Sequenzen für einen diagnostischen oder prognostischen Zweck verwendet werden.

**19.** Verwendung der Verbindungen nach einem der Ansprüche 1 bis 13, dadurch gekennzeichnet, daß die Verbindungen zum Nachweis von Mutationen im Bereich einer DNA oder RNA verwendet werden.

**20.** Verwendung der Verbindungen nach einem der Ansprüche 1 bis 13, dadurch gekennzeichnet, daß diese Verbindungen zur Erzielung einer spezifischen Blockierung von ausgewählten Zellgenen oder pathogenen Agentien wie Viren, Bakterien oder Parasiten mit Ausnahme der Verfahren nach Artikel 52(4) EPÜ verwendet werden.

**21.** Verwendung eines Derivats nach einem der Ansprüche 1 bis 13 zum selektiven Blockieren der Expression eines Gens entweder durch Hybridisierung oder durch selektives Schneiden, entweder durch chemische Modifizierung

des Gens oder seiner Messenger-RNA zellulären, viralen, bakteriellen oder parasitären Ursprungs mit Ausnahme der Verfahren nach Artikel 52(4) EPÜ.

**22.** Verwendung eines Derivats nach einem der Ansprüche 1 bis 13 zum selektiven Blockieren der Expression einer Viren-RNA entweder durch Hybridisierung oder durch Schneiden oder durch chemische Modifizierung der viralen RNA oder ihrer Messenger-RNA mit Ausnahme der Verfahren nach Artikel 52(4) EPÜ.

**23.** Verwendung eines Derivats nach einem der Ansprüche 1 bis 13, dadurch gekennzeichnet, daß sie als künstliche, sequenzspezifische Nucleasen oder künstliches Ribozym verwendbar sind mit Ausnahme der Verfahren nach Artikel 52(4) EPÜ.

**24.** Verbindungen nach einem der Ansprüche 1 bis 13 zur Verwendung als Arzneimittel, die in der Antiviren-, Antitumor-, Antibiotika- oder Antiparasiten-Pathologie oder in einer anderen Pathologie verwendbar sind, in der ein Gen entweder durch Mutation oder durch Deregulierung anormal exprimiert wird.

## Claims

**1.** Chemical compounds consisting of an oligo-4'-thio-2'-deoxyribonucleotide or an oligo-4'-thioribonucleotide, characterized in that they comprise a concatenation of 4'-thio-2'-deoxyribonucleotides or 4'-thioribonucleotides respectively, it being possible for the said concatenation to be optionally linked to an effector, especially a radical corresponding to an intercalating agent or a chemical or photoactivable radical, such as a radical carrying a functional group which reacts directly or indirectly with the nucleotide chains or a radical whose presence permits easy and sensitive detection.

**2.** Chemical compounds according to Claim 1, consisting of an oligo-4'-thioribonucleotide or oligo-4'-thio-2'-deoxyribonucleotide, characterized in that they comprise a concatenation of 4'-thioribonucleotides or 4'-thio-2'-deoxyribonucleotides respectively, not linked to an effector radical.

**3.** Compounds according to Claim 1 or 2, characterized in that they correspond to the formula:

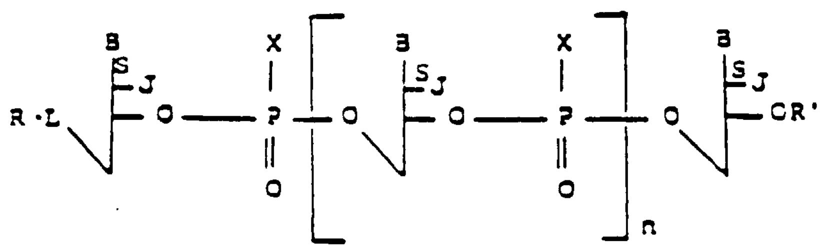

(I)

in which:

the B radicals may be identical or different and each represent a base of a nucleic acid optionally modified, activable and/or comprising an intercalating group and attached to the glycoside ring according to a non-natural alpha anomeric configuration;
the X radicals may be identical or different and each represent an oxoanion $O^-$, a thioanion $S^-$, an alkyl group, an alkoxy group, a thioalkyl group, an alkyl or alkoxy radical substituted by a nitrogen-containing heterocycle or a -Y-Z group;
R and R', which may be identical or different, each represent a hydrogen atom or a -Y-Z or Y'-Z' group;
Y and Y', which are identical or different, each represent a straight or branched alkylene radical -alk- or a radical chosen from

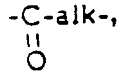, 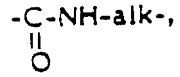, 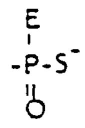, 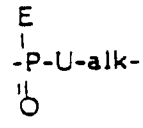, 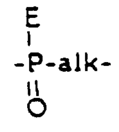,

alk-O-alk

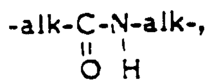, 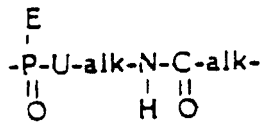 and 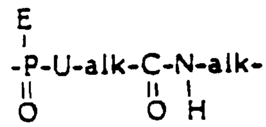

with U = O, S or N
E may have the same meanings as X except Y-Z or Y'-Z';
J represents a hydrogen atom or an hydroxyl group;
Z and Z', which are identical or different, each represent OH or a radical corresponding to an effector, especially a radical corresponding to an intercalating agent or a radical carrying a functional group which reacts directly or indirectly with the nucleotide chains or a radical whose presence permits easy and sensitive detection
n is an integer including 0;
L represents an oxygen atom, a sulphur atom or a group -NH-.

**4.** Compounds according to Claim 3, characterized in that Y and Y' represent a radical chosen from

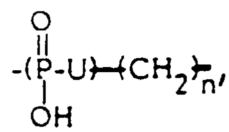, 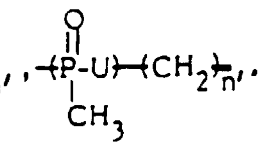, 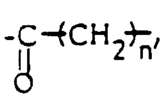, 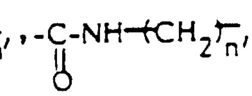,

with U = O, S or N and n' is an integer especially from 1 to 10.

**5.** Compounds according to Claim 3, characterized in that L represents oxygen, R and R' represent a hydrogen atom and B represents a natural nucleic base.

**6.** Compounds according to one of Claims 3 to 5, characterized in that the intercalating agent Z or Z' is chosen from polycyclic compounds having a planar configuration.

**7.** Compounds according to Claim 6, characterized in that the intercalating agent Z or Z' is chosen from acridine and its derivatives, furocoumarin and its derivatives, daunomycin and the other derivatives of anthracycline, 1,10-phenanthroline, phenanthridine and its derivatives, proflavine, porphyrins, derivatives of dipyrido[1,2-a:3'-d]imidazole, ellipticine or ellipticinium and their derivatives and diazapyrene and derivatives.

**8.** Compounds according to one of Claims 3 to 5, characterized in that the reactive chemical groups Z or Z' are chosen from ethylenediaminetetraacetic acid, diethylenetriaminepentaacetic acid, porphyrins, 1,10-phenanthroline, 4-azidoacetophenone, ethylene-imine, beta-chloroethylamine, psoralen and their derivatives.

**9.** Compounds according to one of Claims 3 to 8, characterized in that the B radical is a radical corresponding to thymine, adenine; 2-aminoadenine and its substituted derivatives, cytosine, guanine and its substituted derivatives, uracil, 5-bromouracil, 8-azidoadenine, 7-deazaadenine, 7-deazaguanine, 4-azidocytosine, 4-azidothymine and the derivatives of these bases comprising an intercalating group and/or a chemically or photochemically activable group.

**10.** Compounds according to one of Claims 1 to 9, characterized in that they are oligothioribonucleotide compounds of formula I in which J = OH.

**11.** Mixed oligomeric compounds characterized in that they consist of compounds according to one of Claims 1 to 10 linked to DNA or RNA type oligonucleotides modified or otherwise at the level of the phosphate concatenation and in which the intracyclic heteroatom of the furanose ring of the nucleotides is oxygen.

**12.** Mixed oligomeric compounds characterized in that they consist of a compound according to one of Claims 1 to 10, especially of formula (I), comprising, at one or at each of its ends, a DNA or RNA type oligonucleotide sequence modified or not at the level of the phosphate concatenation, in which the heteroatom of the furanose ring of the nucleotides is oxygen.

**13.** Mixed oligomeric compounds according to Claim 12, characterized in that they consist of an oligothioribonucleotide compound according to one of Claims 1 to 10, comprising a DNA type sequence modified or not at the level of the phosphate concatenation in whose nucleotides the heteroatom of the furanose ring of the nucleotides is oxygen.

**14.** Mixed oligomeric compounds according to Claim 13, characterized in that the said DNA sequence, in which the heteroatom of the furanose ring is oxygen, is flanked by two sequences of the thioribonucleotide type.

**15.** Process for the preparation of compounds according to one of Claims 1 to 13, characterized in that syntheses using phosphodiester, phosphotriester, phosphoramidite or hydrogen phosphonate are applied using 4'-thionucleosides for oligothionucleotide sequences and in that the completely protected derivatives are prepared and that the protecting groups are removed.

**16.** Process for the synthesis of the compounds without effector according to one of Claims 2 to 13, characterized in that to prepare the oligothionucleotide sequences, a supported synthesis is carried out according to the phosphoroamidite method comprising

- a protection in 3' and 5' of the starting 4'-thionucleotides or oligo-4'-thionucleotides with for example dimethoxytrityl in 5' and methyl diisopropylaminophosphoramidite in 3',
- the functionalization of a solid support incorporating a 4'-thionucleoside derivative by for example a succinyl linkage between the 3'-hydroxyl group of the 4'-thionucleoside derivative and an amino group of the solid support,
- the elongation of the oligothionucleotide chain in a synthesizing reactor,
- finally the detachment, deprotection and purification of the extended oligothionucleotide.

**17.** Process for the synthesis of compounds incorporating an effector according to one of Claims 3 to 10, characterized in that there is used as starting material an oligothionucleotide without effector, or a mixed oligomer containing it, protected in 5', whose 3' OH end is reacted with an unprotected functional group of a difunctional arm whose second functional group is protected and after deprotection of the resulting product, the said product is linked to the effector by the second free functional group of the difunctional arm.

**18.** Application of the compounds according to one of Claims 1 to 13, characterized in that the said compounds are used as hybridization probes or as purification components for given DNA or RNA sequences as single strand or as double helix for diagnostic or prognostic purposes.

**19.** Application of the compounds according to one of Claims 1 to 13, characterized in that the compounds are used to detect mutations at the level of a DNA or an RNA.

**20.** Application of the compounds according to one of Claims 1 to 13, characterized in that these compounds are used to carry out the specific blocking of cellular genes or of pathogenic agents chosen beforehand, such as viruses, bacteria or parasites, with the exclusion of the methods according to Art.52 (4)EPC.

**21.** Application of a derivative according to one of Claims 1 to 13, to selectively block the expression of a gene either by hybridization or by selective cutting, or by chemical modification of the gene or of its messenger RNA of cellular, viral, bacterial or parasitic origin, with the exclusion of the methods according to Art.52 (4)EPC.

**22.** Application of a derivative according to one of Claims 1 to 13 to selectively block the expression of a viral RNA either by hybridization or by cutting or by chemical modification of the viral RNA or of its messenger RNA, with the exclusion of the methods according to Art.52 (4)EPC.

**23.** Application of a derivative according to one of Claims 1 to 13, characterized in that it can be used as sequence-specific artificial nucleases or as artificial ribozyme, with the exclusion of the methods according to Art.52 (4)EPC.

**24.** As medicinal products, compounds according to one of Claims 1 to 13, which can be used as antiviral agents, antitumor agents, antibiotics or antiparasitic agents or in any pathology where a gene is abnormally expressed either by mutation or by deregulation.

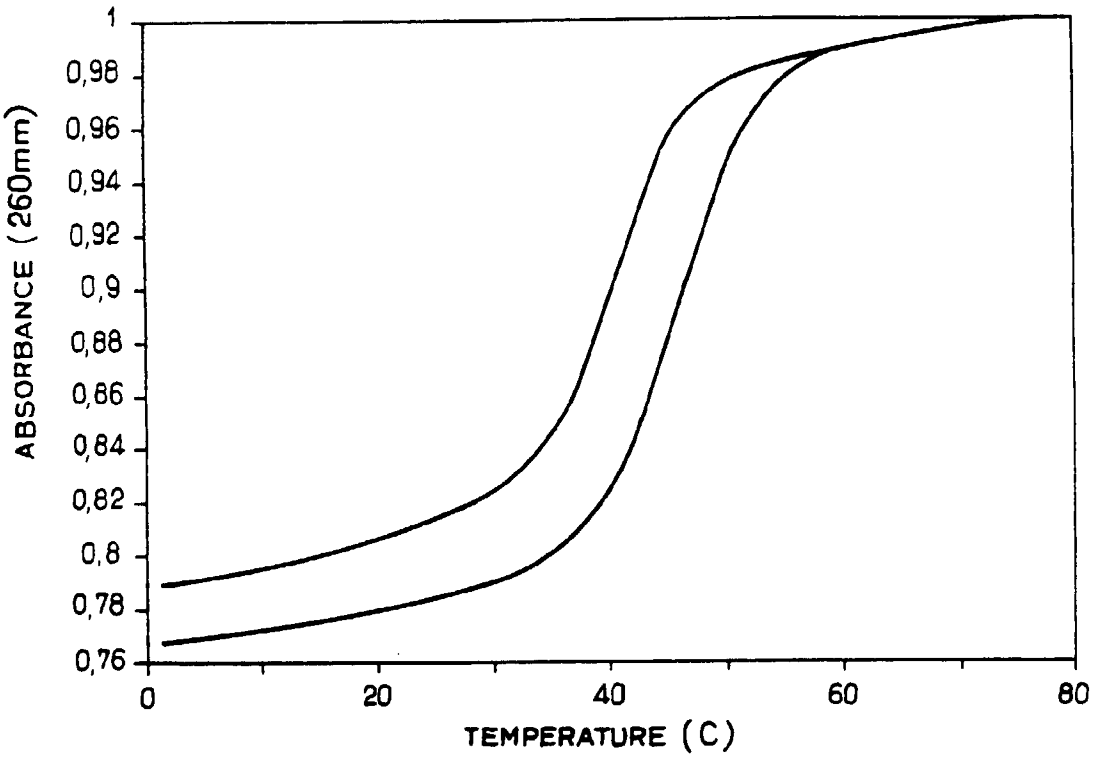
ABSORBANCE (260mm)
1
0,98
0,96
0,94
0,92
0,9
0,88
0,86
0,84
0,82
0,8
0,78
0,76
0
20
40
60
80
TEMPERATURE (C)

FIG_1

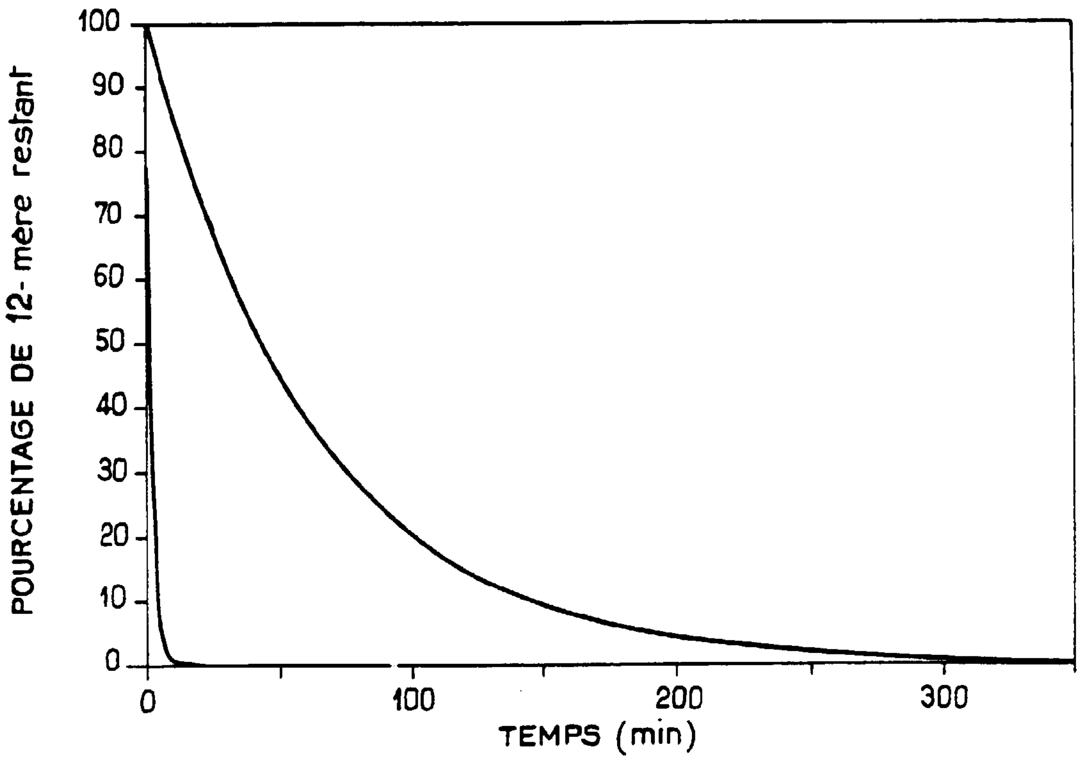
POURCENTAGE DE 12- mère restant
100
90
80
70
60
50
40
30
20
10
0
0
100
200
300
TEMPS (min)

FIG_2